# EUROPÄISCHE PATENTSCHRIFT

(11) **EP 1 337 648 B1**
(45) Veröffentlichungstag und Bekanntmachung des Hinweises auf die Patenterteilung: **19.09.2007**
(21) Anmeldenummer: 01984740.9
(22) Anmeldetag: 17.11.2001
(51) Int. Cl.: C12N 15/54, C12N 15/56, C12N 9/10, C12N 9/28, C12N 1/21, C11D 3/386, D06M 16/00, A61K 38/45, A23K 1/00

(54) **CYCLODEXTRIN -GLUCANOTRANSFERASE(CG TASE) AUS BACILLUS AGARADHERENS(DSM 9948)SOWIE WASCH-UND REINIGUNGSMITTEL MIT DIESER NEUEN CYCLODEXTRIN-GLUCANOTRANSFERASE**
NOVEL CYCLODEXTRIN GLUCANOTRANSFERASE (CGTASE), OBTAINED FROM I BACILLUS AGARADHERENS /I (DSM 9948) AND DETERGENTS AND CLEANING AGENTS CONTAINING SAID NOVEL CYCLODEXTRIN GLUCANOTRANSFERASE
NOUVELLE CYCLODEXTRINE-GLUCANOTRANSFERASE (CGTASE) ISSUE DE I BACILLUS AGARADHERENS /I (DSM 9948) ET PRODUITS DETERGENTS ET DE NETTOYAGE COMPRENANT CETTE NOUVELLE CYCLODEXTRINE-GLUCANOTRANSFERASE

(30) Priorität: 28.11.2000 DE 10059108; 28.11.2000 DE 10059105
(43) Veröffentlichungstag der Anmeldung: 27.08.2003
(73) Patentinhaber: Henkel Kommanditgesellschaft auf Aktien, 40589 Düsseldorf (DE)
(72) Erfinder: KOTTWITZ, Beatrix, 40593 Düsseldorf (DE); MAURER, Karl-Heinz, 40699 Erkrath (DE); BREVES, Roland, 40882 Ratingen (DE); SCHMIDT, Irmgard, 42697 Solingen (DE); WEBER, Angrit, 51467 Bergisch-Gladbach (DE); HELLEBRANDT, Angela, 50735 Köln (DE); POLANYI, Laura, 50859 Köln (DE)
(86) Internationale Anmeldenummer: PCT/EP2001/013278
(87) Internationale Veröffentlichungsnummer: WO 2002/044350

(56) Entgegenhaltungen:
- WO-A-02/06508
- WO-A-96/33267
- WO-A-99/57254
- US-A- 5 922 586

## Beschreibung

Die vorliegende Anmeldung betrifft eine neue Cyclodextrin-Glucanotransferase (CGTase) aus *Bacillus agaradherens* (DSM 9948) und hinreichend ähnliche amylolytische und/oder CGTase-Aktivität ausübende Proteine oder Derivate, die zugehörigen Nukleinsäuren und produzierenden Mikroorganismen, Verfahren zur Herstellung dieser Proteine sowie diverse Einsatzmöglichkeiten für sie. Sie betrifft neben anderen technischen Einsatzgebieten insbesondere Wasch- und Reinigungsmittel mit derartigen CGTasen, Verfahren zur Reinigung von Textilien oder harten Oberflächen unter Beteiligung derartiger CGTasen oder entsprechender Mittel, sowie deren Verwendung zur Reinigung von Textilien oder harten Oberflächen.

α-Amylasen (E.C. 3. 2. 1. 1) hydrolysieren interne α-1,4-glycosidische Bindungen von Stärke und stärkeähnlichen Polymeren wie Amylose, Amylopektin oder Glycogen, unter Bildung von Dextrinen und β-1,6-verzweigten Oligosacchariden. Sie gehören zu den wichtigsten industriell genutzten Enzymen überhaupt. Denn zum einen werden sie zumeist wie viele substratabbauende Enzyme von Mikroorganismen in das umgebende Medium abgegeben, so daß sie durch Fermentation und Aufreinigung aus dem Kulturmedium mit vergleichsweise geringem Aufwand in industriellem Maßstab gewonnen werden können. Zum anderen werden Amylasen für ein breites Anwendungsspektrum benötigt.

Eine wichtige technische Verwendung von α-Amylase ist die Herstellung von Glucosesirup. Andere Verwendungen sind beispielsweise die als aktive Komponenten in Wasch- und Reinigungsmitteln, zur Behandlung von Rohmaterialien in der Textilherstellung, zur Herstellung von Klebstoffen oder zur Herstellung von zuckerhaltigen Lebensmitteln oder Lebensmittelbestandteilen. CGTasen werden insbesondere für die Synthese und Verarbeitung von Cyclodextrinen eingesetzt.

Unter anderem hydrolytische Enzyme, wie beispielsweise Proteasen, Cellulasen oder Lipasen, werden seit geraumer Zeit in Wasch- und Reinigungsmitteln eingesetzt. Hierunter sind auch Amylasen als stärkespaltende Enzyme ebenfalls gut etabliert.

Eine technisch bedeutende und auch in Wasch- und Reinigungsmitteln häufig eingesetzte α-Amylase ist die aus *Bacillus licheniformis.* Das entsprechende Produkt der Fa. Novozymes A/S, Bagsværd, Dänemark, beispielsweise, trägt den Handelsnamen Termamyl^{®}; von der Fa. Genencor Int., Rochester, New York, USA, heißt es Purastar®. Das aus *B. subtilis,* beziehungsweise B. *amyloliquefaciens* gewonnene Homolog wird von Novozymes unter dem Namen BAN^{®} vertrieben.

Dieses Amylase-Molekül, beziehungsweise dessen nahen Verwandte, sind in zahlreichen Erfindungen weiterentwickelt worden, denen die Aufgabe zugrunde gelegen hat, mithilfe diverser, insbesondere molekularbiologischer Modifikationen ihre enzymatischen Eigenschaften auf spezifische Anwendungen hin zu optimieren. Solche Optimierungen können beispielsweise die Substratspezifitäten, die Stabilität des Enzyms unter verschiedenen Reaktionsbedingungen oder die enzymatische Aktivität selbst betreffen. Beispielhaft für solche Optimierungen seien folgende Anmeldungen genannt: EP 410498 für das Schlichten von Textilien und WO 96/02633 zur Stärkeverflüssigung. Zur Stabilisierung gegenüber der Oxidation von Methioninresten durch Wasserstoffperoxid zum Beispiel beim Waschen von Textilien und maschinellen Reinigen sind Amylasevarianten beschrieben, bei denen Methionin durch nichtoxidierbare Aminosäuren ausgetauscht wurde. Beispiele hierfür sind unter WO 94/18314, WO 96/30481, WO 95/26397 und WO 94/02597 beschrieben.

α-Amylasen werden vor allem aber auch hinsichtlich ihrer Verwendung in Wasch- und Reinigungsmitteln weiterentwickelt. Als Beispiele dafür seien nur folgende Anmeldungen genannt: Die Amylasen der Anmeldung WO 99/02702 sind bei höheren Temperaturen stabiler als das Ausgangsmolekül. Die Enzyme der Anmeldung WO 99/23211 sind bei hohen pH-Werten, in Gegenwart von Calcium-lonen und bei höheren Temperaturen stabil. Die α-Amylasen der Anmeldung WO 97/43424 zeigen ein geändertes Calciumionen-Bindungsverhalten und damit geänderte enzymatische Eigenschaften. Das Mutagenese-Verfahren der Anmeldung WO 99/20768 führt zu α-Amylase-Varianten, die in Gegenwart von Reinigungsmittelbestandteilen besonders stabil sind.

Praktisch immer wirkt sich bei derartigen Modifikationen eine Änderung einzelner enzymatischer Eigenschaften auch auf andere Eigenschaften und auf die Waschleistung des betreffenden Enzyms aus. Ein Beispiel für ein auf solche Weise erhaltenes, inzwischen kommerziell vermarktetes Optimierungsprodukt ist Duramyl^{®} (WO 94/02597) mit verringerter Oxidationsempfindlichkeit (Fa. Novozymes A/S, Bagsværd, Dänemark; S6FVI/ Journal 123, (1997), S. 723-731).

Da Entwicklungen, die lediglich in Optimierungen von nur wenigen bekannten Ausgangsenzymen bestehen, möglicherweise in den erzielbaren Ergebnissen beschränkt sind, findet parallel dazu eine intensive Suche nach vergleichbaren Enzymen aus anderen natürlichen Quellen statt. Identifiziert wurden stärkespaltende Enzyme beispielsweise aus *Pimelobacter, Pseudomonas* und *Thermus* für die Lebensmittelherstellung, Kosmetik und Pharmaka (EP 636693), ebensolche aus *Rhizobium, Arthrobacter, Brevibacterium* und *Micrococcus* (EP 628630), aus *Pyrococcus* (WO 94/19454) und aus *Sulfolobus* zur Stärkeverflüssigung bei hohen Temperaturen, beziehungsweise stark sauren Reaktionsbedingungen (EP 727485 und WO 96/02633). Für den Einsatz bei alkalischen pH-Werten sind Amylasen aus *Bacillus sp.* (WO 95/26397 und WO 97/00324) gefunden worden. Wegen ihrer geringen Empfindlichkeit gegenüber Detergenzien eignen sich andere Amylasen aus verschiedenen *Bacilli* (EP 670367) zur Verwendung in Wasch- oder Reinigungsmitteln.

Enzyme aus neu erschlossenen Organismen eignen sich aufgrund ihrer Herkunft möglicherweise besser als die wenigen etablierten Enzyme, um auf spezifische Anwendungen hin weiterentwickelt zu werden. Ein Beispiel dafür ist die Amylase aus *Thermoalcalibacter* (WO 98/13481), deren natürliche Aktivität weitgehend unempfindlich gegenüber Calcium-lonen ist, so daß sie von vornherein gute Voraussetzungen für die Verwendung in Waschmitteln aufweist.

Weitere Optimierungen der aus natürlichen Quellen isolierten Enzyme für das jeweilige Anwendungsgebiet können beispielsweise über molekularbiologische Methoden (etwa gemäß US 5171673 oder WO 99/20768) oder über chemische Modifikationen vorgenommen werden (DE 4013142). Die Anmeldungen WO 99/57250 und WO 99/57254, beispielsweise, geben Lehren an die Hand, für die Verwendung in Wasch- und Reinigungsmitteln geeignete Enzyme über chemische Linker oder in Form von chimären Proteinen mit einer Bindungsdomäne zu verknüpfen, welche die effektive Enzymkonzentration auf dem Reinigungsgut erhöht.

In der Patentanmeldung WO 99/43793 wird eine Weiterentwicklung der bekannten Novamyl^{®}-α-Amylase beschrieben. Demnach können Sequenzähnlichkeiten zwischen Novamyl^{®} und bekannten Cyclodextringlucanotransferasen (CGTasen) ausgenutzt werden, um mithilfe molekularbiologischer Techniken eine Schar verwandter Moleküle zu konstruieren. Bei diesen handelt es sich um α-Amylasen mit zusätzlichen CGTasespezifischen Consensus-Sequenzen (Boxen) und Funktionen oder, umgekehrt, um CGTasen mit zusätzlichen für α-Amylasen typischen Bereichen und Funktionen oder um Chimären beider Moleküle. Bereits mit der Anmeldung WO 91/04669 war die Anwendung der Novamyl^{®}-α-Amylase wegen ihres Effekts zur Verzögerung des Altbackenwerdens bei der Herstellung von Brot oder anderen Backwaren (anti staling effect) offenbart worden. Der Sinn dieser neuen Weiterentwicklung liegt in erster Linie darin, Novamyl^{®} für diese Anwendungen zu optimieren.

In der Anmeldung WO 99/57250 wird offenbart, wie Waschmittelenzyme, beispielsweise Lipasen, Cellulasen, Proteasen, Amylasen oder auch CGTasen, in ihrer Waschleistung gesteigert werden können. Das dort beschriebene Prinzip besteht darin, daß die betreffenden Enzyme über einen nicht-Aminosäure-Linker an Cellulose-Bindungsdomänen (CBD) bakteriellen Ursprungs gebunden werden. Diese sorgen dafür, daß das Enzym verstärkt auf der Oberfläche des Textils wirksam wird. Mit der Schrift WO 99/57252 werden in dieses Konzept andere mögliche Linker miteinbezogen, und mit der Schrift WO 99/57254 andere Enzyme, wie beispielsweise Glykosyl-Transferasen oder Acyl-Transferasen, die entweder direkt, das heißt unter Bildung eines chimären Proteins, oder über die in WO 99/57252 erwähnten Linker an die CBD gebunden werden.

Als spezielle α-Amylasen sind Cyclodextringlucanotransferasen (CGTasen; E.C. 2. 4. 1. 19) anzusehen, da sie dieselben Domänen A, B und C wie α-Amylasen besitzen, welche für deren amylolytische Funktion verantwortlich sind; zusätzlich verfügen CGTasen über die zwei weiteren Domänen D und E, deren Funktion darin besteht, intramolekulare Transglykosylierungen zu ermöglichen. Die enzymatische Aktivität einer CGTase besteht also darin, ähnlich wie eine α-Amylase interne α-(1,4)-glycosidische Bindungen von Amylose oder Amylopektin zu spalten, aber nicht hydrolytisch, sondern über intramolekulare Transglykosylierungen. Dadurch werden die stärkeähnlichen Polysaccharide unter Bildung von Cyclodextrinen abgebaut.

Cyclodextrine sind α-(1,4)-glycosidisch verknüpfte, cyclische Oligosaccharide, von denen die aus 6, 7 oder 8 Glucose-Monomeren, die α-, β-, beziehungsweise γ-Cyclodextrine (oder Cyclohexa-, hepta-, beziehungsweise octa-Amylosen), wirtschaftlich die größte Bedeutung besitzen; es sind aber auch Cyclodextrine aus mehr als acht Monomeren bekannt. Sie bilden, insbesondere wenn sie im Kristallgitter übereinandergeschichtet sind, durchgehende innermolekulare Kanäle aus, in welche hydrophobe Gastmoleküle eingelagert werden können. Solche Einschlußverbindungen sind bislang unter anderem für die Herstellung von Nahrungsmitteln, für die Pharmazie oder die Kosmetik von Bedeutung.

CGTasen gehören wie die α-Amylasen zur Glycosyl-Hydrolase-Familie, deren Vertreter in den Bereichen der Domänen A, B und C das Strukturmerkmal eines (β/α)₈-Barrels und vergleichbare katalytische Aktivitäten aufweisen. Es sind zahlreiche CGTasen bakteriellen Ursprungs bekannt. Sie weisen bei ansonsten eher geringen Sequenzhomologien die fünf hochkonservierte Regionen A bis E auf; die beiden C-terminalen Regionen D und E sind dabei für CGTasen kennzeichnend. Nakamura et al. (FEBS-Letters, 296 (1992), S. 37-40) haben gezeigt, daß drei Aminosäurereste von α-Amylasen, die als die katalytisch aktiven Reste angesehen werden, ebenso in den N-terminalen Regionen der CGTasen konserviert sind, was den Schluß zuläßt, daß beide Amylose-spaltenden Enzyme einen ähnlichen katalytischen Reaktionsmechanismus aufweisen. Dennoch liegen auch in den Regionen A, B und C einzelne Aminosäuren, die die CGTasen von den α-Amylasen unterscheiden (Wind et al., Eur. J. Biochem., 253 (1998), S. 598 - 605).

Für CGTasen sind verschiedene Anwendungen bekannt, beispielsweise im Lebensmittelbereich oder im pharmazeutischen Sektor. Zur Herstellung von Backwaren, beispielsweise, werden sie wegen ihrer stärkespaltenden Aktivität zur Verhinderung des unter Altbacken-Werden bekannten Geschmacksverlusts (Anti-staling-Effekt) verwendet. Die Fähigkeit von Cyclodextrinen, hydrophobe Verbindungen einzulagern, macht sie für die Aufnahme und zeitverzögerte Freisetzung von Farbstoffen oder pharmakologisch aktiven Substanzen interessant. Somit kommt auch den CGTasen bei der Herstellung und Nutzung solcher Einschlußverbindungen eine wichtige Funktion zu.

Dieser Effekt kommt, wie in den Schriften JP 7-107971 und JP 7-109488 für die CGTasen aus alkaliphilien Bacilli beschrieben worden ist, auch ihrer Reinigungsleistung zugute. Denn wahrscheinlich über ihre Fähigkeit, niedermolekulare Verbindungen in Cyclodextrine einzuschließen und damit zu maskieren, können sie auch eine desodorierende Wirkung entfalten.

Wie an den α-Amylasen sind auch an CGTasen Punktmutationen zur Modifikation ihrer enzymatischen Eigenschaften vorgenommen worden, etwa gemäß der Anmeldung WO 96/33267. Dort werden ausgehend von bekannten bakteriellen CGTasen diverse Varianten offenbart, die in Hinblick auf Substratbindung und/oder Produktselektivität modifiziert worden sind. Auch die Enzymmodifikation der Anmeldung WO 99/43793 kann als Mutagenese an einer CGTase angesehen werden, wenn auch die Eigenschaften des resultierenden Enzyms als eine modifizierte α-Amylase im Vordergrund der betreffenden Anmeldung stehen.

Jedes für Wasch- und/oder Reinigungsmittel verwendete Enzym, so auch jedes Stärkehydrolysierende Enzym besitzt ein individuelles Leistungsprofil. Dies belegt zusätzlich die Notwendigkeit dafür, den Stand der Technik um weitere, beispielsweise amylolytische Enzyme zu bereichern. Diese Notwendigkeit ergibt sich unter anderem auch aus den sich ändernden Gewohnheiten und Ansprüchen der Verbraucher, nach denen, beispielsweise, ein zunehmender Bedarf nach Waschmitteln für die Reinigung bei niedrigen und mittleren Temperaturen besteht.

Darüberhinaus können neue Enzyme, wie sie aus bislang für diesen Zweck noch nicht erschlossenen Organismen erhalten werden können, im Sinne eines "Protein Engineering" als Ausgangspunkt für weitere gentechnische oder sonstige Modifizierungen dienen. Deren Ziel ist die Herbeiführung von Eigenschaften, die die bisher bekannten Enzyme oder die von diesen abgeleiteten Waschmittelenzyme nicht aufweisen.

Andererseits erscheinen gerade solche natürlichen Enzyme als besonders geeignete Kandidaten für derartige Optimierungen, die von vornherein im Zusammenspiel mit üblichen Wasch- oder Reinigungsmittelbestandteilen eine gewisse Wasch-, beziehungsweise Reinigungsleistung aufweisen.

Trotz all dieser Entwicklungen besteht also unverändert die Aufgabe, neben den wenigen natürlichen amylolytischen Enzymen, die unverändert oder in Gestalt von Weiterentwicklungen-tatsächlich industriell genutzt werden, weitere aufzufinden, die a *priori* ein breites Anwendungsspektrum aufweisen und als Ausgangspunkt für spezifische Weiterentwicklungen dienen können.

Der vorliegenden Erfindung hat somit die Aufgabe zugrunde gelegen, ein natürliches, bislang noch nicht beschriebene amylolytisches Enzym zu identifizieren, welches selbst für technische Einsatzmöglichkeiten, insbesondere in Wasch- oder Reinigungsmitteln als geeignet erscheint oder als Grundlage für anwendungsspezifische Weiterentwicklungen dienen kann.

Eine Teilaufgabe bestand darin, die für ein derartiges amylolytisches Enzym codierende Nukleinsäure zu erhalten, da diese sowohl für die biotechnologische Herstellung als auch für die Weiterentwicklung dieser Enzyme unerläßlich ist.

Eine weitere Teilaufgabe bestand darin, einen Organismus aufzufinden, welcher solch ein amylolytisches Enzym natürlicherweise produziert.

Eine weitere Teilaufgabe bestand darin, die biotechnologische Produktion eines solchen amylolytischen Enzyms zu ermöglichen.

Eine weitere Teilaufgabe bestand darin, Wasch- oder Reinigungsmittel mit einem neuen amylolytischen Enzym bereitzustellen. Dabei sollte das aus einem Wildtyp-Stamm erhältliche, amylolytische und gegebenenfalls über weitere Aktivitäten verfügende Enzym im Zusammenspiel mit den für solche Mittel üblichen Inhaltsstoffen vorteilhafterweise Wasch-, beziehungsweise Reinigungsleistungen zeigen, die denen von etablierten und für den Gebrauch in solchen Mitteln optimierten Enzymen überlegen oder zumindest gleichwertig sind. Eventuelle über die reine amylolytische Funktion hinausgehende Eigenschaften dieses Enzyms sollten seinem Beitrag zur Waschleistung oder der Waschleistung des Mittels zugute kommen oder sie zumindest nicht beeinträchtigen.

Weitere Teilaspekte liegen in der Bereitstellung entsprechender Wasch- oder Reinigungsverfahren und dem Aufzeigen entsprechender Verwendungsmöglichkeiten.

Eine weitere Teilaufgabe bestand darin, weitere technische Einsatzmöglichkeiten für solch ein amylolytisches Enzym zu definieren.

Überraschenderweise stellen Enzyme wie die mit der vorliegenden Anmeldung zur Verfügung gestellte Cyclodextrin-Glucanotransferase (CGTase) aus *Bacillus agaradherens* (DSM 9948) hierfür geeignete Vertreter dar. Dieses natürlich vorkommende Enzym kann als Cyclodextringlucanotransferase (CGTase) und gleichermaßen als α-Amylase angesehen werden. Es kann somit aufgrund beider Aktivitäten eingesetzt werden. Die α-Amylase-Aktivität residiert in den Domänen A, B und C. Über die von den Domänen D und E ausgeübten Funktionen ergibt sich die CGTase-Aktivität und damit eine entsprechende Erweiterung des Wirkungsspektrums und damit der Einsatzmöglichkeiten.

Die Lösung der Aufgabe stellen somit amylolytische und/oder CGTase-Aktivität aufweisende Proteine dar, deren Aminosäuresequenz zu der in SEQ ID NO. 2 angebenen Aminosäuresequenz hinreichende Ähnlichkeit, insbesondere in den Teilbereichen des maturen Proteins und ganz besonders in den Domänen A, B und C aufweisen. Hiervon abgeleitete Proteinfragmente, Deletionsvarianten, Insertionsvarianten, Chimäre, Derivate oder immunologisch verwandte Proteine oder Derivate stellen ebenfalls Lösungen der der Erfindung zugrundegelegenen Aufgabe dar, sofern sie über eine amylolytische und/oder CGTase-Aktivität verfügen.

Vorzugsweise werden sie von einer natürlichen Quelle, vorzugsweise von einem Mikroorganismus und zunehmend bevorzugt von einem Bakterium, von einem gram-positiven Bakterium, von einem der Gattung Bacillus, von einem alkaliphilen Bacillus, von einem der Spezies *Bacillus agaradherens* und insbesondere von *Bacillus agaradherens* (DSM 9948) gebildet.

Weitere Lösungen der Aufgabe stellen die für diese Proteine, beziehungsweise Derivate codierenden Nukleinsäuren dar, die zu der unter SEQ ID NO.1 angebenen Nukleotidsequenz eine hinreichende Ähnlichkeit aufweisen, vorzugsweise solche, die für die oben erwähnten Proteine oder Derivate codieren.

Weitere Lösungen der Aufgabe bestehen in Organismen, die solche Proteine oder Derivate natürlicherweise bilden oder dafür codierende Nukleinsäuren enthalten; hierbei sind Mikroorganismen, Bakterien, gram-positive Bakterien, Bacilli, alkaliphile Bacilli, *Bacillus agaradherens* und *B. agaradherens* (DSM 9948) zunehmend bevorzugt; in Vektoren, die entsprechende Nukleinsäurebereiche enthalten, vorzugsweise Klonierungs- und/oder Expressionsvektoren; in Zellen, die derartige Nukleinsäuren oder Vektoren enthalten, vorzugsweise solchen, die sie exprimieren können; hierzu gehören sowohl prokaryontische als auch eukaryontische Zellen, bei den prokaryontischen vorzugsweise solche, die das gebildete Protein oder Derivat ins umgebende Medium sekretieren, hierunter besonders solche der Species *Bacillus licheniformis, Bacillus amyloliquefaciens, Bacillus subtilis* oder *Bacillus alcalophilus* oder gram-negative Bakterien, und hierunter besonders bevorzugt solche der Gattung Escherichia, bevorzugt der Spezies *Escherichia coli* oder *Klebsiella,* und besonders bevorzugt die Stämme *E. coli* JM 109, *E. coli* DH 100B, *E. coli* DH 12S oder *Klebsiella planticola* (Rf); unter den eukaryontischen sind solche bevorzugt, die das Protein oder Derivat posttranslational modifizieren.

Diese Erfindung umfaßt darüberhinaus alle, vorzugsweise alle mikrobiologischen Verfahren zur Herstellung der betreffenden Proteine oder Derivate.

Die Lösung einer Teilaufgabe und damit einen weiteren Aspekt der vorliegenden Erfindung stellen Wasch- oder Reinigungsmittel dar, die von den erfindungsgemäßen Proteinen oder Derivaten gekennzeichnet sind, besonders in geeigneten Konzentrationsbereichen; vorzugsweise solche, die weitere Amylasen, besonders α-Amylasen, weitere Enzyme wie insbesondere eine oder mehrere Proteasen, Lipasen, Oxidoreduktasen, Hemicellulasen und/oder Cellulasen oder Cyclodextrine enthalten; solche, die mehr als eine Phase aufweisen, die, wenn sie fest sind, mindestens zwei verschiedene feste Komponenten, insbesondere Pulver, Granulate oder Extrudate, in insgesamt loser Form miteinander vermischt enthalten; die kompaktiert sind; bei denen wenigstens eine der Phasen ein Amylase-sensitives Material, insbesondere Stärke enthält oder von diesem zumindest teilweise umgeben oder beschichtet ist; die flüssig, gelförmig oder pastös sind und bei denen das enthaltene Protein und/oder wenigstens eines der enthaltenen Enzyme und/oder wenigstens eine der sonstigen enthaltenen Komponenten einzeln oder zusammen mit anderen Bestandteilen verkapselt vorliegen, bevorzugt in Mikrokapseln, besonders bevorzugt in solchen aus einem Amylase-sensitiven Material; und/oder bei denen die amylolytische und/oder CGTase-Aktivität durch einen der sonstigen Bestandteile des Mittels modifiziert, insbesondere stabilisiert und/oder in ihrem Beitrag zur Wasch-, beziehungsweise Reinigungsleistung des Mittels gesteigert wird.

Weitere Lösungen sind Verfahren zur Reinigung von Textilien oder von harten Oberflächen, bei denen in wenigstens einem der Verfahrensschritte ein erfindungsgemäßes amylolytisches und/oder CGTase-Aktivität aufweisendes Protein oder ein entsprechendes Mittel, vorzugsweise in den geeigneten Konzentrationsbereichen aktiv wird; entsprechende Verwendungen, vorzugsweise in entsprechenden Konzentrationen, besonders in einer Geschirrspülmaschine oder einer Waschmaschine; weitere Lösungen sind die Verwendung eines erfindungsgemäßen Proteins oder Derivats allein oder zusammen mit mindestens einem anderen reinigungsaktiven oder die Reinigungswirkung unterstützenden Wirkstoff in einem aus mehr als einer Phase bestehenden Wasch- oder Reinigungsmittel zur Aktivierung der eigenen oder anderer Phasen; oder bei Wasch- oder Reinigungsmitteln mit verkapselten Inhaltsstoffen zur Freisetzung der Inhaltsstoffe.

Als weitere Lösungen sind technische Einsatzmöglichkeiten für das erfindungsgemäße amylolytische und/oder CGTase-Aktivität aufweisende Protein oder Derivat: deren Einsatzgebiete liegen unter anderem in der Behandlung von Rohmaterialien oder Zwischenprodukten in der Textilherstellung, insbesondere dem Entschlichten von Baumwolle oder dem Entfernen von Stärke-oder Stärke-ähnlichen Schutzschichten von technischen Zwischenprodukten; in der Stärkeverflüssigung, insbesondere der Ethanolproduktion; in der Herstellung oder Modifizierung von linearen und/oder kurzkettigen Oligosacchariden; in der Aufnahme oder Freisetzung von niedermolekularen Verbindungen in, beziehungsweise aus Polysaccharidträgern, insbesondere Cyclodextrinen, vor allem zur Stabilisierung von chemischen Verbindungen während ihrer Herstellung oder Verarbeitung; in der Herstellung oder als Bestandteil von Kosmetika oder Pharmaka, weshalb auch entsprechende Kosmetika und Pharmaka beansprucht werden, die dadurch gekennzeichnet sind, daß sie ein entsprechendes Protein oder Derivat enthalten oder wenigstens ein Bestandteil unter Einsatz eines solchen Proteins hergestellt worden ist; in der Verwendung eines erfindungsgemäßen Proteins oder Derivats zur Herstellung von Lebensmitteln, Lebensmittelbestandteilen, Tierfutter und/oder Tierfutterbestandteilen; in der Papierrestaurierung; in der Herstellung oder Auflösung Stärke oder ähnliche Verbindungen enthaltender Klebeverbindungen und damit besonders in temporären Klebeverfahren.

Unter einem **Protein** ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet.

Unter einem **Enzym** ist im Sinne der vorliegenden Anmeldung ein Protein zu verstehen, das eine bestimmte biochemische Funktion ausübt. Unter **amylolytischen Proteinen** oder Enzymen mit amylolytischer Funktion sind solche zu verstehen, die α-1,4-glykosidische Bindungen von Polysacchariden hydrolysieren, insbesondere solche, die im Inneren der Polysaccharide liegen, und deshalb auch als α-1,4-Amylasen (E.C. 3.2.1.1) bezeichnet werden können. **CGTasen** sind solche α-Amylasen, die in einem analogen Mechanismus keine Hydrolyse sondern am Substrat intramolekulare Transglykosylierungen katalysieren, wodurch die stärkeähnlichen Polymere unter Bildung von Cyclodextrinen abgebaut werden.

Zahlreiche Proteine werden als sogenannte **Präproteine**, also zusammen mit einem **Signalpeptid** gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne die zunächst vorhandenen N-terminalen Aminosäuren ausübt. Die native CGTase aus *Bacillus agaradherens* (DSM 9948), beispielsweise, ist, wie in SEQ ID NO.2 gezeigt ist, 713 Aminosäuren lang. Wie in den Anwendungsbeispielen ausgeführt ist, umfaßt das Signalpeptid dieses Enzyms ca. 34 Aminosäuren, so daß sich für das mature Enzym eine Länge von ca. 679 Aminosäuren ergibt.

Unter **Nukleinsäuren** sind im Sinne der vorliegenden Anmeldung die natürlicherweise aus Nukleotiden aufgebauten, als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure **DNA** für molekularbiologische Arbeiten bevorzugt. Demgegenüber wird für die Realisierung der Erfindung in natürlicher Umgebung, wie beispielsweise in einer exprimierenden Zelle, eine **RNA** gebildet, weshalb erfindungswesentliche RNA-Moleküle ebenfalls Ausführungsformen der vorliegenden Erfindung darstellen.

Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann **(Degeneriertheit des genetischen Codes).** Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden, und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als **Gen** bezeichnet.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als **Mutationen** bezeichnet. Je nach Art der Änderung kennt man beispielsweise **Deletions-, Insertions-**oder **Substitutionsmutationen** oder solche, bei denen verschiedene Gene oder Teile von Genen miteinander fusioniert **(shuffling)** werden; dies sind **Genmutationen.** Die zugehörigen Organismen werden als **Mutanten** bezeichnet. Die von mutierten Nukleinsäuren abgeleiteten Proteine werden als **Varianten** bezeichnet. So führen beispielsweise Deletions-, Insertions- Substitutionsmutationen oder Fusionen zu deletions-, insertions- substitutionsmutierten oder Fusionsgenen und auf Proteinebene zu entsprechenden **Deletions-,** Insertions- oder **Substitutionsvarianten,** beziehungsweise **Fusionsproteinen.**

Unter **Fragmenten** werden alle Proteine oder Peptide verstanden, die kleiner sind als natürliche Proteine oder als solche Proteine, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder amylolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter **chimären** oder **hybriden Proteinen** sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder **Fusionsmutagenese** genannt. Der Sinn einer solchen Fusion kann beispielsweise darin bestehen, mithilfe des heranfusionierten Proteinteils eine bestimmte enzymatische Funktion herbeizuführen oder zu modifizieren.

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

**lnversionsmutagenese,** also eine partielle Sequenzumkehrung, kann als Sonderform sowohl der Deletion, als auch der Insertion angesehen werden. Dasselbe gilt für eine von der ursprünglichen Aminosäureabfolge abweichende-Neugruppierung verschiedener Molekülteile. Sie kann sowohl als Deletionsvariante, als Insertionsvariante, als auch als Shuffling-Variante des ursprünglichen Proteins angesehen werden.

Unter **Derivaten** werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise um andere Proteine handeln, die beispielsweise über bifunktionelle chemische Verbindungen an erfindungsgemäße Proteine gebunden werden. Ebenso ist unter Derivatisierung die kovalente Bindung an einen makromolekularen Träger zu verstehen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Unter **Vektoren** werden im Sinne der vorliegenden Erfindung aus Nukleinsäuren bestehende Elemente verstanden, die als kennzeichnenden Nukleinsäurebereich ein bestimmtes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten **Klonierungsvektoren,** und andererseits denen, die die Funktion erfülllen, das interessierende Gen in der Wirtszelle zu realisieren, das heißt, die Expression und damit die Biosynthese des betreffenden Proteins zu ermöglichen. Diese Vektoren werden als **Expressionsvektoren** bezeichnet.

Durch Vergleich mit bekannten Enzymen, die beispielsweise in allgemein zugänglichen Datenbanken hinterlegt sind, lassen sich aus der Aminosäure- oder Nukleotid-Sequenz charakteristische Molekülteile wie beispielsweise Strukturelemente oder die enzymatische Aktivität eines betrachteten Enzyms folgern. Solch ein Vergleich geschieht dadurch, daß ähnliche Abfolgen in den Nukleotid- oder Aminosäuresequenzen der betrachteten Proteine einander zugeordnet werden. Dies nennt man **Homologisierung.** Eine tabellarische Zuordnung der betreffenden Positionen wird als **Alignment** bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben. Eine Zusammenstellung aller in den verglichenen Sequenzen übereinstimmenden Positionen wird als Consensus-**Sequenz** bezeichnet.

Solch ein Vergleich erlaubt auch eine Aussage über die **Ähnlichkeit** oder **Homologie** der verglichenen Sequenzen zueinander. Diese wird in Prozent Identität, das heißt dem Anteil der identischen Nukleotide oder Aminosäurereste an denselben Positionen widergegeben. Ein weiter gefaßter Homologie-Begriff bezieht auch konservierte Variationen, also Aminosäuren mit ähnlicher chemischer Aktivität in diesen Wert mit ein: so sind unter konservierten Austauschen solche zu verstehen, bei denen beispielsweise eine aliphatische Aminosäure (Gly, Ala, Val, Leu, Ile) gegen eine andere aliphatische Aminosäure ausgetauscht worden ist oder eine aromatische (Phe, Tyr, Trp) gegen eine andere aromatische oder, allgemein formuliert, eine Aminosäure gegen eine Aminosäure ausgetauscht ist, die, gegebenenfalls unter Berücksichtigung der jeweiligen chemischen Umgebung innerhalb des Proteins, eine ähnliche chemische Aktivität auszuüben vermag. Es ist dann von Prozent Ähnlichkeit die Rede. Solche Aussagen können über ganze Proteine oder Gene oder nur über einzelne, jeweils einander zuzuordnende Bereiche getroffen werden.

**Homologie- oder Ähnlichkeitsangaben** können sich sowohl auf die Aminosäuresequenz als auch auf die Nukleotidsequenz beziehen. Für letztere entfällt allerdings der weiter gefaßte Begriff an "konservierten" Austauschen und man spricht nur von dem Prozentsatz an Identität.

**Homologe Bereiche** von verschiedenen Proteinen sind zumeist solche mit gleichen Strukturelementen und/oder Funktionen, die sich durch Übereinstimmungen in der primären Aminosäuresequenz erkennen lassen. Sie geht bis zu völligen Identitäten in kleinsten Bereichen, sogenannten **Boxen,** die nur wenige Aminosäuren umfassen und meist für die Gesamtaktivität essentielle **Funktionen** ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes.

Die **enzymatische Aktivität** kann durch andere Bereiche des Proteins, die nicht an der eigentlichen Reaktion (beispielsweise dem Lösen und/oder Knüpfen chemischer Bindungen im Substrat) beteiligt sind, qualitativ oder quantitativ modifiziert werden. Dies betrifft beispielsweise die Enzymstabilität, die Aktivität, die Reaktionsbedingungen oder die Substratspezifität.

Im Rahmen der vorliegenden Erfindung ist der **Begriff amylolytische Aktivität** also weit auszulegen: sowohl die allein auf die Aminosäuren des katalytisch aktiven Zentrums zurückzuführende, einer α-Amylase entsprechende Aktivität, als auch jede von anderen Teilbereichen des Proteins ausgeübte Hilfsfunktion zur Hydrolyse, beziehungsweise Spaltung von α-1,4-glycosidischen Bindungen sollen erfindungsgemäß als amylolytische Funktion aufgefaßt werden, sofern es sich bei der beeinflußten Reaktion selbst um eine Amylase-und/oder CGTase-Aktivität handelt. Der Begriff der amylolytischen und/oder CGTase-Funktion umfaßt auch allein solche modifizierenden Funktionen. Denn einerseits ist nicht genau bekannt, welche Aminosäurereste des erfindungsgemäßen Proteins tatsächlich die Hydrolyse, beziehungsweise Spaltung und Neuknüpfung der Bindungen katalysieren, und andererseits können nicht von vornherein bestimmte Einzelfunktionen definitiv von der Beteiligung an der Katalyse ausgenommen werden. Zu den Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische, beziehungsweise glycolytische Aktivität. Dabei kann es sich beispielsweise auch um die Ausbildung eines Strukturelements handeln, das fern vom aktiven Zentrum liegt, oder um ein Signalpeptid, dessen Funktion die Ausschleusung des gebildeten Proteins aus der Zelle und/oder dessen korrekte Faltung betrifft und ohne das *in vivo* in der Regel kein funktionsfähiges Enzym gebildet wird.

Insbesondere den in CGTasen vorhandenen Domänen C und/oder D werden im Sinne der vorliegenden Erfindung ebenfalls amylolytische und/oder CGTase-Funktionen zugeschrieben, weil sie mechanistisch eine spezielle Art der Spaltung der α-(1,4)-glycosidischen Bindungen katalysieren, nämlich die intramolekulare Transglycosylierung. Reaktionsprodukte sind neben Cyxclodextrinen entsprechend abgebaute Stärken und/oder stärkeähnliche Polymere, wie sie beispielsweise auch durch die von α-Amylasen katalysierte Hydrolyse erhalten werden können.

Der Begriff der **CGTase-Aktivität** ist aus den gleichen Gründen, wie oben für die amylolytische Aktivität dargelegt, ebenfalls im weitesten Sinne auszulegen und bezieht sich auch auf kleinste Molekülteile, die in in irgendeiner Form Einfluß auf die unter Abbau der stärkeähnlichen Verbindung erfolgende Cyclodextrinsynthese nehmen. In diesem Sinne muß insbesondere auch die Aktivität der Domänen A, B und C als CGTase-Aktivität angesehen werden.

Unter der **Leistung** eines Enzyms wird dessen Wirksamkeit im jeweils betrachteten technischen Bereich verstanden. Diese basiert auf der eigentlichen enzymatischen Aktivität, hängt darüberhinaus aber von weiteren, für den jeweiligen Prozeß relevanten Faktoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien. So wird beispielsweise bei der Untersuchung, ob sich ein Enzym für den Einsatz in Wasch- oder Reinigungsmitteln eignet, dessen **Beitrag** zur **Wasch- oder Reinigungsleistung** eines mit weiteren Bestandteilen formulierten Mittels betrachtet. Für verschiedene technische Anwendungen kann ein Enzym u.a. über an sich bekannte molekularbiologische Techniken, insbesondere die oben genannten weiterentwickelt und optimiert, das heißt in seiner Leistung, beispielsweise in seinem Beitrag zur Wasch- oder Reinigungsleistung eines entsprechenden Mittels verbessert werden.

Das erfindungsgemäße Enzym einer besonders bevorzugten Ausführungsform kann durch Isolierung aus dem Kulturüberstand von *Bacillus agaradherens* DSM-9948 gewonnen werden, also dem Organismus, von dem es natürlicherweise gebildet wird. Dieser ist gemäß dem Budapester Vertrag über die internationale Anerkennung der Hinterlegung von Mikroorganismen vom 28. April 1977 bei der Deutschen Sammlung von Mikroorganismen und Zellkulturen GmbH in Braunschweig (DSMZ) hinterlegt worden. Er trägt dort die Bezeichnung C/M 1-6 und die Eingangsnummer DSM 9948 (DSM ID 94-759). Die maßgeblichen Angaben über die Merkmale dieses biologischen Materials, wie sie von der DSMZ bei der Hinterlegung bestimmt worden sind, werden in folgender Tabelle 1 zusammengestellt.

### Tabelle 1: Mikrobiologische Eigenschaften des Bacillus agaradherens (DSM 9948) (Bestimmt von der DSMZ am 20.4.1995)

| **Eigenschaft** | | **Ergebnis** |
|---|---|---|
| Gram-Reaktion | | positiv |
| Zellform | | Stäbchen |
| | Breite [µm] | 0,6-0,8 |
| | Länge [µm] | 2,5-5,0 |
| Sporen | | positiv |
| | geschwollenes Sporangium | positiv |
| Anaerobes Wachstum | | positiv |
| Maximale Temperatur | | |
| | Wachstum positiv bei °C | 45 |
| | Wachstum negativ bei °C | 50 |
| Wachstum in | | |
| | Medium pH 5,7 | negativ |
| | NaCl 2 % | positiv |
| | NaCl 5 % | positiv |
| | NaCl 7 % | positiv |
| | NaCl 10 % | positiv |
| Säure aus | | |
| | D-Glucose | negativ |
| | L-Arabinose | negativ |
| | D-Xylose | negativ |
| | D-Mannit | negativ |
| | D-Fructose | negativ |
| Gas aus Glucose | | negativ |
| Lecithinase | | negativ |
| Hydrolyse von | | |
| | Stärke | positiv |
| | Gelatine | positiv |
| | Casein | positiv |
| Verwertung von | | |
| | Citrat | positiv |
| | Propionat | positiv |
| Abbau von Tyrosin | | negativ |
| NO₂ aus NO₃ | | positiv |
| Indol | | negativ |
| Phenylalanindesaminase | | negativ |
| Ergebnis | | Stamm [...] = *Bacillus spec.,* Gruppe 6 |

Wie über diese Charakterisierung hinaus nun überraschenderweise festgestellt werden konnte, weist das von diesem Stamm produzierte amylolytische Enzym Eigenschaften auf, die es für verschiedene Einsatzmöglichkeiten, darunter als aktive Komponente in Wasch- und Reinigungsmitteln prädestinieren. Es läßt sich, wie anhand der Beispiele der vorliegenden Anmeldung nachvollzogen werden kann, folgendermaßen biochemisch charakterisieren:

Das native amylolytische und CGTase-Aktivität aufweisende Enzym des Stammes *Bacillus agaradherens* (DSM 9948) weist in der denaturierenden SDS-Polyacrylgelelektrophorese ein apparentes Molekulargewicht von 89 kD auf (Beispiel 7), während sich aus der 713 Aminosäuren umfassenden Proteinsequenz (SEQ ID NO. 2) ein Molekulargewicht von 88,9 kD, beziehungsweise ohne Signalpeptid von 76,4 kD ableiten läßt (Beispiel 3). Andere CGTasen, beispielsweise aus *Bacillus stearothermophilus* oder *Thermoanaerobacter thermosulfurogenes* weisen vergleichbare Werte auf, nämlich bei Größen von 710 und 711 Aminosäuren Molekulargewichte von 78,9, beziehungsweise 78,4 kD (vergleiche auch: Nakamura et al., FEBS-Letters, 296 (1992), S. 37-40). Gemäß isoelektrischer Fokussierung liegt der isoelektrische Punkt des nativen Enzyms aus *B. agaradherens* (DSM 9948) bei 6,0 (Beispiel 7).

In einem Aktivitätstest, der speziell-auf die Cyclodextrinbildung gerichtet ist (Beispiel 8), weist bereits der nicht aufgereinigte Kulturüberstand von B. *agaradherens* (DSM 9948) eine CGTase-Aktivität auf. Gleichzeitig verfügt es über eine meßbare Amylase-Aktivität (Beispiel 9). Darüberhinaus verfügt es bis wenigstens 50°C über eine weitgehende Temperaturstabilität, bei pH-Werten zwischen 5 und 12 über weitgehende Stabilität gegenüber Schwankungen des pH-Werts und außerdem eine gewisse Stabilität gegenüber Tensiden und Proteasen (Beispiel 9).

Die 2142 bp umfassende Nukleotidsequenz dieses Enzyms wird im Sequenzprotokoll unter der Bezeichnung SEQ ID NO. 1 angegeben. Sie steht somit beispielsweise für Weiterentwicklungen über an sich bekannte molekularbiologische Methoden zur Verfügung. Die 713 Aminosäure umfassende Aminosäuresequenz dieses Enzyms wird im Sequenzprotokoll unter der Bezeichnung SEQ ID NO. 2 angegeben.

Proteine, die zu diesem eine hinreichende Ähnlichkeit aufweisen, stellen Ausführungsformen der vorliegenden Erfindung dar.

Gegenstand der vorliegenden Anmeldung sind somit amylolytische und/oder CGTase-Aktivität aufweisende Proteine mit einer Aminosäuresequenz, die zu der in SEQ ID NO. 2 angegebenen Aminosäuresequenz zunehmend bevorzugt mindestens zu 65 %, 67,5 %, 70 %, 72,5 %, 75 %, 77,5 %, 80 %, 82,5 %, 85 %, 87,5 %, 90 %, 92,5 %, 95 %, 96 %, 97 %, 98 %, 99 % oder zu 100 % identisch ist.

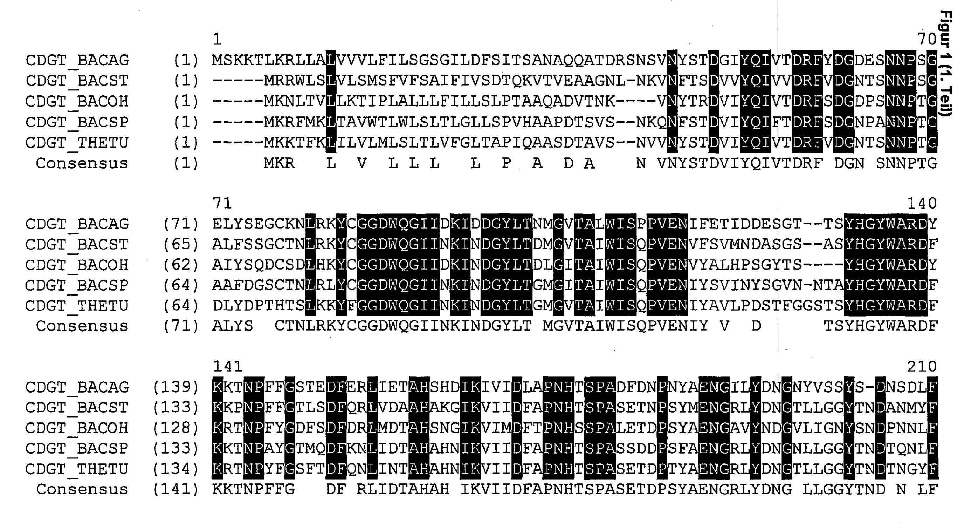

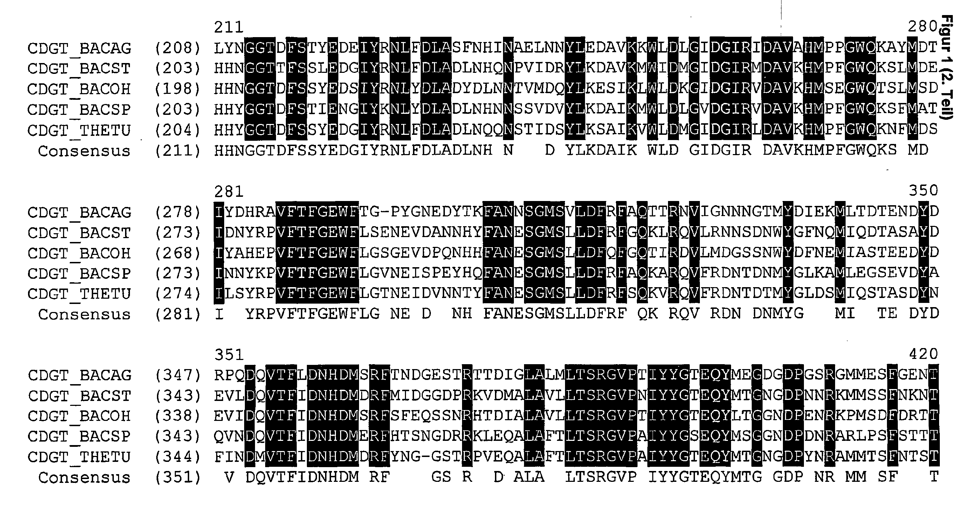

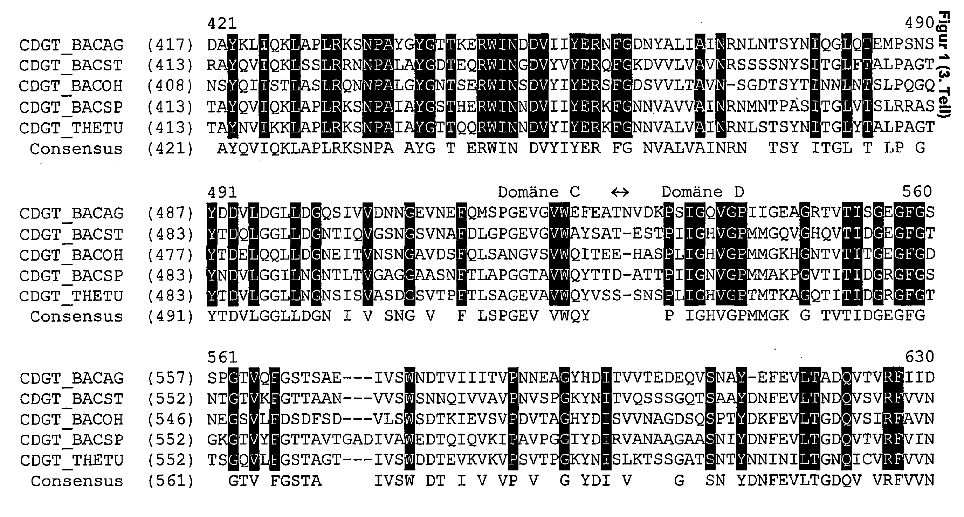

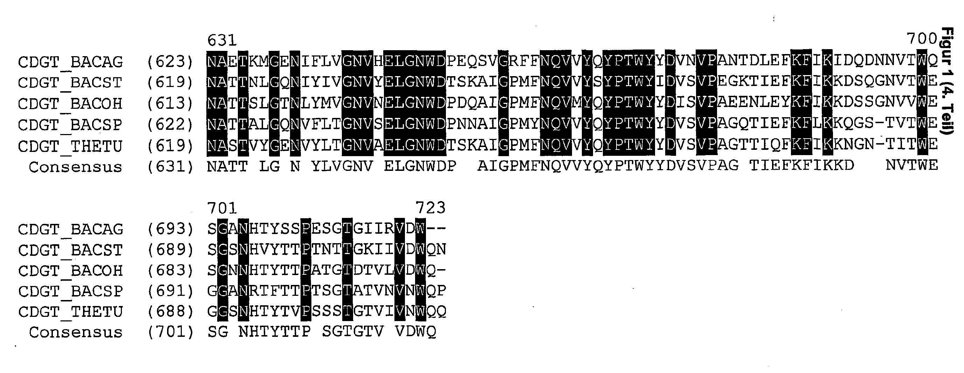

Denn bei den beiden nächstähnlichen, bis zum 25.5.2000 bekannten Proteinen handelt es sich gemäß einer Datenbankrecherche (Beispiel 4) um die CGTasen aus *Bacillus stearothermophilus* (Q9ZAQ0) und *Thermoanaerobacter thermosulfurogenes* (P 26827; beide Bezeichnungen gemäß ihrer Listung in der Swiss-Prot-Datenbank; Geneva Bioinformatics, Genf, Schweiz; http://www.genebio.com/sprot.html). Beide weisen zur erfindungsgemäßen CGTase aus *B. agaradherens* (DSM 9948) Sequenz-Homologien von 56 % Identität auf Proteinebene auf. Bezieht man die konservierten Aminosäure-Austausche mit ein, ergeben sich Werte von jeweils 68 %. Das erfindungsgemäße Protein ist über die Ähnlichkeit der homologen Bereiche, insbesondere den Domänen A, B, C, D und E eindeutig als CGTase charakterisiert. Repräsentative verwandte Proteine werden im Alignment der Figur 1 vorgestellt. Das zu den Domänen A bis C nächstähnliche Enzym, eine CGTase, verfügt zu diesen über eine Homologie von 60,1 % Identität auf Aminosäure-Ebene. Auf DNA-Ebene verfügt das nächstähnliche Enzym über eine Homologie von 61,1 % Identität.

Die Homologiewerte zu bekannten α-Amylasen liegen deutlich darunter. Beipielsweise zu der unter dem Namen Termamyl^{®} erhältlichen α-Amylase aus *Bacillus licheniformis* besteht über die Domänen A, B und C, das heißt den Positionen 35 bis 526 gemäß der SEQ ID NO. 2 der vorliegenden Anmeldung eine Homologie von lediglich 30 % Identität.

Bevorzugt sind jeweils solche Abweichungen der angegebenen Sequenzen, die konservierte Aminosäure-Austausche darstellen.

Unter den innerhalb des oben angegebenen Ähnlichkeitsbereich liegenden Varianten sind insbesondere solche bevorzugt, die hinsichtlich der vorgesehenen Einsatzmöglichkeiten optimierte Eigenschaften aufweisen. Solche sind, wie einleitend dargestellt, nach an sich bekannten, vorzugsweise molekularbiologischen Methoden herstellbar. Beispielsweise wäre es auch möglich, in Anwendung der Lehre aus der Anmeldung WO 94/18314 Methionin-, Tryptophan-, Cystein- und/oder Tyrsosinreste erfindungsgemäßer Proteine zu deletieren und/oder gegen weniger leicht oxidierbare Aminosäurereste zu substituieren. Dadurch können die Oxidationsstabilität, das pH-Aktivitätsprofil und/oder die Thermostabilität verbessert werden. Weiterentwicklungen über gezielte Punktmutagenese können sich beispielsweise auch an den Anmeldungen WO 99/09183 und WO 99/23211 orientieren.

Der beanspruchte Homologiebereich gilt für die Gesamtlänge des Proteins, das heißt für die Positionen 1 bis 713, zunehmend bevorzugt aber für Teilproteine, die sich von den Positionen 35 bis 713 oder 35 bis 526 erstrecken.

Diesen Teilsequenzen, das heißt den maturen Proteinen, gilt deshalb besonderes Interesse, weil die ersten 34 Aminosäuren, wie aus der Aminosäuresequenz geschlossen werden kann, das für die Produktion wichtige Signalpeptid darstellen.

Dieses wird vermutlich *in vivo* nach der Ausschleusung abgespalten, so daß die eigentlichen amylolytischen, beziehungsweise CGTase-Aktivitäten von den übrigen Teilen des Proteins ausgeübt werden. Somit sind die Aminosäuren 1 bis 34 für die enzymatische Aktivität im engeren Sinne wahrscheinlich nicht von Bedeutung, wohl aber für die Produktion, weshalb sie nicht aus dem Schutzbereich der vorliegenden Erfindung ausgeschlossen werden, die übrigen Teile aber bevorzugt sind.

Verwandte, hinreichend ähnliche Proteine aus anderen Organismen können Schwankungen in der Größe des Signalpeptides aufweisen. Längen von 20 bis 40 Aminosäuren sind nicht ungewöhnlich. Somit wird mit den genannten Positionen 1 bis 34 auf die Teilsequenz abgehoben, die wahrscheinlich als das Signalpeptid anzusehen ist. Sollte sich also herausstellen, daß das Signalpeptid ein anderes ist, so gilt das oben gesagte analog für das tatsächliche Signalpeptid, beziehungsweise die tatsächlichen maturen Proteine.

Insbesondere gilt jedem Molekül oder Molekülteil, der den Positionen 35 bis 492 und somit den Domänen A, B und C der CGTase aus *Bacillus agaradherens* (DSM 9948) entspricht, großes Interesse. Denn allein dieser Teil kann ohne die übrigen Bereiche eine amyloytische Funktion ausüben. Dies kann für zahlreiche Anwendungen ausreichend sein, und sich somit der Verzicht auf die übrigen Bereiche günstiger, beispielsweise kostengünstiger in der Herstellung des Proteins darstellen. Das ist insbesondere dann der Fall, wenn gegenüber der Cyclodextrin-Synthese die Hydrolyse von stärkeähnlichen Verbindungen im Vordergrund steht.

Im Alignment der Figur 1 ist der Übergangsbereich der Domänen C und D durch einen Doppelpfeil nahe der Position 530 gekennzeichnet; die Aminosäurefolge VWE (Positionen 524 bis 526 gemäß der SEQ ID NO.2) wird noch der Domäne C zugerechnet, während die Aminosäurefolge PSI (Positionen 535 bis 537 gemäß der SEQ ID NO. 2) bereits der Domäne D zugerechnet wird.

Der Ähnlichkeitsbereich umfaßt zunehmend bevorzugt auch alle amylolytischen und/oder CGTase-Aktivität ausübenden Proteine, die sich von einer Nukleotidsequenz ableiten, die zu der in SEQ ID NO. 1 angegebenen Nukleotidsequenz zunehmend bevorzugt mindestens zu 65 %, 67,5 %, 70 %, 72,5 %, 75 %, 77,5 %, 80 %, 82,5 %, 85 %, 87,5 %, 90 %, 92,5 %, 95 %, 96 %, 97%, 98 %, mindestens zu 99 % oder zu 100 % identisch ist.

Dies gilt entsprechend dem oben Gesagten insbesondere für jene Teilbereiche des Proteins, die den Aminosäuren 35 bis 713 oder die Positionen 35 bis 526 gemäß der SEQ ID NO. 2 entsprechen und für die entsprechend bevorzugten Austausche in den einzelnen Positionen.

Weitere Ausführungsformen der vorliegenden Erfindung sind amylolytische und/oder CGTase-Aktivität aufweisende Proteinfragmente oder durch Deletionsmutation erhältliche Proteine innerhalb des oben beanspruchten Ähnlichkeitsbereichs.

Unter erfindungsgemäßen Fragmenten werden im Sinne der vorliegenden Erfindung alle Proteine oder Peptide verstanden, die kleiner sind als jene Proteine, die denen von SEQ ID NO. 1 oder SEQ ID NO. 2 entsprechen, zu ihnen in den entsprechenden Teilsequenzen aber hinreichend homolog sind. Sofern sie eine amylolytische und/oder CGTase-Aktivität aufweisen, stellen sie Ausführungsformen der vorliegenden Erfindung dar. Das betrifft beispielsweise solche Fragmente, die zur Komplexierung eines Substrats oder zur Ausbildung eines für die Hydrolyse erforderlichen Strukturelements beitragen. Bei den Fragmenten kann es sich beispielsweise um einzelne Domänen handeln oder um Bruchstücke, die nicht mit den Domänen übereinstimmen. Solche Fragmente können kostengünstiger herzustellen sein, bestimmte eventuell nachteilige Charakteristika des Ausgangsmoleküls nicht mehr besitzen, wie möglicherweise einen aktivitätssenkenden Regulationsmechanismus, oder ein günstigeres Aktivitätsprofil entfalten. Derartige Proteinfragmente können auch nicht-biosynthetisch, sondern beispielsweise chemisch hergestellt werden. Die chemische Synthese ist beispielsweise dann vorteilhaft, wenn im Anschluß an die Synthese chemische Modifikationen vorgenommen werden sollen.

Den Fragmenten sind ihrer prinzipiellen Gleichartigkeit wegen auch durch Deletionsmutation erhaltene Proteine zuzuordnen. Die Deletionsmutagenese ist zur Entfernung inhibierender Bereiche besonders sinnvoll. Im Ergebnis können mit den Deletionen sowohl eine Spezialisierung als auch eine Erweiterung des Anwendungsbereichs des Proteins einhergehen. Sofern dadurch eine im weitesten Sinne amylolytische und/oder CGTase-Funktion aufrechterhalten, modifiziert, spezifiziert oder auch erst erreicht wird, handelt es sich bei den durch Deletionsmutation erhaltenen Proteinen wie bei den Fragmenten um erfindungsgemäße Proteine; einzige zusätzliche Voraussetzung dafür ist, daß die noch vorhandenen homologen Teilsequenzen innerhalb des oben bereits angegeben Ähnlichkeitsbereichs zu den Sequenzen SEQ ID NO. 1 und SEQ ID NO. 2 liegen.

Als natürlicherweise gebildete Fragmente, beziehungsweise deletionsmutierte Proteine kann man auch die von Präproteinen durch Abspaltung der N-terminalen Aminosäuren erhältlichen Proteine und Signalpeptide ansehen. Solch ein Spaltungsmechanismus kann beispielsweise dazu verwendet werden, um mithilfe bestimmter Sequenzbereiche, die von Signalpeptidasen erkannt werden, in rekombinanten Proteinen spezifische Spaltstellen vorzugeben. Somit können *in vitro* Aktivierung und/oder Deaktivierung erfindungsgemäßer Proteine vorgenommen werden.

Besonderes Interesse gilt den Teilen erfindungsgemäßer Proteine, die eine oder mehrere der Domänen A, B und C enthalten, die eine der typischen α-Amylasen vergleichbare stärkespaltende Aktivität ausüben. Insbesondere diese Fragmente ohne eigentliche CGTase-Funktion fallen in den Schutzbereich der vorliegenden Erfindung. Denn manche Anwendungen, wie beispielsweise die bloße Stärke-Hydrolyse oder die als aktiver Inhaltsstoff von Wasch- oder Reinigungsmitteln erfordern nicht unbedingt die gleichzeitige Synthese von Cyclodextrinen.

Umgekehrt können insbesondere die Domänen D und E zur Synthese oder Modifizierung von Stärke oder stärkeähnlichen Polysacchariden oder von Cyclodextrinen eingesetzt werden. Dafür kann es sinnvoll sein, einzelne Teile aus den vorderen Domänen des erfindungsgemäßen Proteins zu deletieren.

Weitere Ausführungsformen der vorliegenden Erfindung sind amylolytische und/oder CGTase-Aktivität aufweisende, durch Insertionsmutation erhältliche oder chimäre Proteine, welche wenigstens in einem die amylolytische Aktivität und/oder die CGTase-Aktivität verleihenden Teil aus einem bisher beschriebenen Protein oder Fragment bestehen.

Erfindungsgemäße chimäre Proteine weisen im weitesten Sinne eine amylolytische und/oder CGTase-Aktivität auf. Diese kann von einem Molekülteil ausgeübt oder modifiziert werden, das sich von einem erfindungsgemäßen Protein herleitet und innerhalb des beanspruchten Ähnlichkeitsbereichs liegt. Die chimären Proteine können über ihre Gesamtlänge hinweg somit auch außerhalb des oben beanspruchten Bereichs liegen. Der Sinn einer solchen Fusion kann beispielsweise darin bestehen, mithilfe des heranfusionierten erfindungsgemäßen Proteinteils eine amylolytische oder die Hydrolyse oder Neuverknüpfung von α-1,4-glycosidischen Bindungen, die Cyclodextrinsynthese oder eine unterstützende Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können. Zur Realisierung der letztgenannten Alternative ist es beispielsweise möglich, posttranslational oder erst nach einem Aufreinigungsschritt durch eine gezielte proteolytische Spaltung eine einzelne chimäre Polypeptidkette in mehrere zu zerlegen.

So ist es beispielsweise in Anlehnung an WO 99/57250 und WO 99/57254 möglich, ein erfindungsgemäßes Protein oder Teile davon über peptidische oder nicht-peptidische Linker mit Bindungsdomänen aus anderen Proteinen zu versehen und dadurch die Hydrolyse des Substrats effektiver zu gestalten. Solche Konstrukte liegen dann innerhalb des Schutzbereichs der vorliegenden Erfindung, wenn sie amylolytische und/oder CGTase-Aktivitäten ausüben und die Teile des Konstrukts, die diese Funktion ausüben, zu den angegebenen erfindungsgemäßen Sequenzen hinreichend ähnlich sind. Ebenso können erfindungsgemäße Proteine beispielsweise auch mit Proteasen verknüpft werden, um eine Doppelfunktion auszuüben.

Die durch Insertionsmutation erhältlichen erfindungsgemäßen Proteine sind ihrer prinzipiellen Gleichartigkeit wegen den erfindungsgemäßen chimären Proteinen zuzuordnen. Hierher gehören auch Substitutionsvarianten, also solche, bei denen einzele Bereiche des Moleküls gegen Elemente aus anderen Proteinen ersetzt worden sind.

Der Sinn von Insertions- und Substitutionsmutagenese besteht wie bei der Hybridbildung darin, einzelne Eigenschaften erfindungsgemäßer Proteine mit denen anderer Proteine zu kombinieren. Es handelt sich dann um erfindungsgemäße, durch Insertions- oder Substitutionsmutation erhaltene Proteine oder chimäre Proteine, wenn die über ihre Homologie auf die Sequenzen SEQ ID NO. 1 oder SEQ IDNO. 2 zurückzuführenden Bereiche dem oben gesagten entsprechende Homologiewerte aufweisen und das Protein aufgrund dieser Bereiche eine im weitesten Sinne amylolytische und/oder CGTase-Funktion besitzt.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Weitere Ausführungsformen der vorliegenden Erfindung sind amylolytische und/oder CGTase-Aktivität aufweisende Derivate der bisher ausgeführten Proteine.

Darunter werden solche Proteine verstanden, die sich von Proteinen ableiten, die selbst im Sinne dieser Anmeldung amylolytische und/oder CGTase-Aktivität besitzen. Dies können beispielsweise solche Proteine sein, die nach ihrer Synthese modifiziert worden sind. Solche Derivatisierungen können beispielsweise biologisch erfolgen, etwa im Zusammenhang mit der Proteinbiosynthese über Prozessierung durch den produzierenden Wirtsorganismus. Sie können aber auch chemisch durchgeführt werden, etwa durch die chemische Umwandlung einer Seitenkette einer Aminosäure oder durch kovalente Bindung einer anderen Verbindung an das Protein. Dies können niedrigmolekulare Verbindungen oder Makromoleküle wie beispielsweise Polyethylenglycol handeln. Bei solch einer Verbindung kann es sich beispielsweise auch um andere Proteine handeln, die beispielsweise über bifunktionelle chemische Verbindungen an erfindungsgemäße Proteine gebunden werden.

Derartige Modifikationen können beispielsweise die Stabilität, Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Gekoppelte Polymere können beispielsweise auch die Allergenizität und/oder Immunogenizität des Enzyms herabsetzen und damit beispielsweise dessen Hautverträglichkeit erhöhen.

Weitere Ausführungsformen der vorliegenden Erfindung sind amylolytische und/oder CGTase-Aktivität aufweisende Proteine oder Derivate, die wenigstens eine antigene Determinante mit einem der oben beschriebenen Proteine oder Derivate gemeinsam haben.

Denn entscheidend für die enzymatischen Aktivitäten sind die Sekundärstrukturelemente eines Proteins und dessen dreidimensionale Faltung. So können in ihrer Primärstruktur deutlich voneinander abweichende Domänen räumlich weitgehend übereinstimmende Strukturen ausbilden und somit gleiches enzymatisches Verhalten ermöglichen. Solche Gemeinsamkeiten in der Sekundärstruktur werden üblicherweise als übereinstimmende antigene Determinanten von Antiseren oder reinen oder monoklonalen Antikörpern erkannt. Über immunchemische Kreuzreaktionen können somit einander ähnliche Proteine oder Derivate detektiert und zugeordnet werden. Deshalb werden in den Schutzbereich der vorliegenden Erfindung gerade auch solche Proteine oder Derivate einbezogen, die möglicherweise nicht über ihre Homologiewerte in der Primärstruktur, wohl aber über ihre immunchemische Verwandtschaft den oben definierten erfindungsgemäßen Proteinen oder Derivaten zugeordnet werden können.

Die bezeichneten Varianten sind über an sich bekannte Methoden erhältlich (siehe unten), insbesondere über molekularbiologische Methoden, wie sie beispielsweise in dem Handbuch von Fritsch, Sambrook und Maniatis "Molecular cloning: a laboratory manual", Cold Spring Harbour Laboratory Press, New York, 1989 beschrieben sind. Mit solchen können erfindungsrelevante Proteine weiter optimiert werden, beispielsweise durch Punktmutagenese oder durch Fusion mit Sequenzen aus anderen Genen. Der Einsatzbereich der erfindungsgemäßen Proteine kann auf diese Weise erweitert werden. Dies gilt beispielsweise für Proteine, die gegenüber Temperatur-Einflüssen, pH-Wert-Schwankungen, den Redox-Verhältnissen und/oder sonstigen Einflüssen, wie etwa Bleichmitteln oder denaturierenden Agentien, insbesondere Tensiden weniger empfindlich sind.

Amylolytische und/oder CGTase-Aktivität aufweisende Proteine oder Derivate, die dem bisher Gesagten entsprechen und aus natürlichen Quellen stammen, sind bevorzugte Ausführungsformen der vorliegenden Erfindung, insbesondere wenn sie aus Mikroorganismen erhältlich sind.

Dies können beispielsweise einzellige Pilze oder Bakterien sein. Denn sie lassen sich zumeist einfacher handhaben als vielzellige Organismen oder die von Vielzellern abgeleiteten Zellkulturen; obgleich diese für spezielle Ausführungsformen sinnvolle Optionen darstellen können und somit nicht grundsätzlich vom Erfindungsgegenstand ausgeschlossen sind.

Besonders bevorzugt sind solche aus gram-positiven Bakterien, weil diese keine äußere Membran besitzen und sekretierte Proteine somit unmittelbar ins umgebende Medium abgeben.

Ganz besonders bevorzugt sind solche aus gram-positiven Bakterien der Gattung Bacillus, weil diese als Produktionsorganismen mit einer besonders hohen Produktionsleistung in technischen Prozessen etabliert sind.

Unter denen aus den Bacillus-Species, wiederum, sind solche aus alkaliphilen Bacilli bevorzugt, insbesondere aus dem Stamm *Bacillus agaradherens* und ganz besonders *B. agaradherens* (DSM 9948). Denn aus diesem wurde die Ausführungsform des erfindungsgemäßen Enzyms ursprünglich erhalten, dessen zugehörige Sequenzen im Sequenzprotokoll angegeben sind und dessen enzymatische Charakteristika in den Beispielen beschrieben sind.

Bevorzugt sind jeweils solche Stämme, die das gebildete amylolytische und/oder CGTase-Aktivität ausübende Protein in das sie umgebende Medium abgeben. Es ist möglich, daß natürlich vorkommende Produzenten zwar ein erfindungsgemäßes Enzym herstellen können, dieses aber unter den zunächst ermittelten Bedingungen nur in geringem Maße exprimieren und/oder in das umgebende Medium abgeben. Sie unterfallen dennoch solange dem Schutzbereich der vorliegenden Erfindung, wie die Möglichkeit besteht, geeignete Umweltbedingungen oder niedermolekulare oder sonstige Faktoren experimentell zu ermitteln, unter deren Einwirken sie zu einer Produktion des erfindungsgemäßen Proteins angeregt werden können, die eine wirtschaftliche Nutzung sinnvoll erscheinen läßt. Solch ein Regulationsmechanismus kann für die biotechnologische Produktion gezielt eingesetzt werden, zum Beispiel zur Regulation der verantwortlichen Promotoren.

Je nach Gewinnung, Aufarbeitung oder Präparation eines Proteins kann es mit diversen anderen Stoffen vergesellschaftet sein, insbesondere wenn es aus natürlichen Produzenten dieses Proteins gewonnen worden ist. Es kann dann, aber auch unabhängig davon, mit bestimmten anderen Stoffen gezielt versetzt worden sein, beispielsweise zur Erhöhung seiner Lagerstabilität. Unter dem Begriff des erfindungsgemäßen Proteins sind deshalb zusätzlich auch alle Präparationen des eigentlichen erfindungswesentlichen Proteins zu verstehen. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine amylolytische Funktion entfaltet. Dies kann beispielsweise vom Faltungszustand des Proteins abhängig sein oder aus der reversiblen Bindung eines oder mehrer Begleitstoffe aus der Präparation oder aus einem sonstigen Kontrollmechanismus resultieren.

Die erfindungsgemäßen Proteine können vor allem während der Lagerung durch Stabilisatoren beispielsweise vor Denaturierung, Zerfall oder Inaktivierung, etwa durch physikalische Einflüsse, Oxidation oder Proteolyse geschützt werden. Vielfach werden auch sich ergänzende oder gegenseitig steigernde Kombinationen von Stabilisatoren verwendet.

Je nach Gewinnung, Aufarbeitung oder Präparation eines Proteins kann ein solches mit diversen anderen Stoffen vergesellschaftet sein. Es kann auch, beispielsweise zur Erhöhung seiner Lagerstabilität, mit bestimmten anderen Stoffen gezielt versetzt worden sein. Unter dem Begriff des erfindungswesentlichen Proteins sind deshalb auch alle Präparationen des eigentlichen erfindungswesentlichen Proteins zu verstehen. Das ist unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine amylolytische Funktion entfaltet. Dies kann beispielsweise vom Faltungszustand des Proteins abhängig sein oder aus der reversiblen Bindung eines oder mehrer Begleitstoffe aus der Präparation oder sonstiger Bestandteile des erfindungsgemäßen Mittels an ein erfindungswesentliches Protein oder aus einem anderen Kontrollmechanismus resultieren.

Ein erfindungswesentliches Protein kann besonders während der Lagerung durch Stabilisatoren beispielsweise vor Denaturierung, Zerfall oder Inaktivierung, etwa durch physikalische Einflüsse, Oxidation oder proteolytische Spaltung geschützt werden. Vielfach werden auch sich ergänzende oder gegenseitig steigernde Kombinationen von Stabilisatoren verwendet.

Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, etwa gemäß WO 95/12655 ortho-substituierte, gemäß WO 92/19707 metasubstituierte und gemäß US 5972873 para-substituierte Phenylboronsäuren, beziehungsweise deren Salze oder Ester. In den Anmeldungen WO 98/13460 und EP 583534 werden Peptidaldehyde, das heißt Oligopeptide mit reduziertem C-Terminus, und zwar solche aus 2 - 50 Monomeren, zur reversiblen Inhibierung von Wasch- und Reinigungsmittelproteasen offenbart. Zu den peptidischen reversiblen Proteaseinhibitoren gehört unter anderem Ovomucoid (WO 93/00418). Beispielsweise mit der Anmeldung WO 00/01826 werden spezifische, reversible Peptid-Inhibitoren für die Protease Subtilisin zur Verwendung in Protease-haltigen Mitteln offenbart, und mit WO 00/01831 entsprechende Fusionsproteine aus Protease und Inhibitor.

Weitere Enzymstabilisatoren sind Aminoalkohole wie Mono-, Di-, Triethanol- und - propanolamin und deren Mischungen, aliphatische Carbonsäuren bis zu C₁₂, wie beispielsweise aus den Anmeldungen EP 378261 und WO 97/05227 bekannt ist, wie Bernsteinsäure, andere Dicarbonsäuren oder Salze der genannten Säuren. Mit der Anmeldung DE 19650537 werden für diesen Zweck endgruppenverschlossene Fettsäureamidalkoxylate offenbart. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Ebenso werden Calciumsalze verwendet, wie beispielsweise Calciumacetat oder das in der Patentschrift EP 028865 für diesen Zweck offenbarte Calcium-Formiat, und Magnesiumsalze, etwa gemäß der europäischen Anmeldung EP 378262.

Polyamid-Oligomere (WO 99/43780) oder polymere Verbindungen wie Lignin (WO 97/00932), wasserlösliche Vinyl-Copolymere (EP 828 762) oder, wie in EP 702 712 offenbart, Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere (EP 587550 und EP 581751) wirken gleichzeitig als Enzymstabilisatoren und als Farbübertragungsinhibitoren. Andere polymere Stabilisatoren sind die in WO 97/05227 neben anderen Bestandteilen offenbarten linearen C₈-C₁₈ Polyoxyalkylene. Alkylpolyglycoside könnten wie in den Anmeldungen WO 97/43377 und WO 98/45396 die enzymatischen Komponenten des erfindungsgemäßen Mittels stabilisieren und sogar in ihrer Leistung steigern. Vernetzte N-haltige Verbindungen, wie in WO 98/17764 offenbart, erfüllen eine Doppelfunktion als Soil-release-Agentien und als Enzym-Stabilisatoren. Hydrophobes, nichtionisches Polymer wirkt in einer Mischung mit anderen Stabilisatoren gemäß der Anmeldung WO 97/32958 stabilisierend auf eine Cellulase, so daß sich solche oder ähnliche Bestandteile auch für das erfindungsgemäße Enzym eignen können.

Reduktionsmittel und Antioxidantien erhöhen, wie unter anderem in EP 780466 offenbart, die Stabilität der Enzyme gegenüber oxidativem Zerfall. Schwefelhaltige Reduktionsmittel sind beispielsweise aus den Patentschriften EP 080748 und EP 080223 bekannt. Andere Beispiele sind Natrium-Sulfit (EP 533239) und reduzierende Zucker (EP 656058).

Vielfach werden auch Kombinatonen von Stabilisatoren verwendet, beispielsweise aus Polyolen, Borsäure und/oder Borax in der Anmeldung WO 96/31589, die Kombination von Borsäure oder Borat, reduzierenden Salzen und Bernsteinsäure oder anderen Dicarbonsäuren in der Anmeldung EP 126505 oder die Kombination von Borsäure oder Borat mit Polyolen oder Polyaminoverbindungen und mit reduzierenden Salzen, wie in der Anmeldung EP 080223 offenbart. Die Wirkung von Peptid-Aldehyd-Stabilisatoren wird gemäß WO 98/13462 durch die Kombination mit Borsäure und/oder Borsäurederivaten und Polyolen gesteigert und gemäß WO 98/13459 durch die zusätzliche Verwendung von Calcium-Ionen noch weiter verstärkt.

Nukleinsäuren, die für amylolytische und/oder CGTase-Aktivität ausübende Proteine codieren, stellen Ausführungsformen der vorliegenden Erfindung dar, sofern sie zu der in SEQ ID NO. 1 angegebenen Nukleotidsequenz zunehmend bevorzugt mindestens zu 65 %, 67,5 %, 70 %, 72,5 %, 75 %, 77,5 %, 80 %, 82,5 %, 85 %, 87,5 %, 90 %, 92,5 %, 95 %, 96 %, 97 %, 98 %, mindestens zu 99 % oder zu 100 % identisch ist. Dies gilt entsprechend dem oben Gesagten insbesondere für jene Teilbereiche des Proteins, die den Aminosäuren 35 bis 713 oder den Positionen 35 bis 526 gemäß der SEQ ID NO. 2 entsprechen.

Besonders bevorzugte Ausführungsformen stellen jene Nukleinsäuren dar, die für eines der oben ausgeführten erfindungsgemäßen amylolytischen und/oder CGTase-Aktivität ausübenden Proteine oder Derivate codieren.

Dies schließt auch jene Varianten ein, die nicht über ihre ganze Sequenzlänge hinweg in den nach SEQ ID NO.1 definierten Ähnlichkeitsbereich fallen, dies aber in einzelnen Bereichen tun. Dazu gehören beispielsweise die Nukleotidsequenzen, die, wie oben ausgeführt, durch Insertions- oder Deletionsmutation erhalten worden sind, chimäre Proteine oder Proteinfragmente. Aber auch sogenannte Antisense-Konstrukte, beispielsweise über einzelne Teilabschnitte, stellen Ausführungsformen der vorliegenden Erfindung dar, weil sie zur Regulation der amylolytischen, beziehungsweise der CGTase-Aktivität eingesetzt werden können.

Nukleinsäuren bilden den Ausgangspunkt für molekularbiologische Untersuchungen und Weiterentwicklungen. Solche Methoden sind beispielsweise in dem Handbuch von Fritsch, Sambrook und Maniatis "Molecular cloning: a laboratory manual", Cold Spring Harbour Laboratory Press, New York, 1989 beschrieben. Auf dem Gen, insbesondere auf dem klonierten Gen beruhen auch die meisten im Stand der Technik unter dem Begriff Protein-Engineering zusammengefaßten gentechnischen und Proteinbiochemischen Methoden. Mit solchen können erfindungsgemäße Proteine in Hinblick auf verschiedene Verwendungen weiter optimiert werden, beispielsweise durch Punktmutagenese oder durch Fusion mit Sequenzen aus anderen Genen.

Zu den über an sich bekannte molekularbiologische Methoden erhältlichen, erfindungsgemäßen Varianten eines Proteins gehören insbesondere solche mit einzelnen gezielten Aminosäureaustauschen oder randomisierten Punktmutationen, Deletionen einzelner Aminosäuren oder von Teilsequenzen, Fusionen mit anderen Fragmenten oder anderen Enzymen, Insertionen oder Inversionen. Derartige Mutationen oder Modifikationen stellen entsprechend dem oben Gesagten bevorzugte Ausführungsformen für spezifische Anwendungen dar. Solch eine Mutagenese kann zielgerichtet oder über zufallsgemäße Methoden durchgeführt werden. Dies kann beispielsweise mit einem anschließenden auf die Aktivität gerichteten Erkennungs-und/oder Auswahlverfahren (Screening und Selektion) an den klonierten Genen kombiniert werden.

Weitere Ausführungsformen der vorliegenden Erfindung stellen die Organismen dar, die erfindungsgemäße Proteine oder Derivate natürlicherweise bilden oder für diese codierende Nukleinsäuren enthalten.

Der zuletzt genannte Fall ist beispielsweise dann von Bedeutung, wenn zwar ein entsprechendes Gen vorhanden, unter bestimmten Bedingungen aber nicht exprimiert wird. Dies kann bei nicht funktionierender Expressionsregulation auch für den gesamten Lebenszyklus des betreffenden Organismus gelten.

Derartige Organismen sind in Anwendung allgemein bekannter Techniken, beispielsweise durch Isolieren von Stämmen aus einem natürlichen Habitat oder durch Screening von Genbanken erhältlich. Die in SEQ ID NO. 1 angegebene Nukleotidsequenz oder Teile davon können hierbei beispielsweise als Sonde zum Screening eingesetzt werden oder als Vorlage für die Konstruktion entsprechender PCR-Primer dienen. Analog dazu können kurzkettige oder vollständige Peptide mit Aminosäuresequenzen nach SEQ ID NO. 2 zur Bildung entsprechender Antiseren verwendet werden, mit deren Hilfe entsprechende Organismen, beziehungsweise die von ihnen freigesetzten Proteine identifiziert werden können.

Entsprechend dem oben Gesagten handelt es sich zunehmend bevorzugt um einen Mikroorganismus, um ein- Bakterium, um ein gram-positives Bakterium, um einen Bacillus, um einen alkaliphilen Bacillus, um *Bacillus agaradherens* und insbesondere um *Bacillus agaradherens* (DSM 9948), welcher bei der Deutschen Sammlung von Mikroorganismen und Zellkulturen GmbH, Mascheroder Weg 1b, 38124 Braunschweig (http://www.dsmz.de) hinterlegt worden ist (siehe oben).

Weitere Ausführungsformen der vorliegenden Erfindung stellen Vektoren dar, die einen oben definierten Nukleinsäurebereich enthalten, insbesondere solche, die für ein erfindungsgemäßes Protein oder Derivat codieren. Entsprechend des Einsatzgebiets sind Klonierungsvektoren und/oder Expressionsvektoren bevorzugt.

Vektoren stellen eine im Stand der Technik etablierte Form dar, um mit Nukleinsäuren umzugehen; dafür werden sie nach an sich bekannten Methoden in den gewählten Vektor kloniert. Vektoren leiten sich beispielsweise von bakteriellen Plasmiden, von Viren oder von Bacteriophagen ab, sind überwiegend synthetisch oder Plasmide mit Elementen verschiedenster Herkunft. Sie vermögen, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend sind beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen.

Vektoren bilden geeignete Ausgangspunkte für molekularbiologische und biochemische Untersuchungen des betreffenden Gens oder zugehörigen Proteins und für erfindungsgemäße Weiterentwicklungen und letztlich für die Amplifikation und Produktion erfindungsgemäßer Proteine. Zellen, die Vektoren, insbesondere mit zusätzlichen Genen enthalten, werden als Wirtszellen bezeichnet; besonders geläufig ist dieser Begriff für solche, die die betreffenden Proteine exprimieren.

Bevorzugte Ausführungsformen sind Klonierungsvektoren. Diese eignen sich neben der Lagerung, der biologischen Amplifikation oder der Selektion des interessierenden Gens für dessen Charakterisierung, etwa über das Erstellen einer Restriktionskarte oder die Sequenzierung. Klonierungsvektoren sind auch deshalb bevorzugt, weil sie eine transportierbare und lagerfähige Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.

Expressionsvektoren unterscheiden sich von den Klonierungsvektoren in jenen Teilsequenzen, die sie dazu befähigen, in den für die Produktion von Proteinen optimierten Wirtsorganismen zu replizieren und dort das enthaltene Gen zur Expression zu bringen. Sie ermöglichen somit die Biosynthese der interessierenden Proteine. Bevorzugte Ausführungsformen sind Expressionsvektoren, die selbst die zur Expression notwendigen genetischen Elemente tragen. Die Expression hängt beispielsweise von Promotoren ab, welche die Transkription des Gens regulieren. So kann die Expression durch den natürlichen, ursprünglich vor diesem Gen lokalisierten Promotor erfolgen, aber auch nach gentechnischer Fusion, sowohl durch einen auf dem Expressionsvektor bereitgestellten Promotor der Wirtszelle, als auch durch einen modifizierten oder einen völlig anderen Promotor eines anderen Organismus.

Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind. Expressionsvektoren ermöglichen, daß das zugehörige Protein heterolog, also in einem anderen Organismus als dem, aus dem es natürlicherweise gewonnen werden kann, produziert wird. Auch eine homologe Proteingewinnung aus einem das Gen natürlicherweise exprimierenden Wirtsorganismus über einen passenden Vektor liegt innerhalb des Schutzbereichs der vorliegenden Erfindung. Diese kann den Vorteil aufweisen, daß natürliche, mit der Translation in einem Zusammenhang stehende Modifikationsreaktionen an dem entstehenden Protein genauso durchgeführt werden, wie sie auch natürlicherweise ablaufen würden.

Weitere Ausführungsformen der vorliegenden Erfindung stellen Zellen dar, die eine der oben ausgeführten Nukleinsäuren enthalten, vorzugsweise auf einem oben beschriebenen Vektor.

Denn über sie kann die betreffende Nukleinsäure nach an sich bekannten Methoden zur Gewinnung erfindungsgemäßer Proteine und Derivate genutzt werden. Vorteilhafterweise enthalten sie hierfür die betreffenden Klonierungs- und/oder Expressionsvektoren. Dementsprechend stellen sie Lager- und Transportformen für diese Vektoren dar, zum Beispiel auch für die Übertragung in andere Organismen, oder dienen der Amplifikation oder Modofikation des betreffenden Gens. Solche Zellen, die Expressionsvektoren enthalten und somit zur Biosynthese der betreffenden Proteine befähigt sind, dienen der Produktion der entsprechenden Proteine.

Bevorzugte Ausführungsformen sind entsprechende Zellen, die eines der erfindungsgemäßen Proteine oder Derivate exprimieren oder zu dessen Expression angeregt werden können, insbesondere unter Einsatz eines oben bezeichneten Expressionsvektors.

Mit dem Transfer des Gens in eine Wirtszelle, deren sogenannter Transformation ist die *In-vivo*-Synthese eines erfindungsgemäßen Proteins möglich. Als Wirtszellen hierfür eignen sich prinzipiell alle Organismen, das heißt Prokaryonten, Eukaryonten oder Cyanophyta. Bevorzugt sind solche, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Protein kann auf diese Weise aus einer Vielzahl von Wirtsorganismen gewonnen werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine sehr wirtschaftliche Produktion der interessierenden Proteine.

In einer bevorzugten Ausführungsform handelt es sich bei den Wirtszellen um Bakterien, insbesondere solche, die das gebildete Protein oder Derivat ins umgebende Medium sekretieren.

Denn Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Proteine etabliert werden. Insbesondere grampositive Bakterien, wie beispielsweise Bacilli, besitzen keine äußere Membran, so daß sekretierte Proteine sogleich in das die Zellen umgebende Nährmedium abgegeben werden, aus welchem sich nach einer anderen bevorzugten Ausführungsform die exprimierten erfindungsgemäßen Proteine direkt aufreinigen lassen. Aber auch für gram-negative Bakterien sind ähnliche Systeme entwickelt worden (siehe unten).

Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Proteine beeinflussen. Hierbei kann es sich um modifizierende Genprodukte handeln oder um solche, die mit dem erfindungsgemäßen Protein gemeinsam aufgereinigt werden sollen, etwa um dessen enzymatische Funktion zu beeinflussen. Dabei kann es sich beispielsweise um andere Proteine oder Enzyme, um Inhibitoren oder um solche Elemente handeln, die die Wechselwirkung mit verschiedenen Substraten beinflussen.

In einer bevorzugten Ausführungsform handelt es sich bei den erfindungsgemäßen Wirtszellen zur Produktion erfindungsgemäßer Proteine oder Derivate um solche, die der Gattung Bacillus angehören, insbesondere der Species *Bacillus licheniformis, Bacillus amyloliquefaciens, Bacillus subtilis* oder *Bacillus alcalophilus.*

So nutzt beispielsweise eine Ausführungsform der vorliegenden Erfindung *B. agaradherens* (DSM 9948) selbst, um erfindungsgemäße Proteine homolog zu exprimieren. Dies kann beispielsweise über einen eingebrachten Vektor erfolgen, der das bereits endogen vorhandene Gen, oder erfindungsgemäße Abwandlungen desselben, in diese Zellen einbringt, beispielsweise in einer vielfachen Kopienzahl. Dies kann dann besonders vorteilhaft sein, wenn das Protein im Anschluß an seine Synthese Modifikationen erfahren soll, die geeigneterweise von den betreffenden Zellen selbst durchgeführt werden.

Demgegenüber ist jedoch die heterologe Expression bevorzugt, und zwar besonders in Stämmen der Gattung Bacillus. So wurde in Beispiel 5 der vorliegenden Anmeldung eine heterologe Expression in B. *subtilis* durchgeführt. Ganz besonders bevorzugt sind solche der Species *Bacillus licheniformis, Bacillus amyloliquefaciens, Bacillus subtilis* oder *Bacillus alcalophilus.* Denn mit diesen hat ma in der technischen Produktion weitreichende Erfahrungen.

In einer bevorzuten Ausführungsform handelt es sich bei erfindungsgemäßen Wirtszellen um gram-negative Bakterien.

Dies gilt insbesondere für solche, die der Klonierung und/oder gentechnischen Modifikation der enthaltenen erfindungsgemäßen Nukleinsäuren dienen, weil man hierfür mit gram-negativen Bakterien, insbesondere *Escherichia coli* die meisten Erfahrungen hat.

Bei gram-negativen Bakterien werden in der Regel eine Vielzahl von Proteinen in den periplasmatischen Raum sekretiert, also in das Kompartiment zwischen den beiden die Zellen einschließenden Membranen. Dies kann für spezielle Anwendungen vorteilhaft sein.

In einer bevorzuten Ausführungsform handelt es sich bei erfindungsgemäßen gram-negativen Bakterien um solche der Gattung Escherichia, bevorzugt der Spezies *Escherichia coli* oder *Klebsiella,* besonders bevorzugt solche der Stämme *E*. *coli* JM 109, *E. coli* DH 100B, *E. coli* DH 12S oder *Klebsiella planticola* (Rf).

Denn für derartige Bakterien sind inzwischen Systeme beschrieben worden, nach denen auch sie zur Produktion extrazellulärer Proteine eingesetzt werden können. Derartige Systeme werden beispielsweise in der Anmeldung PCT/EP01/04227 beschrieben.

In weiteren Ausführungsformen handelt es sich bei erfindungsgemäßen Zellen um eukaryontische Zellen, besonders solche, die das Protein exprimieren, ganz besonders solche, die das Protein posttranslational modifizieren.

Sie können ebenfalls nach im Stand der Technik etablierten Methoden zur Klonierung genutzt werden. Vorzugsweise werden sie aber zur Produktion erfindungsgemäßer Proteine genutzt. Dies ist beispielsweise dann besonders vorteilhaft, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccharide. Beispiele für hierfür bevorzugt einsetzbare eukaryontische Zellen sind Pilze wie Actinomyceten oder Hefen wie *Saccharomyces* oder *Kluyveromyces.*

Einen weiteren Erfindungsgegenstand stellen Verfahren zur Herstellung eines erfindungsgemäßen Proteins oder Derivats unter Verwendung einer oben definierten erfindungsgemäßen Nukleinsäure und/oder unter Verwendung eines oben definierten erfindungsgemäßen Organismus und/oder unter Verwendung eines oben definierten erfindungsgemäßen Vektors und/oder unter Verwendung einer oben definierten erfindungsgemäßen Zelle, dar.

Hierunter fallen alle an sich bekannten Verfahren, die auf einem der oben ausgeführten Elemente aufbauen. So können prinzipiell alle bereits ausgeführten Elemente zu Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar. Das optimale Verfahren muß für jeden konkreten Einzelfall, beispielsweise abhängig von der Größe und Identität des Gens, der Art der Muationen, den Eigenschaften des Wirtsstammes, wie etwa die Codon-Usage oder das Selektionssystem, experimentell ermittelt werden.

Prinzipiell wird dabei folgendermaßen vorgegangen: Erfindungsgemäße Nukleinsäuren werden geeigneterweise in Form der DNA - gegebenenfalls nach Bildung, Modifikation und/oder Zwischenlagerung in einem Konierungsvektor - in einen geeigneten Expressionsvektor ligiert. Dieser wird in die zur Expression geignete Wirtszelle transformiert, beispielsweise in Zellen eines leicht zu kultivierenden Bakterienstammes, der die Proteine, deren Gene unter der Kontrolle entsprechender genetischer Elemente stehen, in das umgebende Nährmedium ausschleust; regulierende Elemente dafür können beispielsweise vom Expressionsvektor zur Verfügung gestellt werden. Aus dem umgebenden Medium kann das erfindungsgemäße Protein über mehrere Aufreinigungsschritte, wie beispielsweise Filtrationen, Fällungen oder Chromatographien, aufgreinigt werden. Ein Fachmann ist in der Lage, ein System, welches im Labormaßstab experimentell optimiert worden ist, auf einen großtechnischen Produktionsmaßstab zu übertragen.

Auf jeder Stufe dieses Verfahrens ergeben sich Variationsmöglichkeiten, die dem Fachmann an sich vertraut sind und je nach gewünschtem Ergebnis (Mutation, Ausbeute, Aufarbeitbarkeit etc.) ausgeschöpft werden können. Ausführungsformen der vorliegenden Erfindung können beispielsweise auch zellfreie Expressionssysteme sein, bei denen die Proteinbiosynthese *in vitro* nachvollzogen wird. Derartige Expressionssysteme sind im Stand der Technik ebenfalls etabliert.

Im folgenden werden die wichtigsten technischen Einsatzgebiete für erfindungsgemäße Proteine ausgeführt. Zahlreiche in der Technik etablierte Einsatzmöglichkeiten für amylolytische Enzyme werden in Handbüchern, wie beispielsweise dem Buch "Industrial enyzmes and their applications" von H. Uhlig, Wiley-Verlag, New York, 1998, ausgeführt. Folgende Zusammenstellung ist nicht als abschließende Aufzählung zu verstehen, sondern stellt eine Auswahl der besonders relevanten Gebiete dar. Sollte sich herausstellen, daß einzelne, innerhalb des Ähnlichkeitsbereichs liegende Proteine aufgrund ihrer enyzmatischen, das heißt amylolytischen und/oder CGTase-Eigenschaften für zusätzliche hier nicht ausdrücklich beanspruchte Anwendungsmöglichkeiten geeignet sind, so werden diese hiermit in den Schutzbereich der vorliegenden Erfindung miteingeschlossen.

Ein wichtiges Einsatzgebiet für amylolytische Enzyme ist das als aktive Komponenten in Wasch- oder Reinigungsmitteln zur Reinigung von Textilien oder von festen Oberflächen.

Dies gilt insbesondere auch für solche erfindungsgemäßen Proteine oder Derivate, bei denen die Domänen D und E unter Verzicht auf die CGTase-Aktivität deletiert worden sind, die aber aufgrund ihrer amylolytischen Aktivität zur Verwendung zur Reinigung von Textilien oder harten Oberflächen geeignet sind. Dies kann für zahlreiche Anwendungen ausreichend sein, insbesondere dann, wenn gegenüber der Cyclodextrin-Synthese die Hydrolyse von stärkeähnlichen Verbindungen im Vordergrund steht. Somit stellt sich der Verzicht auf die übrigen Bereiche günstiger, beispielsweise kostengünstiger in der Herstellung des Proteins dar.

Andererseits können erfindungsgemäße Proteine mit CGTase-Aktivität zusätzlich zur Reinigungsleistung eine desodorierende Wirkung entfalten. Dies ist möglicherweise darauf zurückzuführen, daß entsprechende niedermolekulare Verbindungen in die entstehenden Cyclodextrinverbindungen eingeschlossen und auf diese Weise maskiert werden.

Einen Erfindungsgegenstand stellen somit Wasch- oder Reinigungsmittel dar, die dadurch gekennzeichnet, daß sie eines der oben beschriebenen, erfindungsgemäßen Proteine oder Derivate enthalten.

In diesen Mitteln dient die amylolytische Aktivität dazu, kohlenhydrathaltige, insbesondere stärkeähnliche Verunreinigungen hydrolytisch aufzulösen und/oder vom Untergrund abzulösen. Sie wird vorzugsweise durch die CGTase-Aktivität ergänzt, beispielsweise hinsichtlich der Freisetzung oder Maskierung von Begleiststoffen. Diese Mittel zeichnen sich dadurch aus, daß die amylolytischen und/oder CGTase-Aktivität aufweisenden Enzyme und die übrigen Komponenten synergistisch die Beseitigung der Verunreinigungen bewirken, beispielsweise indem die Hydrolyseprodukte der amylolytischen Proteine durch andere Bestandteile der Mittel wie etwa Tenside solubilisiert werden.

Ein erfindungsgemäßes Protein oder Derivat kann sowohl in Mitteln für Großverbraucher oder technische Anwender als auch in Produkten für den Privatverbraucher Anwendung finden, wobei alle im Stand der Technik etablierten Darreichungsformen auch entsprechende Ausführungsformen der vorliegenden Erfindung darstellen.

Damit sind alle denkbaren Reinigungsmittelarten gemeint, sowohl Konzentrate, als auch unverdünnt anzuwendende Mittel; zum Einsatz im industriellen, das heißt kommerziellen Maßstab, in Wasch- oder Spülmaschinen oder bei der individuellen, das heißt manuellen Wäsche, beziehungsweise Reinigung. Dazu gehören beispielsweise Waschmittel für Textilien, Teppiche oder Naturfasern, für die nach der vorliegenden Erfindung die Bezeichnung Waschmittel verwendet wird. Dazu gehören beispielsweise auch Geschirrspülmittel für Geschirrspülmaschinen, manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die nach der vorliegenden Erfindung die Bezeichnung Reinigungsmittel verwendet wird. Jegliche Reinigungsmittelart stellt eine Ausführungsform der vorliegenden Erfindung dar, sofern sie um ein erfindungsgemäßes Protein bereichert ist.

Ausführungsformen der vorliegenden Erfindung umfassen alle nach den Stand der Technik etablierten und/oder alle zweckmäßigen Darreichungsformen der erfindungsgemäßen Mittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Neben einem erfindungsgemäßen Protein oder Derivat enthält ein erfindungsgemäßes Mittel gegebenenfalls weitere Inhaltsstoffe wie Tenside, zum Beispiel nichtionische, anionische und/oder amphotere Tenside, und/oder Bleichmittel, und/oder Builder, sowie gegebenenfalls weitere Inhaltsstoffe, die im folgenden ausgeführt werden.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden kann, sind die Alkylpolyglycoside (APG). Einsetzbare Alkypolyglycoside genügen der allgemeinen Formel RO(G)_{z}, in der R einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II),

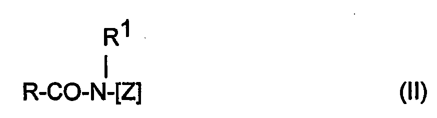

in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 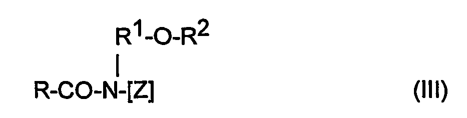 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose; Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, das heißt Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.-%, eingesetzt.

Weitere geeignete Aniontenside sind auch die Salze der Alkylsulfobemsteinsäure, die auch als Sulfosuccinate oder als Sulfobemsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbemsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di-oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 1 Gew.-% bis 50 Gew.-%, insbesondere von 5 Gew.-% bis 30 Gew.-%, bezogen auf das fertige Mittel, enthalten sein.

Erfindungsgemäße Mittel können Bleichmittel enthalten. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Mittel an Bleichmittel kann 1 bis 40 Gew.-% und insbesondere 10 bis 20 Gew.-%, betragen, wobei vorteilhafterweise Perboratmonohydrat oder Percarbonat eingesetzt wird. Eine synergistische Verwendung von Amylase mit Percarbonat oder von Amylase mit Percarbonsäure wird mit den Anmeldungen WO 99/63036, beziehungsweise WO 99/63037 offenbart. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Um beim Waschen bei Temperaturen von 60°C und darunter, und insbesondere bei der Wäschevorbehandlung eine verbesserte Bleichwirkung zu erreichen, können Bleichaktivatoren in die Wasch- und Reinigungsmittelformkörper eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (*n-* beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16770 sowie der internationalen Patentanmeldung WO 95/14075 beschriebenen Acyllactame werden ebenfalls bevorzugt eingesetzt. Auch die aus der deutschen Patentanmeldung DE 44 43 177 bekannten Kombinationen konventioneller Bleichaktivatoren können eingesetzt werden, Ebenso können Nitrilderivate wie Cyanopyridine, Nitrilquats, zum Beispiel N-Alkylammoniumacetonitrile, und/oder Cyanamidderivate eingesetzt werden. Bevorzugte Bleichaktivatoren sind Natrium-4-(octanoyloxy)-benzolsulfonat, *n*-Nonanoyl-oder Isononanoyloxybenzolsulfonat (*n*- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonitril (MMA). Derartige Bleichaktivatoren können im üblichen Mengenbereich von 0,01 bis 20 Gew.-%, vorzugsweise in Mengen von 0,1 bis 15 Gew.-%, insbesondere 1 Gew.-% bis 10 Gew.-%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren geeignet, wobei solche Verbindungen bevorzugt eingesetzt werden, die in der DE 19709284 A1 beschrieben sind. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Die erfindungsgemäßen Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilicate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 0164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt. Im Handel befinden sich derartige Verbindungen beispielsweise unter der Bezeichnung SKS^{®} (Fa. Clariant). So handelt es sich bei SKS-6^{®} vorwiegend um ein δ-Natriumdisilicat mit der Formel Na₂Si₂O₅·yH₂O, bei SKS-7® vorwiegend um das β-Natriumdisilicat. Durch Reaktion mit Säuren (zum Beispiel Citronensäure oder Kohlensäure) entsteht aus dem δ-Natriumdisilicat Kanemit NaHSi₂O₅·yH₂O, im Handel unter den Bezeichnungen SKS-9^{®} beziehungsweise SKS-10^{®} (Fa. Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂O • y SiO₂ • z P₂O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 19601063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe. Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2- 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°C) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°C), 7 Mol. (Dichte 1,68 gcm⁻³, Schmelzpunkt 48°C unter Verlust von 5 H₂O) und 12 Mol. Wasser (Dichte 1,52 gcm⁻³, Schmelzpunkt 35°C unter Verlust von 5 H₂O), wird bei 100°C wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340°C und ist in Wasser mit alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°C, auch 880°C angegeben) und als Decahydrat (Dichte 1,815-1,836 gcm⁻³, Schmelzpunkt 94°C unter Wasserverlust). Beide Substanzen sind farblose, in Wasser mit alkalischer Reaktion lösliche Kristalle. Na₄P₂O₇ entsteht beim Erhitzen von Dinatriumphosphat auf >200°C oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³dar, das in Wasser löslich ist, wobei der pH-Wert der 1%igen Lösung bei 25°C 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natriumbeziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60°C ca. 20 g, bei 100°C rund 32 g des kristallwasserfreien Salzes; nach zweistündigem Erhitzen der Lösung auf 100°C entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entWässert. Ähnlich wie Grahamsches Salz und Natriurndiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀+H₂O

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei systemunbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.-%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/ Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden Können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauch-bar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472042, WO 97/25399, und EP 755944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, zum Beispiel als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cobuilder eingesetzt werden.

Buildersubstanzen können in den erfindungsgemäßen Mitteln gegebenenfalls in Mengen bis zu 90 Gew.-% enthalten sein. Sie sind vorzugsweise in Mengen bis zu 75 Gew.-% enthalten. Erfindungsgemäße Waschmittel weisen Buildergehalte von insbesondere 5 Gew.-% bis 50 Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.-% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch-und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder - ethylether, Di-isopropylenglykolmonomethyl-, oder -ethylether, Methoxy-, Ethoxy- oder Butoxytriglykol; 1-Butoxyethoxy-2-propanol, 3=Methyl-3-methoxybutanol, Propylen-glykolt-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.-%, bevorzugt aber unter 15 Gew.-% und insbesondere unterhalb von 10 Gew.-% eingesetzt werden.

Zur Einstellung der Viskosität können der erfindungsgemäßen Zusammensetzung ein oder mehrere Verdicker beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickern zählen beispielsweise Polykieselsäuren, Tonmineralien wie Montmorillonite, Zeolithe, Kieselsäuren und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl-und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- oder Reinigungsmittel kann gegebenenfalls als weitere übliche Inhaltsstoffe Sequestrierungsmittel, Elektrolyte und weitere Hilfsstoffe, wie optische Aufheller, Vergrauungsinhibitoren, Silberkorrosionsinhibitoren, Farbübertragungsinhibitoren, Schauminhibitoren, Abrasivstoffe, Farb- und/oder Duftstoffe, sowie mikrobielle Wirkstoffe und/oder UV-Absorbenzien enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt-oder Cersalze und/oder -komplexe, in denen die genannten Metalle in einer der Oxidationsstufen II, III, IV, V oder VI vorliegen. Beispiele für derartige Verbindungen sind MnSO₄, V₂O₅ V₂O₄, VO₂, TiOSO₄, K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 16 17 141, beziehungsweise DT 22 00911). In der deutschen Offenlegungsschrift DT 22 53 063 sind saure Mittel genannt, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyl- oder Ethylgruppenendverschlossene Polyester mit Ethylen- und/oder Propylen-terephthalat- und Polyethylenoxid-terephthalat-Einheiten und Waschmitel, die derartiges Soil-release-Polymer enthalten, bekannt. Das europäische Patent EP 0241984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272033 sind zumindest anteilig durch C₁₋₄-Alkyl- oder Acylreste endgruppenverschlossene Polyester mit Poly-propylenterephthalat- und Polyoxyethylenterephthalat-Einheiten bekannt. Das europäische Patent EP 0 274 907 beschreibt sulfoethylendgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Üblicherweise liegt der Gehalt von Wasch- und Reinigungsmitteln an Farbstoffen unter 0,01 Gew.-%, während Duftstoffe bis zu 2 Gew.-% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

Sofern eine erfindungswesentliche CGTase-Aktivität der Herstellung solcher Komplexe eingesetzt werden kann, oder der Freisetzung der Inhaltsstoffe dient, so handelt es sich um bevorzugte Ausführungsformen der vorliegenden Erfindung. Das gleiche gilt für weitere niedermolekulare Verbindungen wie Farbstoffe oder antimikrobielle Wirkstoffe, die weiter unten dargestellt werden.

Ein weiter bevorzugter Träger für Duftstoffe ist der beschriebene Zeolith X, der anstelle von oder in Mischung mit Tensiden auch Duftstoffe, aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von K. H. Wallhäußer in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, lod, lodophore, Peroxoverbindungen, Halogenverbindungen sowie beliebigen Gemischen der voranstehenden.

Der antimikrobielle-Wirkstoff-kann dabei ausgewählt sein-aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alpha-methyl-.beta.-phenyldiguanido-N₅,N₅')-hexan-dihydrochlorid, 1,6-Di-(N₁,N₁')-p-nitrophenyldiguanido-N₅,N₅')hexan-dihydrochlorid, omega:omega-Di-(N₁,N₁'-phenyldiguanido-N₅,N₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecantetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormeta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, lodonium-oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel (R¹)(R²)(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium-oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzyl-ammoniumchlorid, CAS No. 8001-54-5), Benzalkon B (*m,p*-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)-pheno-xy]ethoxy]ethyl]-benzylammoniumchlorid, CAS No. 121-54-0), Dialkyldimethylammonium-chloride wie Di-*n*-decyl-dimethyl-ammoniumchlorid (CAS No. 7173-51-5-5), Didecyldi-methylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethyl-ammoniumchlorid.

Benzalkoniumhalogenide und/oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Sälicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, das heißt hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet. Weitere geeignete UV-Lichtschutzfilter sind der Übersicht von P. Finkel in SÖFW-Journal 122 (1996), S. 543 zu entnehmen.

Die UV-Absorbenzien werden üblicherweise in Mengen von 0,01 Gew.-% bis 5 Gew.-%, vorzugsweise von 0,03 Gew.-% bis 1 Gew.-%, eingesetzt.

Insbesondere in Wasch- und Reinigungsmitteln können erfindungsgemäße und/oder andere Proteine eines besonderen Schutzes bedürfen. Bevorzugte erfindungsgemäße Mittel enthalten zu diesem Zweck Stabilisatoren. Diese sind weiter oben bereits ausgeführt worden. Mittel mit den wie oben ausgeführt stabilisierten Enzymaktivitäten stellen bevorzugte Ausführungsformen der vorliegenden Erfindung dar. Besonders bevorzugt sind solche mit Enzymen, die auf mehrere der dargestellten Weisen stabilisiert sind.

Erfindungsgemäße Mittel bevorzugter Ausführungsformen enthalten das erfindungsgemäße amylolytische und/oder CGTase-Aktivität aufweisende Protein oder Fragment in Anteilen von 0,0001 Gewichts-Prozent bis 5 Gew.-% und zunehmend bevorzugt in Anteilen von 0,00025 bis 4,5 Gew.-%, von 0,0005 bis 4 Gew.-%, von 0,00075 bis 3,5 Gew.-%, 0,001 bis 3 Gew.-% oder 0,002 bis 2 Gew.-%.

Die Proteinkonzentration kann mit Hilfe bekannter Methoden, zum Beispiel dem Bicinchoninsäure-Verfahren (2,2'-Bichinolyl-4,4'-dicarbonsäure; BCA-Verfahren; Pierce Chemical Co., Rockford, IL) oder dem Biuret-Verfahren (A.G. Gornall, C.S. Bardawill und M.M. David, J. Biol. Chem. 177 (1948), S. 751-766) bestimmt werden.

In einer bevorzugten Ausführungsform enthalten erfindungsgemäße Mittel zusätzlich zu dem erfindungsgemäßen Protein oder Derivat ein oder mehrere andere amylolytische Enzyme, insbesondere α-Amylasen.

Darunter können sich aus dem Stand der Technik bekannte, insbesondere für die Verwendung in Wasch- und Reinigungsmitteln etablierten Enzyme befinden. Beispiele für kommerziell erhältliche Amylasen sind BAN^{®}, Termamyl^{®}, Purastar^{®}, Amylase-LT^{®}, Maxamyl^{®}, Duramyl^{®} und/oder Purafect^{®} OxAm und andere eingangs ausgeführte Enzyme. Dies ist dann angebracht, wenn sich die verschiedenen Enzyme einander zu ergänzen vermögen. Solch eine Ergänzung kann beispielsweise in regulatorischer Hinsicht erfolgen, etwa durch gegenseitige Aktivierung oder durch Inaktivierung. Sie kann sich beispielsweise dadurch ergeben, daß mindestens ein nicht zu den bekannten α-Amylasen homologer Teil des erfindungswesentlichen Enzyms Einfluß auf die zusätzlich vorhandenen amylolytischen Aktivitäten nimmt. Die gemeinsame Verwendung kann aber auch aufgrund abweichender Substratspezifitäten sinnvoll sein. Für beide Ausführungsformen kann die CGTase-Aktivität der erfindungsgemäßen Varianten vorteilhaft sein.

Eine bevorzugte Ausführungsform stellen erfindungsgemäße Mittel dar, die dadurch gekennzeichnet sind, daß sie zusätzlich andere Enzyme, insbesondere eine oder mehrere Proteasen, Lipasen, Oxidoreduktasen, Hemicellulasen und/oder Cellulasen enthalt.

Denn insbesondere an chemisch diversen Anschmutzungen ist es vorteilhaft, zusätzliche reinigungsaktive, jeweils spezifisch wirkende Enzymen einzusetzen. Dazu gehören beispielsweise, wie dem Stand der Technik entnommen werden kann, Proteasen, Lipasen, Cutinasen, Oxidoreduktasen oder Peroxidasen als Komponenten von enzymatischen Bleichsystemen, wie beispielsweise Laccasen(WO 00/39306), Esterasen, Pullulanasen, Cellulasen, Hemicellulasen, wie beispielsweise Xylanasen, Pektin-lösende Enzyme (WO 00/42145) oder β-Glucanasen (WO 99/06515 und WO 99/06516), die insbesondere in Spezialwaschmitteln zum Einsatz kommen, sowie deren Gemische.

Beispiele für kommerziell erhältliche Enzyme zum Gebrauch in erfindungsgemäßen Mitteln sind Proteasen wie Subtilisin BPN', Properase^{®}, BLAP^{®}, Optimase^{®}, Opticlean^{®}, Maxatase^{®}, Maxacal^{®}, Maxapem^{®}, Alcalase^{®}, Esperase^{®}, Savinase^{®}, Durazym^{®}, Everlase^{®} und/oder Purafect^{®}G oder Purafect^{®}OxP und Lipasen wie Lipolase^{®}, Lipomax^{®}, Lumafast^{®} und/oder Lipozym^{®}.

Die Proteaseaktivität in derartigen Mitteln kann nach der in Tenside, Bd. 7 (1970), S. 125-132 beschriebenen Methode ermittelt werden. Sie wird dementsprechend in PE (Protease-Einheiten) angegeben. Die Proteaseaktivität bevorzugter Mittel kann bis zu 1.500.000 Proteaseeinheiten pro Gramm Zubereitung betragen (PE, bestimmt nach der in Tenside, Bd. 7 (1970), S. 125-132 beschriebenen Methode).

Hinsichtlich ihrer Gewinnung kommen unter den verwendbaren Enzymen in erster Linie die aus Mikroorganismen, wie Bakterien oder Pilzen, gewonnenen in Frage, beispielsweise aus *Bacillus subtilis, Bacillus licheniformis, Streptomyces griseus, Humicola lanuginosa, Humicola insolens, Pseudomonas pseudoalcaligenes* oder *Pseudomonas cepacia,* insbesondere die von diesen Stämmen natürlicherweise gebildeten Enzymgemische, beziehungsweise Gemische mit denen anderer Stämme. Sie werden in bekannter Weise durch Fermentationsprozesse aus geeigneten Mikroorganismen gewonnen, die zum Beispiel in den deutschen Offenlegungsschriften DE 1940488 und DE 2121397, den US-amerikanischen Patentschriften US 3623957 und US 4264738, der europäischen Patentanmeldung EP 006638, sowie der internationalen Patentanmeldung WO 91/02792 beschrieben sind.

Auch diese gegebenenfalls zusätzlich verwendeten Enzyme können, wie zum Beispiel in der europäischen Patentschrift EP 564476 oder in der internationalen Patentanmeldungen WO 94/23005 beschrieben, an Trägerstoffen adsorbiert und/oder in Hüllsubstanzen eingebettet sein, um sie gegen vorzeitige Inaktivierung zu schützen. Sie sind in Waschmitteln vorzugsweise in Mengen bis zu 10 Gew.-%, insbesondere von 0,2 Gew.-% bis 2 Gew.-%, enthalten, wobei besonders bevorzugt gegen oxidativen Abbau stabilisierte Enzyme, wie zum Beispiel aus den internationalen Patentanmeldungen WO 94/18314 bekannt, eingesetzt werden.

In einer bevorzugten Ausführungsform sind erfindungsgemäße Mittel dadurch gekennzeichnet, daß sie Cyclodextrine enthalten, insbesondere solche, in die niedermolekulare Verbindungen eingelagert sind.

Bei diesen kann es sich beispielsweise um die oben ausgeführten Duftstoffe, Farbstoffe oder antimikrobiellen Wirkstoffe handeln. Dies ist dann besonders vorteilhaft, wenn die Freisetzung dieser Inhaltsstoffe zu einem bestimmten Zeitpunkt, beispielsweise innerhalb eines mehrstufigen Verfahren gewünscht ist. Durch Zugabe oder Aktivierung der Amylase und/oder der CGTase können diese dann freigesetzt werden.

Umgekehrt kann die Aktivität der CGTase dazu dienen, um unerwünschte niedermolekulare Verbindungen, wie Geruchsstoffe oder giftige Stoffe in Cyclodextrine zu verkapseln und somit die Reinigungsleistung des betreffenden Mittels, beziehungsweise des betreffenden Verfahrensschritts oder der betreffenden Verwendung zu steigern. Zur Realisierung dieser Variante iste es vorteilhaft, den betreffenden Mitteln eine Stärke oder stärkeähnliche Verbindung zuzusetzen.

Bevorzugte Mittel sind dadurch gekennzeichnet, daß sie aus mehr als einer Phase bestehen.

Dabei kann es sich um Phasen in zwei verschiedenen Aggregatzuständen, insbesondere aber um zwei Phasen in denselben Aggregatzuständen handeln. Liegen beide oder mehrere Phasen miteinander verbunden vor, so werden die jeweiligen Wirkungen besonders gut aufeinander abgestimmt. Ein Vorteil dieser nach dem Stand der Technik auszugestaltenden Ausführungsform liegt darin, daß derartige Mittel beispielsweise die enthaltenen Wirkstoffe zeitlich oder räumlich voneinander getrennt freisetzen können. Zudem können empfindlichere Bestandteile, wie beispielsweise Enzyme, vor anderen Komponenten während der Lagerung geschützt werden.

Eine bevorzugte derartige Ausführungsform ist dadurch gekennzeichnet, daß das Mittel fest ist und mindestens zwei verschiedene feste Komponenten, insbesondere Pulver, Granulate oder Extrudate, in insgesamt loser Form miteinander vermischt vorliegen.

Derartige Mittel können auf einfache Weise dadurch hergestellt werden, daß die verschiedenen festen Komponenten, insbesondere Pulver, Granulate oder Extrudate mit verschiedenen Inhaltsstoffen und/oder Gemischen von Inhaltsstoffen und/oder unterschiedlichem Feisetzungsverhalten in insgesamt loser Form miteinander vermischt werden. Die Herstellung dieser Komponenten kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 486592 bekanntes, einen Extrusionschritt auf weisendes Verfahren bevorzugt. Eine weitere bevorzugte Herstellung mit Hilfe eines Granulationsverfahrens ist in der europäischen Patentschrift EP 642576 beschrieben. Einzelne dieser Komponenten können dabei auch in komprimierter Form vorgelegt werden.

Proteine können für feste oder wasserarme Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Die verkapselte Form bietet sich an, um die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt-sind. Solche Kapseln-werden-beispielsweise mit den Patentanmeldungen WO 97/24177 und DE 19918267 offenbart. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden. Ein solches Verkapselungsverfahren wird mit der deutschen Anmeldung DE 19956382 mit dem Titel "Verfahren zur Herstellung von mikroverkapselten Enzymen" beschrieben.

In einer bevorzugten Ausführungsform sind die erfindungsgemäßen Mittel dadurch gekennzeichnet, daß sie kompaktiert sind.

Bevorzugt ist das zusätzliche Verpressen oder Kompaktieren zu Tabletten. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile- gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN/cm², vorzugsweise bei 60 bis 70 kN/cm² verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN/cm², insbesondere bei 10 bis 15 kN/cm² durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind.

Erfindungsgemäße Mittel bevorzugter Ausführungsformen sind dadurch gekennzeichnet, daß wenigstens eine der Phasen ein Amylase-sensitives Material, insbesondere Stärke enthält oder von diesem zumindest teilweise umgeben oder beschichtet ist.

Denn dann können die Inhaltsstoffe der betreffenden Phase mithilfe der Aktivität eines erfindungsgemäßen Proteins oder Derivats freigesetzt werden. Dies ist insbesondere dann vorteilhaft, wenn die betreffenden Stoffe insbesondere nicht während der Lagerung oder noch nicht zu Beginn des Einsatzes des betreffenden Mittels aktiv werden sollen. Dies kann für Mittel mit mindestens zwei unterschiedlichen Phasen genutzt werden, beispielsweise zwei oder mehr feste, miteinander verbundene Phasen eines tablettenförmigen Wasch- oder Reinigungsmittels, oder verschiedene Ganulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist dann eine günstige Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert. Alternativ können die amylolytische Aktivität und ihr Substrat auch unmittelbar benachbart sein, so daß mit hinzutretendem Wasser unmittelbar die Hydrolyse und Desintegration einsetzen.

Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen der vorliegenden Erfindung darstellen.

Erfindungsgemäße Mittel bevorzugter Ausführungsformen sind dadurch gekennzeichnet, daß sie flüssig, gelförmig oder pastös sind und das enthaltene Protein und/oder wenigstens eines der enthaltenen Enzyme und/oder wenigstens eine der sonstigen enthaltenen Komponenten einzeln oder zusammen mit anderen Bestandteilen verkapselt vorliegt, bevorzugt in Mikrokapseln, besonders bevorzugt in solchen aus einem Amylase-sensitiven Material.

Derartigen Mitteln können die erfindungsgemäßen Proteine oder Derivate in konzentrierter wäßriger oder nichtwäßriger Lösung beispielsweise in flüssiger Form, etwa als Lösung, Suspension oder Emulsion, aber auch in Gelform oder verkapselt oder als getrocknetes Pulver zugesetzt werden. Derartige Mittel werden in der Regel durch einfaches Mischen der Inhaltsstoffe hergestellt, die in Substanz oder als homogene flüssige Phase in einen automatischen Mischer gegeben werden.

Besonders bevorzugt sind darunter solche flüssigen, gelförmigen oder pastösen Mittel mit Kapseln aus amylasesensitivem Material. Dadurch unterstützt das amylolytische Enzym die Spaltung der Mikrokapseln und steuert somit wie oben beschrieben den Freisetzungsprozeß der verkapselten Inhaltsstoffe. Vorteilhafterweise erfolgt diese Freisetzung zu einem bestimmten Zeitpunkt.

In einer bevorzugten Ausführungsform sind erfindungsgemäße Mittel dadurch gekennzeichnet, daß die amylolytische und/oder CGTase-Aktivität durch einen der sonstigen Bestandteile des Mittels modifiziert, insbesondere stabilisiert und/oder in ihrem Beitrag zur Wasch-, beziehungsweise Reinigungsleistung des Mittels gesteigert wird.

Die Inhaltsstoffe erfindungsgemäßer Wasch- oder Reinigungsmitteln vermögen sich geeigenterweise also gegenseitig in ihrer Leistung zu unterstützen. Die synergistische Verwendung von Amylase und Farbübertragungsinhibitoren zur Steigerung der Reinigungsleistung wird beispielsweise mit der Anmeldung WO 99/63035 offenbart. Es ist auch bekannt, daß Polymere, die gleichzeitig als Cobuilder eingesetzt werden können, wie beispielsweise Alkyl-Poly-Glykoside, die Aktivität und die Stabilität von enthaltenen Enzymen stabilisieren und steigern können, so aus der Anmeldung WO 98/45396. Ebenso ist es möglich, daß auch eine erfindungswesentliche amylolytische Aktivität durch einen der übrigen, oben aufgeführten Bestandteile modifiziert, insbesondere stabilisiert und/oder gesteigert wird. Entsprechend abgestimmte Rezepturen für erfindungsgemäße Mittel stellen somit besonders bevorzugte Ausführungsformen der vorliegenden Erfindung dar.

Einen weiteren Erfindungsgegenstand stellen Verfahren zur Reinigung von Textilien oder von harten Oberflächen dar, die dadurch gekennzeichnet sind, daß in wenigstens einem der Verfahrensschritte ein amylolytisches und/oder CGTase-Aktivität aufweisendes erfindungsgemäßes Protein oder Derivat aktiv wird.

Ausführungsformen hiervon stellen alle Reinigungsverfahren dar, darunter manuelle, insbesondere aber maschinelle Reinigungsverfahren. Das können Verfahren zur Reinigung aller denkbaren Materialien sein, insbesondere von Textilien oder vergleichbaren Materialien und von harten Oberflächen sein. So wird mit den Beispielen in der vorliegenden Anmeldung belegt, daß sich ein erfindungsgemäßes Enzym, eingebunden in ein Wasch- oder Reinigungsmittel, sowohl die Waschleistung von maschinellen Waschmitteln für Textilien als auch die Reinigungsleistung von maschinellen Geschirrspülmitteln steigert.

Bevorzugt sind dabei solche Verfahren, bei denen die CGTase-Aktivität des eingesetzten erfindungswesentlichen Proteins Cyclodextrine bildet und in diese niedermolekulare Verbindungen, wie beispielsweise Geruchs- oder Giftstoffe einschließt. Solch ein Verfahrensschritt kann auch als "Maskierung" bezeichnet werden.

Gerade maschinelle Reinigungsverfahren zeichnen sich durch ein mehrstufiges Reinigungsprogramm aus, so daß verschiedene reinigungsaktive Komponenten zeitlich voneinander getrennt auf das Reinigungsgut aufgebracht werden können. Solche Verfahren werden beispielsweise bei der Reinigung von gewerblichen Produktionsanlagen für Lebensmittel angewendet. Andererseits besitzt das erfindungswesentliche Enzym aufgrund seiner enzymatischen Aktivität selbst die Fähigkeit, kohlenhydrathaltige Verunreinigungen anzugreifen, und zwar auch in teilweiser oder völliger Abwesenheit von Detergenzien oder anderen für Wasch- oder Reinigungsmittel charakteristischen Inhaltsstoffen. Gemäß einer Ausführungsform der vorliegenden Erfindung kann somit auch ein maschinelles Verfahren zur Reinigung von Textilien oder von festen Stoffen gewählt werden, in welchem das erfindungswesentliche Protein ohne andere reinigungsaktive Komponenten auf die Verunreinigungen einwirkt. Vorzugsweise wird es hierzu mit Stabilisatoren und/oder Puffersubstanzen versetzt.

Bevorzugte Ausführungsformen stellen solche Verfahren zur Reinigung von Textilien oder von harten Oberflächen dar, die dadurch gekennzeichnet sind, daß in wenigstens einem der Verfahrensschritte ein erfindungsgemäßes Mittel eingesetzt wird.

Denn diese sind dadurch gekennzeichnet, daß sie ein erfindungsgemäßes Protein oder Derivat enthalten, vorzugsweise im Rahmen einer Rezeptur, die entsprechend dem oben Gesagten darauf abgestimmt ist.

Bevorzugte Ausführungsformen stellen solche Verfahren zur Reinigung von Textilien oder von harten Oberflächen dar, die dadurch gekennzeichnet sind, daß das amylolytische und/oder CGTase-Aktivität aufweisende Protein oder Derivat in dem betreffenden Verfahrensschritt in einer Menge von 0,035 mg bis 2 000 mg oder zunehmend bevorzugt von 0,07 mg bis 1 900 mg, von 0,1 mg bis 1 800 mg, von 0,15 mg bis 1 700 mg, von 0,2 mg bis 1 600 mg, von 0,3 mg bis 1 500 mg oder von 0,4 mg bis 1 250 mg pro Anwendung eingesetzt wird.

In einer möglichen Ausführungsform maschineller Verfahren zur Reinigung von Textilien öder festen Oberflächen haben sich für ein erfindungsgemäßes Protein aktive Konzentrationen von 0,005 mg bis 10 mg pro I Waschflotte, vorzugsweise von 0,01 mg bis 8 mg pro I Waschflotte als geeignet herausgestellt. In anderen geeigneten Ausführungsformen können sich davon deutlich abweichende Werte ergeben, wenn man berücksichtigt, daß für maschinelle Reinigungsverfahren Geräte in Gebrauch sind, die deutlich unterschiedliche Volumina an Waschflotte bei nahezu identischer Wasch-, beziehungsweise Spülmittelmenge umsetzen. So werden erfindungsgemäße Waschmittel üblicherweise in maschinellen Verfahren in Mengen von 50 bis 100 g in Volumina von 50 bis 100 I und erfindungsgemäße Reinigungsmittel in Mengen von 20 bis 40 g in Volumina von 8 bis 20 I eingesetzt.

Einen weiteren Erfindungsgegenstand stellt die Verwendung eines amylolytischen und/oder CGTase-Aktivität aufweisenden, erfindungsgemäßen Proteins oder Derivats allein oder zusammen mit mindestens einem anderen reinigungsaktiven oder die Reinigungswirkung unterstützenden Wirkstoff zur Reinigung von Textilien oder von harten Oberflächen dar.

Solch eine Verwendung kann maschinell oder auf sonstige, insbesondere manuelle Weise erfolgen. Dies betrifft die Reinigung von allen erdenklichen Materialien, insbesondere von Textilien oder von harten Oberflächen. Sie kann entsprechend dem oben Gesagten eingebettet sein in eine Rezeptur aus anderen waschaktiven Substanzen oder entsprechend der Natur der erfindungswesentlichen Enzyme auch weitgehend ohne solche Verbindungen erfolgen. Aus den obigen Ausführungen ergeben sich entsprechend bevorzugte Ausführungsformen. Hierzu gehören beispielsweise die Verwendung innerhalb eines mehrstufigen Reinigungsverfahrens oder solche Verwendungsmöglichkeiten, bei denen die CGTase-Aktivität des eingesetzten erfindungswesentlichen Proteins Cyclodextrine bildet und in diese niedermolekulare Verbindungen, wie beispielsweise Geruchs- oder Giftstoffe einschließt und somit maskiert.

Einen weiteren Erfindungsgegenstand bildet die Verwendung eines erfindungsgemäßen Mittels zur Reinigung von Textilien oder von harten Oberflächen. Hierfür ergeben sich die bevorzugten Ausführungsform jeweils entsprechend dem oben Gesagten.

Bevorzugte Ausführungsformen sind dadurch gekennzeichnet, daß pro Anwendnung, vorzugsweise pro Anwendung in einer Geschirrspülmaschine oder einer Waschmaschine 0,035 mg bis 2 000 mg oder zunehmend bevorzugt 0,07 mg bis 1 900 mg, 0,1 mg bis 1 800 mg, 0,15 mg bis 1 700 mg, 0,2 mg bis 1 600mg, 0,3 mg bis 1 500 mg oder 0,4 mg bis 1 250 mg des amylolytischen und/oder CGTase-Aktivität aufweisenden Proteins oder Derivats eingesetzt werden.

Eine weitere Ausführungsform stellt die Verwendung eines erfindungsgemäßen Proteins oder Derivats allein oder zusammen mit mindestens einem anderen reinigungsaktiven oder die Reinigungswirkung unterstützenden Wirkstoff in einem aus mehr als einer Phase bestehenden Wasch- oder Reinigungsmittel zur Aktivierung der eigenen oder anderer Phasen dar.

Auf diese Weise werden wie oben dargestellt in entsprechenden Mitteln Schutzschichten um die jeweiligen Bestandteile teilweise oder vollständig aufgelöst oder feste Phasen mit enthaltenen oder umgegebenden Amylase-sensitiven Materialien desintegriert. Besonders bevorzugt ist auch unter diesem Aspekt die Freisetzung der Inhaltsstoffe zur Herbeiführung eines reinigenden Effekts der Inhaltsstoffe auf harte Oberflächen oder textilartige Materialien.

Eine besonders bevorzugte Ausführungsform stellt die Verwendung eines erfindungsgemäßen Proteins oder Derivats allein oder zusammen mit mindestens einem anderen reinigungsaktiven oder die Reinigungswirkung unterstützenden Wirkstoff in einem Wasch- oder Reinigungsmittel mit verkapselten Inhaltsstoffen zur Freisetzung der Inhaltsstoffe aus den Kapseln dar.

Denn durch dessen Aktivität werden in entsprechenden, vorzugsweise flüssigen, gelförmigen oder pastösen Mitteln die teilweise oder vollständige Auflösung von kohlenhydrathaltigen Kapseln, insbesondere Nano-, Mikro- oder Millikapseln herbeigeführt. Dadurch wird der bereits erörterte kontrollierte Freisetzungsprozeß der jeweiligen Bestandteile des Mittels ermöglicht. Vorzugsweise erfolgt die Freisetzung der Inhaltsstoffe zur Herbeiführung eines reinigenden Effekts der Inhaltsstoffe auf eine harte Oberfläche oder ein textilartiges Material.

Einen weiteren Erfindungsgegenstand stellt die Verwendung eines erfindungsgemäßen Proteins oder Derivats zur Behandlung von Rohmaterialien oder Zwischenprodukten in der Textilherstellung dar, insbesondere zum Entschlichten von Baumwolle oder zum Entfernen von Stärke- oder Stärke-ähnlichen Schutzschichten von technischen Zwischenprodukten.

Rohmaterialien der Textilherstellung, beispielsweise für solche auf Baumwollbasis, werden im Rahmen ihrer Herstellung und Weiterverarbeitung mit Stärke ausgerüstet, um eine bessere Verarbeitung zu ermöglichen. Dieses sowohl auf Garne, auf Zwischenprodukte, als auch auf Textilien angewendete Verfahren nennt man Schlichten (sizing). Zur Entfernung der Schlichte, also der stärkehaltigen Schutzschicht (Entschlichten, desizing) sind erfindungsgemäße Proteine geeignet.

Einen weiteren Erfindungsgegenstand stellen Verfahren zur Stärkeverflüssigung, insbesondere zur Ethanolproduktion dar, die dadurch gekennzeichnet sind, daß darin in wenigstens einem Verfahrensschritt ein erfindungsgemäßes Protein oder Derivat eingesetzt wird.

Zur Stärkeverflüssigung wird in Wasser oder Puffer gequollene Stärke mit amylolytischen Enzymen inkubiert, wodurch das Polysaccharid in kleinere Bestandteile, zuletzt überwiegend in Maltose gespalten wird. Erfindungsgemäße Enzyme werden bevorzugt für diesen Prozeß oder einen Teilschritt davon verwendet, wenn sie sich aufgrund ihrer biochemischen Eigenschaften gut in ein entsprechendes Produktionsverfahren einpassen lassen. Dies ist beispielsweise dann der Fall, wenn sie in einem Schritt zusätzlich zu anderen Enzymen eingebracht werden können, die die gleichen Reaktionsbedingungen benötigen und/oder wenn sie in hitzeunempfindlichen Varianten zur Verfügung stehen. Besonders bevorzugt sind erfindungsgemäße amylolytische Proteine, wenn gerade die von ihnen selbst gebildeten Produkte im Zentrum des Interesses stehen. Die Stärkeverflüssigung kann auch einen Schritt in einem mehrstufigen Verfahren zur Gewinnung von Ethanol oder davon abgeleiteten Folgeprodukten, beispielsweise Essigsäure darstellen.

Einen weiteren Erfindungsgegenstand stellt entsprechend dem oben Gesagten auch die Verwendung eines erfindungsgemäßen Proteins oder Derivats zur Stärkeverflüssigung, insbesondere in einem Verfahren zur Ethanolproduktion dar.

Einen weiteren Erfindungsgegenstand stellt die Verwendung eines erfindungsgemäßen Proteins oder Derivats zur Herstellung oder Modifizierung von linearen und/oder kurzkettigen Oligosacchariden dar, insbesondere eines solchen Proteins oder Derivats, dessen CGTase-Aktivität durch Deletion eingeschränkt worden ist.

Es ist bekannt, daß Wildtyp-CGTasen Stärke nur zu einem Teil in Cyclodextrine, zu einem anderen Teil aber auch in lineare Oligosaccharide umwandeln (Wind et al., Eur. J. Biochem., 253 (1998), S. 598 - 605), häufig von solchen mit 2 bis 12 Monomeren. Dementsprechend kann auch das erfindungsgemäße Enzym zur Herstellung oder Modifizierung sowohl von Cyxclodextrinen als auch von linearen Oligosacchariden verwendet werden. Dies kann beispielsweise für die *in* situ-Herstellung entsprechender Verbindungen bei entsprechender Reaktionsführung vorteilhaft sein.

Somit bilden erfindungsgemäße amylolytische Proteine insbesondere wenn ihre Fähigkeit zur Transglykosylierung, das heißt zur Cyclodextrin-Bildung, beispielsweise durch Deletionsmutagense unterbunden worden ist, aus stärkeähnlichen Polymeren nach kürzerer Inkubationszeit überwiegend höhermolekulare Oligosaccharide, wie beispielsweise Maltohexaose, Maltoheptaose oder Maltooctaose. Nach längerer Inkubationszeit steigt unter den Reaktionsprodukten der Anteil niedrigerer Oligosaccharide, wie beispielsweise Maltose oder Maltotriose an. Bei besonderem Interesse an bestimmten Reaktionsprodukten können entsprechende Varianten erfindungsgemäßer Proteine verwendet und/oder die Reaktionsbedingungen entsprechend gestaltet werden. Dies ist insbesondere dann attraktiv, wenn es nicht auf reine Verbindungen, sondern auf Gemische ähnlicher Verbindungen ankommt, wie beispielsweise bei der Bildung von Lösungen, Suspensionen oder Gelen mit lediglich bestimmten physikalischen Eigenschaften.

Einen weiteren Erfindungsgegenstand stellt die Verwendung eines erfindungsgemäßen Proteins oder Derivats zur Herstellung, Modifizierung oder Hydrolyse von Cyclodextrinen.

Aufgrund ihrer CGTase-Aktivität eignen sich erfindungsgemäße Proteine oder Derivate zur Herstellung, Modifizierung oder Hydrolyse von Cycxlodextrinen. Dies geschieht beispeilsweise durch wechselseitige Umwandlung, oder durch Bildung anderer Cyclodextrine. Diese Reaktionen können geeigenterweise im Zusammenspiel anderen, auf Mono- und/oder Oligosaccharide einwirkenden Enzymen durchgeführt werden.

In einer bevorzugten Ausführungsform erfolgt diese Verwendung zur Aufnahme oder Freisetzung von nieder- oder höhermolekularen Verbindungen in, beziehungsweise aus Polysaccharidträgern, insbesondere Cyclodextrinen.

Cyclodextrine eignen sich aufgrund ihres, sich durch ein gewisses Maß an Hydrophobizität auszeichnenden Innenraums zur Aufnahme wenig hydrophiler Verbindungen, besonders dann, wenn sie im Kristallgitter so aufeinandergeschichtet sind, daß sie durchgehende innermolekulare Kanäle bilden. Bei den hydrophoben Gastmolekülen kann es sich beispielsweise um Duftstoffe, Farbstoffe, kosmetisch oder pharmazeutisch aktive oder vergleichbare niedermolekulare Verbindungen handeln. Cyclodextrine können somit die Funktion erfüllen, die jeweiligen Inhaltsstoffe vorübergehend aufzunehmen und wieder kontrolliert freisetzen, etwa nach mechanischem, hydrolytischen oder enzymatischen Aufbrechen ihrer kristallinen Struktur.

Erfindungsgemäße Proteine oder Derivate, insbesondere solche mit CGTase-Aktivität können sowohl bei der Herstellung oder Verarbeitung solcher Einschlußverbindungen als auch bei der Freisetzung der Inhaltsstoffe verwendet werden. Zur Herstellung können beispielsweise Stärke oder stärkeähnliches Polysaccharid und gleichzeitig die niedermolekulare Verbindung mit einem erfindungsgemäßen Enzym inkubiert werden, so daß im Augenblick der Cycxlodextrinherstellung die niedermolekulare Verbindungen sogleich eingelagert werden. Die Freisetzung kann in Umkehrung der Bildungsreaktion erfolgen, indem auf das einen Inhaltsstoff enthaltende Cyclodextrinpräparat die CGTase oder ein anderes, die Cyclodextrine hydrolysierendes Enzym aufgebracht wird, welche die Einschlußverbindungen hydrolysiert und damit den Inhaltsstoff freisetzt.

In ähnlicher Weise können erfindungsgemäße amylolytische Proteine niedermolekulare Verbindungen auch aus anderen α-(1,4)-glycosidisch verknüpften Polysacchariden freisetzen, beispielsweise wenn die Inhaltsstoffe in Form von Mikrokapseln verkapselt vorgelegt werden. Beispielsweise Stärke ist ein im Stand der Technik etabliertes Material, um Verbindungen wie beispielsweise Enzyme, die in definierten Mengen in Reaktionsansätze eingebracht werden sollen, während der Lagerung einzukapseln. Der kontrollierte Freisetzungsprozeß aus derartigen Kapseln kann von erfindungsgemäßen amylolytischen und/oder CGTase-Funktion aufweisenden Enzymen unterstützt werden.

In einer bevorzugten Ausführungsform erfolgt diese Verwendung zur Stabilisierung von chemischen Verbindungen während ihrer Herstellung oder Verarbeitung.

Die Einlagerung hydrophober Verbindungen in Cyclodextrine kann in der Chemie beispielsweise zur Festphasenextraktion, zur Reaktionskatalyse oder zur Enantiomerentrennung eingesetzt werden. Sie kann ferner genutzt werden, um in der chemischen Synthese empfindliche Verbindungen, wie beispielsweise solche, die gegen Oxidation oder Photolyse empfindlich sind, aber auch leicht flüchtige, schwer lösliche oder unangenehm riechende Verbindungen einzuschließen. Sie können dadurch beispielsweise gegen die Umwelteinflüsse geschützt werden, festgehalten oder solubilisiert werden. Dies kann vorzugsweise während der Synthese solcher Verbindungen oder ihrer Weiterverarbeitung durchgeführt werden, beispielsweise bei der der physikalischen Aufbringung solcher Verbindungen auf feste Träger. Für die Herstellung derartiger Einschlußverbindungen sind erfindungsgemäße Proteine oder Derivate mit CGTase-Aktivität geeignet.

In einer bevorzugten Ausführungsform erfolgt diese Verwendung zur Herstellung oder als Bestandteil von Kosmetika oder Pharmaka.

Denn die Cyclodextrine können entsprechend dem oben Gesagten sowohl zur Herstellung der entsprechenden Verbindungen als auch zu deren Lagerung, beziehungsweise Verkapselung oder Maskierung eingesetzt werden. Vorzugsweise ermöglichen sie darin eine kontrollierte Freisetzung der Wirkstoffe. Die Verwendung eines amyloytischen oder CGTase-Aktivität aufweisenden Enzyms selbst in der Kosmetik ist dann besonders sinnvoll, wenn auf ein Polysaccharid-Substrat eingewirkt werden soll, beispielsweise zur Entfernung von Zahnplaque.

Einen weiteren Erfindungsgegenstand stellen dementsprechend auch die Kosmetika oder Pharmaka selbst dar, die dadurch gekennzeichnet sind, daß sie ein erfindungsgemäßes Protein oder Derivat enthalten oder wenigstens ein Bestandteil unter Einsatz eines solchen Proteins hergestellt worden ist, insbesondere nach einer der oben beschriebenen Verwendungen.

Einen weiteren Erfindungsgegenstand stellt die Verwendung eines erfindungsgemäßen Proteins oder Derivats zur Herstellung von Lebensmitteln und/oder Lebensmittelbestandteilen dar.

Wo immer Stärke eine Rolle als Nahrungsbestandteil eine Rolle spielt, kann eine amylolytische Aktivität zu ihrer Herstellung zum Einsatz kommen. Sie erhöht den Anteil von Mono-, oder Oligomeren gegenüber dem polymeren Zucker, was beispielsweise dem Geschmack, der Verdaubarkeit oder der Konsistenz des Lebensmittels zugute kommt. Dies ist beispielsweise bei der Herstellung von Fruchtsäften oder Wein erforderlich, wenn der Anteil polymerer Zucker verringert und der von süßen und/oder leichter löslichen Zuckern erhöht werden soll.

Amylasen und insbesondere CGTasen verhindern darüberhinaus auch den unter Altbacken-Werden bekannten Geschmacksverlust von Backwaren (Anti staling-Effekt). Dafür werden sie geeigneterweise dem Teig vor dem Backen zugesetzt. Weitere bevorzugte Ausführungsformen der vorliegenden Erfindung sind also solche, bei denen das erfindungsgemäße Proteine oder Derivate zur Herstellung von Backwaren verwendet wird.

Die Bildung von Cyclodextrinen kann bei der Lebensmittelherstellung beispielsweise auch dazu verwendet werden, um ungewünschte, hydrophobe Nahrungsmittelbestandteile wie Cholesterin oder Fett aus dem betreffenden Lebensmittel zu entfernen.

Einen weiteren Erfindungsgegenstand stellt die Verwendung eines erfindungsgemäßen Proteins oder Derivats zur Herstellung von Tierfutter und/oder Tierfutterbestandteilen dar.

Denn auch Tierfutter, beispielsweise in der Tiermast können entsprechend dem oben für Nahrungsmittel Gesagten hergestellt oder verbessert werden.

Einen weiteren Erfindungsgegenstand stellt die Verwendung eines erfindungsgemäßen Proteins oder Derivats zur Papierrestaurierung dar.

Neben anderen Naturstoffen wird auch Stärke bereits seit Jahrhunderten als Bindemittel in der Papierherstellung und dem Verkleben unterschiedlicher Papiere und Pappen verwendet. Dies betrifft beispeilsweise Graphiken und Bücher. Im Laufe langer Zeiträume können solche Papiere unter ungünstigen Einflüssen, wie beispielsweise Feuchtigkeit, Wellen aufwerfen oder brechen, was bis zu deren völliger Zerstörung führen kann. Bei der Restaurierung solcher Papiere und Pappen kann das Auflösen der Klebeschichten erforderlich sein, welches durch Verwendung eines erfindungsgemäßen amylolytischen Proteins erheblich erleichtert wird.

Einen weiteren Erfindungsgegenstand stellt die Verwendung eines erfindungsgemäßen Proteins oder Derivats zur Herstellung oder Auflösung Stärke oder ähnliche Verbindungen enthaltender Klebeverbindungen dar.

Pflanzliche Polymere wie Stärke oder Cellulose und deren wasserlösliche Derivate davon werden unter anderem auch als Klebstoffe oder Kleister verwendet. Dafür müssen sie in Wasser zunächst quellen und nach Aufbringen auf dem Klebegut trocknen, wodurch dieses am Untergrund fixiert wird. Erfindungsgemäße Proteine oder Derivate können solch einer wäßrigen Suspension zugesetzt werden, um die Klebeeigenschaften des entstehenden Kleisters zu beeinflussen. Sie können aber auch stattdessen oder zusätzlich zu dieser Funktion dem Kleister zugesetzt werden, um nach dem Antrocknen für lange Zeit, beispielsweise einige Jahre, inaktiv auf dem Klebegut zu verharren. Gezieltes Ändern der Umgebungsbedingungen, beispielsweise durch Anfeuchten, kann dann dazu verwendet werden, um sie zu einem späteren Zeitpunkt zu aktivieren und damit ein Auflösen des Kleisters herbeizuführen. Auf diese Weise ist das Klebegut leichter wieder vom Untergrund abzulösen. In dieser Verwendung fungieren erfindungsgemäße Proteine oder Derivate als scheidendes Agens in einem temporären Klebeverfahren oder als sogenannter "Schalter" zum Ablösen des Klebeguts.

Ebenso kann gerade auch die CGTase-Aktivität erfindungsgemäßer Proteine oder Derivate zur Synthese solcher Klebstoffe Verwendung finden.

Einen weiteren Erfindungsgegenstand bilden entsprechend dem oben Gesagten auch die temporären Klebeverfahren selbst, die dadurch gekennzeichnet sind, daß in wenigstens einem Verfahrensschritt ein erfindungsgemäßes Protein oder Derivat eingesetzt wird.

### Beispiele

### Beispiel 1

### Nachweis einer Amylase aus B. agaradherens

Im Rahmen eines mikrobiellen Screenings auf amylasebildende Mikroorganismen mit dem Auswahlkriterium Wachstum und Hofbildung auf Agarplatten mit 1 % Maisstärke als einziger Kohlenstoff-Quelle wurde der Bacillus-Stamm *Bacillus agaradherens* (DSM ID 94-759, Hinterlegung DSM 9948) als Amylasebildner identifiziert.

Die Anzucht erfolgte in Flüssigmedium bestehend aus 10 g/l lösliche Stärke, 20 g/l Pepton, 1 g/l Hefeextrakt, 1 g/l K₂HPO₄ und 5 g/l NaCl. Der pH-Wert wurde nach dem Autoklavieren mit 20%iger Natriumcarbonatlösung auf 10 eingestellt. 25 ml Medium wurden in sterile 100ml-Erlenmeyerkolben mit Schikane eingefüllt und mit einer Kultur *B. agaradherens* (DSM 9948) beimpft, die auf einer Maisstärkeagarplatte gewachsen war. Die Anzucht erfolgte bei 30°C über 48 h unter Schütteln bei 140 rpm.

Durch Zentrifugation der Kultur konnte die Amylase-Aktivität im Überstand von der Zellmasse abgetrennt und näher untersucht werden.

### Beispiel 2

### Klonierung

Alle einzelnen Arbeitsschritte für die Klonierung folgen Standardmethoden, wie sie beispielsweise in dem Handbuch von Fritsch, Sambrook und Maniatis "Molecular cloning: a laboratory manual", *Cold Spring Harbour Laboratory Press,* New York, 1989, angegeben sind.

Identifiziert wurde das zugehörige Gen über eine "shot-gun"-Klonierungsmethode. Dafür wurde zunächst chromosomale DNA des Stammes *B. agaradherens* (DSM 9948) präpariert und mit dem Enzym *Sau* 3A inkubiert. Nach der Restriktion wurden die erhaltenen Fragmente mit einem Molekulargewicht zwischen 1,5 und 5 kb in die *Bam* HI-und *Bg*/II-Restriktionsschnittstellen des Vektors pCB76C mit einem Kanamycin-Resistenz-Gen als Selektionsmarker ligiert; dieser Vektor ist bereits aus der Anmeldung WO 91/02792 bekannt.

Der auf diese Weise erhaltene Vektor wurde in eine Amylase-negative Mutante des Stamms *Bacillus subtilis* DB104 transformiert, die durch eine teilweise chromosomale Deletion des AmyE-Gens erhalten worden war. Positive Klone konnten durch Hofbildung auf Amylose-haltigen Agarplatten identifiziert werden. Deren Insert-haltige Plasmide wurden isoliert und die enthaltenen Inserts sequenziert.

### Beispiel 3

### Sequenzierung

Die Sequenzierung erfolgte mithilfe eines herkömmlichen Kits nach der Kettenabbruchmethode. Mehrere unabhängige, sequenzierte Klone enthielten ein Insert mit einer Größe von 2.621 bp. Darauf liegt das 2.142 bp umfassende Gen für das interessierende Enzym. Dessen Sequenz ist in SEQ ID NO. NO. 1 im Sequenzprotokoll zur vorliegenden Anmeldung angegeben. Dem entspricht ein Polypeptid von 713 Aminosäuren, einschießlich eines ca. 34 Aminosäure langen Signalpeptids. Die von der DNA-Sequenz abgeleitete Aminosäure-Sequenz ist in SEQ ID NO. NO. 2 im Sequenzprotokoll angegeben. Das abgeleitete Molekulargewicht des Proteins beträgt 88,9 kD und ohne Berücksichtigung des Signalpeptids 76,4 kD.

### Beispiel 4

### Homologien

Sequenzvergleiche wurden nach der FASTA-Methode (W. R. Pearson, D. J. Lipman, PNAS (1988) 85, S. 2444-2448) am 25.5.2000 über den Server des EMBL-European Bioinformatics Institute (EBI) in Cambridge, Großbritannien (http://www.ebi.ac.uk) durchgeführt, und zwar mit dem Programm Fasta3 (Matrix: blosum62 und default parameters). Sie charakterisieren das Enzym als Cyclodextringlucanotransferase (CGTase), welche naturgemäß auch über eine amylolytische Funktion verfügt.

Die Homologiewerte dieses Enzyms zu den mit dieser Suche identifizierten, am engsten verwandten, bekannten Enzymen liegen, wie folgende Tabelle 2 zeigt, bei weniger als 57% Identität und bei weniger als 70% identischer und konservierter Aminosäuren. Über die in der Tabelle unter der Bezeichnung EMBL/SW-Identifier angegebenen Bezeichnungen können die entsprechenden vollständigen Sequenzen über jede öffentlich zugängliche Datenbank, wie beispielsweise die von Datenbank Swiss-PROT (Geneva Bioinformatics, Genf, Schweiz; http://www.genebio.com/sprot.html) erhalten werden.

**Tabelle 2: Ergebnis des FASTA-Vergleichs der CGTase aus B. agaradherens (DSM 9948, CDGT_BACAG) mit den am engsten verwandten, bisher bekannten CGTasen.**

| **Organismus** | **EMBUSW-Identifier** | **Protein-Bezeichnung** | **Identität [%]** | **identische u. konservierte Aminosäuren [%]** |
|---|---|---|---|---|
| *Bacillus stearothermophilus* | Q9ZAQO | CDGT_BACST | 56 | 68 |
| *Thermoanaerobacter thermosulfurogenes* | P26827 | CDGT_THETU | 56 | 68 |
| *Bacillus ohbensis* | P 27036 | CDGT_BACOH | 53 | 66 |
| *Bacillus sp.* Strain 1011 | P 30921 | CDGT_BACSP | 54 | 67 |

Ein Alignment mit den nächstähnlichen Proteinen ist in Figur 1 dargestellt. Es zeigt die drei für die α-Amylase-Aktivität verantwortlichen Domänen A, B und C (Positionen 35 bis 526 gemäß der SEQ ID NO. 2) und die beiden für die Wechselwirkung mit dem Substrat und für die Cyclisierung der anschließend freigesetzten Cyclodextrine verantwortlichen Domänen D und E; letztere in den Positionen 527 bis 713. Der Übergangsbereich zwischen den Domänen C und D ist nahe der Position 530 durch einen Doppelpfeil gekennzeichnet; die Aminosäurefolge VWE (Positionen 524 bis 526 gemäß der SEQ ID NO. 2) wird noch der Domäne C zugerechnet, während die Aminosäurefolge PSI (Positionen 535 bis 537 gemäß der SEQ ID NO. 2) bereits der Domäne D zugerechnet wird. Es handelt sich demnach also auch hinsichtlich der Primärstruktur um eine Cyclodextrin-Glucanotransferase (CGTase), welche naturgemäß über die Aktivität einer α-Amylase verfügt.

Das zu den Domänen A bis C der CGTase aus *Bacillus agaradherens* (DSM 9948) nächstähnliche Enzym, eine CGTase, verfügt zu diesem über eine Homologie von 60,1 % Identität auf Aminosäure-Ebene. Auf DNA-Ebene verfügt das nächstähnliche Enzym über eine Homologie von 61,1 % Identität.

### Beispiel 5

### Kultivierung

Mit einer Einzelkolonie des Laborstamms *B. subtilis* DB 104 mit der klonierten CGTase wurden 100 ml MLBSP-Medium (10 g/l Casiton, Fa. Becton Dickinson, Cockeysville, USA; 20 g/l Trypton, Fa. Becton Dickinson, Cockeysville; 10 g/l Hefeextrakt, Fa. Becton Dickinson, Cockeysville; 5 g/l NaCl; 27 g/l Natrium-Succinat; 100 mg/l MgSO₄*7 H₂O; 75 mg/l CaCl₂*2 H₂O; 0,5 µM MnCl₂; 0,5 µM FeSO₄; 2 %(w/v) Glucose; 50 mM PIPES-Puffer (aus einer 1 M Stammlösung mit pH 7,2); 75 mM KPO₄ (aus einer 1,5 M Stammlösung mit pH 7,0); pH = 7,0, eingestellt mit KOH, sowie 25 mg/l Kanamycin) angeimpft und in einem 250 ml-Schüttelkolben für 24 h bei 37°C und 200 Umdrehungen pro Minute inkubiert. Mit 80 ml von dieser Vorkultur wurde ein herkömmlicher Laborfermenter mit 8 l MLBSP-Medium angeimpft und bei 37°C unter für Bacillus-Spezies üblichen Bedingungen fermentiert. Nach Erreichen der stationären Wachstumsphase konnte aus dem Kulturüberstand das sekretierte amylolytische Enzym gewonnen werden.

### Beispiel 6

### Gewinnung, Aufreinigung

Über folgende Aufreinigungsschritte wurde aus dem Kulturmedium ein singuläres Enzym erhalten: Fällung des Kulturüberstands mit Ethanol; Aufnahme des Proteinpellets in 50 mM Tris/HCI-Puffer, pH 8,6; lonenaustauschchromatographie an Q-Sepharose^{®} (Fa. Pharmacia-Amersham Biotech, Schweden), Umpufferung durch Dialyse in 50 mM Tris/HCI-Puffer, pH 8,0; lonenaustauschchromatographie über Mono-Q^{®} (Fa. Pharmacia-Amersham Biotech, Schweden). Aus 100 ml Kulturmedium wurden auf diese Weise, wie über die BCA-Methode (Bicinchoninsäure; 2,2' -Bichinolyl-4,4'-dicarbonsäure) ermittelt wurde, 1,4 mg eines gemäß SDS-Polyacrylgelelektrophorese und Coomassie-Färbung reinen Proteins gewonnen.

### Beispiel 7

### SDS-Polyacrylgelelektrophorese und isoelektrische Fokussierung

Bei denaturierender SDS-Polyacrylgelelektrophorese in einem 8 - 25%igem Gel, im PHAST^{®}-System der Fa. Pharmacia-Amersham Biotech, Schweden, und Vergleich mit relevanten Größenmarkern weist das native amylolytische Enyzm aus B. *agaradherens* (DSM 9948) ein apparentes Molekulargewicht von 89 kD auf.

Gemäß isoelektrischer Fokussierung von pH 3 bis 9 im PHAST^{®}-System der Fa. Pharmacia-Amersham liegt der isoelektrische Punkt des nativen amylolytischen Enyzms aus *B. agaradherens* (DSM 9948) bei 6,0.

### Beispiel 8

### CGTase-Aktivität

Der Nachweis einer CGTase-Aktivät beruht auf der selektiven Komplexbildung von Cyclodextrinen mit Methylorange im sauren Medium; bei Anwesenheit einer CGTase-Aktivät ändert sich die Extinktion einer entsprechenden Substratlösung. Die Substratlösung wird folgendermaßen angesetzt: 0,011 g Methylorange (0,3 mmol), 0,680 g Imidazol (100 mmol), 0,074 g Calciumchlorid (5 mmol), und 1 g Maltotriose oder 1 g Stärke in 100 ml Wasser (bidest) lösen und den pH-Wert mit HCl auf 6,8 einstellen. Zur Messung werden 1 ml Substratlösung und 0,5 ml der zu testenden Enzymlösung bei 50°C über 60 min inkubiert und die Reaktion durch Zugabe von 20µl HCl (2 M) gestoppt; der Negativkontrolle ohne Enzym werden erst zu diesem Zeitpunkt 0,5 ml Enzymlösung zugegeben und alle Proben für 10 min im Eisbad gekühlt. Abschließend erfolgt die Messung der Absorption bei 505 nm gegen Luft im Photometer. Zur Auswertung wird die CGTase-Aktivität über die gebildete Menge an Cyclodextrinen bestimmt, indem die gemessenen Aktivitäten auf eine Eichkurve (0,2 - 3 mg/ml β-Cyclodextrin) bezogen werden. Dabei wird der Wert der Negativkontrolle von dem jeweiligen Proben-Wert abgezogen.

Es wurden nach dieser Methode folgende vier Enzymlösungen getestet: (1.) Kulturüberstand von *B. agaradherens* (DSM 9948; aus Beispiel 1), (2.) 1 %ige CGTase-Lösung (Fa. Wacker-Chemie GmbH, München), (3.) 1 %ige CGTase-Lösung (Fa. Amano Enzyme Europe Ltd., Milton Keynes, UK) und (4.) 1 %ige Termamyl^{®}-Lösung (Fa. Novozymes A/S, Bagsværd, Dänemark). Die Ergebnisse sind in folgender Tabelle 3 zusammengestellt:

### Tabelle 3: CGTase-Aktivität verschiedener Cyclodextrin-Glucanotransferasen an verschiedenen Substraten.

| **Probe** | **CGTase-Aktivität (mU/g) am Substrat Stärke** | **CGTase-Aktivität (mU/g) am Substrat Maltotriose** |
|---|---|---|
| Kulturüberstand von *B. agaradherens* (DSM 9948) | 299 | 416 |
| 1% CGTase (Fa. Wacker) | 3022 | 2423 |
| 1 % CGTase (Fa. Amano) | 2112 | 3214 |
| 1 % Termamyl^{®} (Fa. Novozymes) | 0 | 0 |

Während für die α-Amylase Termamyl^{®} auf diese Weise erwartungsgemäß keine CGTase-Aktivität nachgewiesen werden konnte, zeigt bereits der Kulturüberstand von *B. agaradherens* (DSM 9948) eine deutlich meßbare Cyclodextrin-Bildungsrate. Die Anwendbarkeit dieser Nachweismethode bestätigen die Positivkontrollen von kommerziell erhältlichen CGTasen.

### Beispiel 9

### Sonstige biochemische Eigenschaften

### Amylase-Aktivität

Der Test auf Amylase-Aktivität erfolgt nach der Amylasebestimmungsmethode mit dem "QuickStart^{®}" Kit der Fa. Abbott GmbH Diagnostika, Wiesbaden-Delkenheim. Als Substrat dient ein blockiertes p-Nitrophenylmaltoheptaosid, welches durch die Amylase hydrolysiert wird. Erst durch diese Spaltung kann nachfolgend durch Glucoamylase und Glucosidase der Farbstoff p-Nitrophenol (pNP) freigesetzt werden. Die Intensität der Farbentwicklung pro Zeiteinheit ist proportional der Aktivität der Amylase. Der Test wird folgendermaßen durchgeführt: Nach Vorschrift des Herstellers wird das Substratreagenz (0,8 ml Reagenz 1 + 20 ml Reagenz 2) hergestellt. Dieses wird mit bidestilliertem Wasser 1:2 zum sogenannten Arbeitsreagenz verdünnt. 980 µl Arbeitsreagenz werden dann mit 20 µl Enzymlösung (Verdünnungen in 44,1 mg Calciumchloriddihydrat + 247,5 µg Brij 35^{®} (Fa. Fluka, Deisenhofen) ad 1 l bidestilliertem Wasser) versetzt und bei 37°C in einem temperierbaren Photometer vorinkubiert. Die Aufnahme der Absorptionskinetik erfolgt bei 37°C über 3,5 Minuten (gemessen wird in 30 Sekunden-Intervallen) bei 405 nm gegen einen Blank-Wert. Die Kalibrierung des Assays erfolgt über eine Enzymkonzentrationsreihe eines Standardenzyms. Die Aktivität wird angegeben in Aktivität bezogen auf den bekannten Standard (zum Beispiel TAU = termostabile Amylase Einheit) pro ml.

Im Folgenden dient die auf diese Weise bestimmbare Amylase-Aktivität als Parameter für die Stabilität des Enzyms unter jeweils verschiedenen Bedingungen.

### Temperatur-Stabilität

Die Temperaturstabilität des wie oben beschrieben gereinigten nativen amylolytischen Enzyms aus B. *agaradherens* (DSM 9948) wurde bei 10minütiger Inkubation bei einem pH-Wert von 10 gemessen. Die amylolytische Aktivität beträgt bei 30°C mindestens 85 % und bei 40°C mindestens 80 % Restaktivität gegenüber einer nicht inkubierten Probe mit 100% Ausgangsaktivität. Bei 50°C liegt sie bei 70 % Restaktivität. Bei 60°C ist dieses Wildtyp-Enzym inaktiv.

### pH-Stabilität

Das amylolytische Enyzm aus *B. agaradherens* (DSM 9948) ist weitgehend stabil bei pH-Werten zwischen 5 und 12, wenn es jeweils für 10 min bei 40°C inkubiert wird.

Das pH-Optimum seiner amylolytischen Aktivtät liegt mit 96 % Restaktivität gegenüber einer nicht inkubierten Probe mit 100 % Ausgangsaktivität bei einem pH-Wert von 12. Bei pH-Werten unterhalb 12 fällt die Aktivität geringfügig ab, so daß das Enzym bei dem sauren pH-Wert von 5 noch eine Restaktivität von 80 % aufweist.

### Stabilität gegenüber Tensiden und Protease

Zur weiteren Charakterisierung wurde die Beeinträchtigung der enzymatischen Aktivität durch möglicherweise störende Faktoren wie Protease oder Tensiden untersucht: Nach Einwirken einer Protease, nämlich von 0,66 Novo-Protease units (NPU) von Savinase^{®} 4.0 T (Fa. Novozymes A/S, Bagsværd, Dänemark) pro ml und 0,1% SDS bei pH 10 für 15 Minuten und 50°C zeigt das Enzym eine Restaktivität von 49 %. In Gegenwart von 3 mM EDTA anstelle von SDS und Protease, ansonsten aber unter gleichen Bedingungen zeigt es noch 20 % seiner amylolytischen Aktivität.

### Beispiel 10

Baumwolltextilien wurden standardisiert mit den vier verschiedenen Anschmutzungen A (Schokoladenpudding), B (Haferflocken mit Kakao), C (Haferflocken mit Kakao und wenig Milch) und D (Kartoffelstärke) versehen und anhand des auf diese Weise präparierten Materials verschiedene Waschmittelrezepturen launderometrisch auf ihre Waschleistung hin untersucht. Dafür wurde jeweils ein Flottenverhältnis von 1:12 eingestellt und für 30 min bei einer Temperatur von 30°C gewaschen. Die Dosierung lag bei 5,88 g des jeweiligen Waschmittels pro I Waschflotte. Die Wasserhärte betrug 16° deutscher Härte.

Als Kontroll-Waschmittel diente für A, B und C eine Waschmittel-Basis-Rezeptur folgender Zusammensetzung (alle Angaben in Gewichts-Prozent): 4 % lineares Natrium-Alkylbenzolsulfonat (Natrium-Salz), 4 % C₁₂-C₁₈-Fettalkoholsulfat (Natrium-Salz), 5,5 % C₁₂-C₁₈-Fettalkohol mit 7 EO, 1 % Natrium-Seife, 11 % Natriumcarbonat, 2,5 % amorphes Natriumdisilikat, 20 % Natriumperborat-Tetrahydrat, 5,5 % TAED, 25 % Zeolith A, 4,5 % Polycarboxylat, 0,5 % Phosphonat, 2,5 % Schauminhibitorgranulat, 5 % Natriumsulfat, 1 % Proteasegranulat, Rest: Wasser, optischer Aufheller, Parfüm, Salze. Sie wurde für die verschiedenen Versuchsreihen mit verschiedenen Amylasen versetzt, so daß sich jeweils eine Endkonzentration von 5,5 mg an amylolytischem Enzym pro I Waschflotte ergab. Mit dem erfindungswesentlichen amylolytischen Enzym, der CGTase aus *Bacillus agaradherens* (DSM 9948) wurden Termamyl^{®}, Duramyl^{®} und BAN^{®} (Hersteller jeweils: Novozymes A/S, Bagsværd, Dänemark) verglichen. Für die Anschmutzung D wurde dieselbe Basis-Rezeptur verwendet, allerdings ohne Protease, und wie A - C als Kontrolle verwendet, beziehungsweise mit Amylasen versetzt.

Der Weißheitsgrad der Textilien wurde im CIELAB-System mit dem Meßgerät Minolta CR 310 vor und nach dem Waschen im Vergleich zu einem Standard gemessen, der auf 100 % normiert wurde. Die Differenzen aus den erhaltenen Werten werden für die jeweiligen Versuche in folgender Tabelle 4 zusammengestellt. Angegeben sind die Mittelwerte aus jeweils 5 Messungen. Sie erlauben einen unmittelbaren Rückschluß auf den Beitrag des enthaltenen Enzyms auf die Waschleistung des verwendeten Mittels.

**Tabelle 4:**

| **Basis-Waschmittel mit** | **A** | **B** | **C** | **D** |
|---|---|---|---|---|
| | | | | |
| **erfindungswesentl. Enzym** | 40,5 | 25,6 | 21,1 | 13,6 |
| **Termamyl**^{®} | 40,3 | 22,7 | 17,4 | 12,3 |
| **Duramyl^{®}** | 41,1 | 23,4 | 20,2 | 14,3 |
| **BAN^{®}** | 39,1 | 22,0 | 17,0 | 13,2 |
| **Kontrolle ohne Amylase** | 37,0 | 22,2 | 11,9 | 10,0 |
| Standardabw. | 0,5 | 0,6 | 1,4 | 2,2 |

Man erkennt, daß die CGTase aus *Bacillus agaradherens* (DSM 9948) an der Anschmutzung B besser zur Waschleistungen beiträgt als jedes der drei Referenzenzyme, und das obwohl im Waschmittel ein Bleichmittel enthalten ist, auf welches enthaltene Enzyme im allgemeinen sehr empfindlich reagieren. Bei den übrigen Anschmutzungstypen zeigt sie im Rahmen der Fehlergrenze mindestens vergleichbare Beiträge, die immer deutlich über den Vergleichswerten ohne amylolytisches Enzym liegen.

### Beispiel 11

Baumwoll-Textilien wurden standardisiert mit der Anschmutzung C (Haferflocken mit Kakao und wenig Milch) versehen. Die launderometrische Untersuchung erfolgte wie in Beispiel 1, unter Verwendung einer anderen Waschmittel-Basis-Rezeptur, nämlich jeweils in Gewichts-Prozent: 14 % Na-Alkylbenzolsulfonat, 6 % Na-Fettalkoholsulfonat, 6 % 7-fach ethoxylierter C₁₂-C₁₈-Fettalkohol, 1 % Seife, 25 % Zeolith Na-A, 10 % Na-Carbonat, 5 % polymeres Polycarboxylat (Sokalan CP5), 11 % Trinatriumcitrat-dihydrat, 4 % Citronensäure, 1 % teilchenförmig konfektionierter Schauminhibitor, 1 % Proteasegranulat, 5 % Natriumsulfat, Rest: Wasser und Salze. Diese Basis-Rezeptur wurde für die verschiedenen Versuchsreihen mit verschiedenen Amylasen versetzt, so daß sich jeweils eine Endkonzentration von 4,15 mg an amylolytischem Enzym pro 1 Waschflotte ergab. Mit dem erfindungswesentlichen amylolytischen Enzym, der CGTase aus *Bacillus agaradherens* (DSM 9948) wurden Termamyl^{®}, Duramyl^{®} und BAN^{®} (Hersteller jeweils: Novozymes A/S, Bagsværd, Dänemark) verglichen. Die Dosierung lag bei 4,45 g des jeweiligen Waschmittels pro I Waschflotte.

Nach dem Waschen wurde der Weißheitsgrad der gewaschenen Textilien wie in dem vorangegangenen Beispiel bestimmt. Die jeweils erhaltenen Differenzwerte werden in folgender Tabelle 5 zusammengestellt. Es handelt sich jeweils um die Mittelwerte aus 5 Messungen, welche wiederum einen unmittelbaren Rückschluß auf den Beitrag des jeweiligen Enzyms auf die Waschleistung des Mittels zulassen.

**Tabelle 5:**

| **Basis-Waschmittel mit** | **C** |
|---|---|
| | |
| **erfindungswesentl. Enzym** | 18,0 |
| **Termamyl^{®}** | 15,0 |
| **Duramyl^{®}** | 16,7 |
| **BAN^{®}** | 15,6 |
| **Kontrolle ohne Amylase** | 14,5 |
| Standardabw. | 1,2 |

Man erkennt, daß die CGTase aus *Bacillus agaradherens* (DSM 9948) in dieser bleichmittelfreien Waschmittel-Rezeptur einen besseren Beitrag zur Waschleistung als jedes der Referenzenzyme erbringt.

### Beispiel 12

Baumwoll-Textilien wurden standardisiert mit der Anschmutzung E (Milchkakao) versehen. Damit erfolgte die launderometrische Untersuchung wie in Beispiel 10 beschrieben. Als Kontroll-Waschmittel diente die Waschmittel-Basis-Rezeptur des Beispiels 11, jedoch ohne Protease. Sie wurde wie in Beispiel 11 mit den verschiedenen Amylasen versetzt und in gleicher Dosierung eingesetzt.

Nach dem Waschen wurde der Weißheitsgrad der gewaschenen Textilien im Vergleich zu dem von Bariumsulfat gemessen, der auf 100 % normiert wurde. Die Messung erfolgte an einem Spektrometer Datacolor SF500-2 bei 460 nm (UV-Sperrfilter 3), 30 mm Blende, ohne Glanz, Lichtart D65, 10°, d/8°. Die erhaltenen Ergebnisse werden als % Remission, das heißt als Prozentangaben im Vergleich zu Bariumsulfat in folgender Tabelle 6 zusammengestellt. Der Anfangswert lag bei 21,1%. Angegeben sind die Mittelwerte aus jeweils 5 Messungen. Sie erlauben einen unmittelbaren Rückschluß auf den Beitrag des jeweils enthaltenen amylolytischen Enzyms auf die Waschleistung des verwendeten Mittels.

**Tabelle 6:**

| **Basis-Waschmittel mit** | **E** |
|---|---|
| | |
| **erfindungswesentl. Enzym** | **69,8** |
| **Termamyl^{®}** | **67,3** |
| **Duramyl^{®}** | **68,3** |
| **BAN^{®}** | **68,7** |
| **Kontrolle ohne Amylase** | **61,1** |
| Standardabw. | 1,0 |

Man erkennt, daß die CGTase aus *Bacillus agaradherens* (DSM 9948) in dieser Rezeptur in keinem Fall einen geringeren Beitrag zur Waschleistung erbringt als alle getesteten Referenzenzyme.

### Beispiel 13

Gefäße mit harten, glatten Oberflächen wurden standardisiert mit in Wasser gequollenen Haferflocken versehen und bei 45°C mit dem Normalprogramm einer Haushaltsgeschirrspülmaschine Typ Miele^{®} G 575 gespült. Pro Spülgang wurden 20 g Spülmittel verwendet; die Wasserhärte betrug 16° deutscher Härte.

Als Spülmittel diente folgende Basis-Rezeptur (alle Angaben jeweils in Gewichts-Prozent): 55 % Natriumtripolyphosphat (berechnet als wasserfrei), 4 % amorphes Natriumdisilikat (berechnet als wasserfrei), 22 % Natriumcarbonat, 9 % Natriumperborat, 2 % TAED, 2 % nichtionisches Tensid, 1,4 % Proteasegranulat, Rest: Wasser, Farbstoffe, Parfüm. Diese Basis-Rezeptur wurde für die verschiedenen Versuche mit verschiedenen Amylasen versetzt, nämlich Termamyl^{®}, Duramyl^{®}, BAN^{®} (Hersteller jeweils: Novozymes A/S, Bagsværd, Dänemark) oder dem erfindungswesentlichen amylolytischen Enzym, der CGTase aus *Bacillus agaradherens* DSM-9948, in wirksamen Mengen von jeweils 20 mg des jeweiligen amylolytischen Enzyms pro Reinigungsgang.

Nach dem Spülen wurde der Abtrag der Verunreinigung in Prozent ermittelt. Die erhaltenen Ergebnisse werden in folgender Tabelle 7 zusammengestellt. Angegeben sind dort die Mittelwerte aus jeweils 8 Messungen. Sie erlauben einen unmittelbaren Rückschluß auf den Beitrag des enthaltenen Enzyms auf die Reinigungsleistung des verwendeten Mittels.

**Tabelle 7:**

| **Basis-Waschmittel mit** | **% Abtrag** |
|---|---|
| | |
| **erfindungswesentl. Enzym** | 86,1 |
| **Termamyl^{®}** | 27,9 |
| **Duramyl^{®}** | 72,2 |
| **BAN^{®}** | 72,1 |
| **Kontrolle ohne Amylase** | 19,3 |

Diese Ergebnisse zeigen, daß die CGTase aus *Bacillus agaradherens* (DSM 9948) hinsichtlich ihres Beitrags zur Reinigungsleistung von maschinellen Geschirrspülmitteln allen anderen getesteten Amylasen überlegen, zumindest aber ebenbürtig ist.

### Beschreibung der Figuren

- **Figur 1:**: Alignment der Aminosäure-Sequenz der erfindungsgemäßen CGTase aus *B. agaradherens* (DSM 9948) mit der von vier bekannten Enzymen; deren Ähnlichkeit zueinander wird in Beispiel 4 angegeben.
Darin bedeuten:
- CDGT_BACAG: Die erfindungsgemäße CGTase aus *B. agaradherens* (DSM 9948)
- CDGT_BACST: Die CGTase aus *Bacillus stearothermophilus* (Q9ZAQ0)
- CDGT_BACOH: Die CGTase aus *Bacillus ohbensis* (P 27036)
- CDGT_BACSP: Die CGTase aus *Bacillus sp.* strain 1011 (P 30921)
- CDGT_THETU: Die CGTase aus *Thermoanaerobacter thermosulfurogenes* (P 26827).
- Consensus: Die sich aus diesem Alignment ergebende Consensus-Sequenz; sie
kann dort angegeben werden, wo mindestens drei der jeweils fünf Aminosäuren identisch sind.

Aminosäuren, die in allen fünf Sequenzen übereinstimmen, sind als schwarze Blöcke hervorgehoben.

Der Übergangsbereich der Domänen C und D ist durch einen Doppelpfeil nahe der Position 530 gekennzeichnet; die Aminosäurefolge VWE (Positionen 524 bis 526 gemäß der SEQ ID NO. 2) wird noch der Domäne C zugerechnet, während die Aminosäurefolge PSI (Positionen 535 bis 537 gemäß der SEQ ID NO.2) bereits der Domäne D zugerechnet wird.

## Patentansprüche

1. Amylolytisches und/oder CGTase-Aktivität aufweisendes Protein mit einer Aminosäuresequenz, die zu der in SEQ ID NO. 2 angegebenen Aminosäuresequenz in den Positionen 1-713 mindestens zu 95 % identisch ist.

2. Amylolytisches und/oder CGTase-Aktivität aufweisendes Protein nach Anspruch 1 mit einer Aminosäuresequenz, die zu der in SEQ ID NO.2 angegebenen Aminosäuresequenz in den Positionen 1-713 mindestens zu 97 % identisch ist.

3. Amylolytisches und/oder CGTase-Aktivität aufweisendes Protein mit einer Aminosäuresequenz, die zu der in SEQ ID NO. 2 angegebenen Aminosäuresequenz in den Positionen 35 bis 713 mindestens zu 95 % identisch ist.

4. Amylolytisches und/oder CGTase-Aktivität aufweisendes Protein nach Anspruch 3 mit einer Aminosäuresequenz, die zu der in SEQ ID NO. 2 angegebenen Aminosäuresequenz in den Positionen 35 bis 713 mindestens zu 97 % identisch ist.

5. Amylolytisches und/oder CGTase-Aktivität aufweisendes Protein mit einer Aminosäuresequenz, die zu der in SEQ ID NO. 2 angegebenen Aminosäuresequenz in den Positionen 35 bis 526 mindestens zu 95 % identisch ist.

6. Amylolytisches und/oder CGTase-Aktivität aufweisendes Protein nach Anspruch 5 mit einer Aminosäuresequenz, die zu der in SEQ ID NO. 2 angegebenen Aminosäuresequenz in den Positionen 35 bis 526 mindestens zu 97 % identisch ist.

7. Amylolytisches und/oder CGTase-Aktivität aufweisendes Protein, das sich von einer Nukleotidsequenz ableitet, die zu der in SEQ ID NO. 1 angegebenen Nukleotidsequenz entsprechend den Aminosäurepositionen 1 - 713 mindestens zu 95 % identisch ist, insbesondere über den Teilbereich, der den Aminosäuren 35 bis 713 gemäß der SEQ ID NO. 2 entspricht.

8. Amylolytisches und/oder CGTase-Aktivität aufweisendes Protein nach Anspruch 7, das sich von einer Nukleotidsequenz ableitet, die zu der in SEQ ID NO. 1 angegebenen Nukleotidsequenz entsprechend den Aminosäurepositionen 1 - 713 mindestens zu 97% identisch ist, insbesondere über den Teilbereich, der den Aminosäuren 35 bis 713 gemäß der SEQ ID NO. 2 entspricht.

9. Amylolytisches und/oder CGTase-Aktivität aufweisendes Protein, das sich von einer Nukleotidsequenz ableitet, die zu der in SEQ ID NO. 1 angegebenen Nukleotidsequenz entsprechend dem Teilbereich der Aminosäuren 35 bis 526 gemäß der SEQ ID NO. 2 mindestens zu 95 % identisch ist.

10. Amylolytisches und/oder CGTase-Aktivität aufweisendes Protein nach Anspruch 9, das sich von einer Nukleotidsequenz ableitet, die zu der in SEQ ID NO. 1 angegebenen Nukleotidsequenz entsprechend dem Teilbereich der Aminosäuren 35 bis 526 gemäß der SEQ ID NO. 2 mindestens zu 97 % identisch ist.

11. Amylolytisches und/oder CGT ase Aktivität aufweisendes Protein nach einem der Ansprüche 1 - 10, das mit der in SEQ ID NO. 2 angegebenen Aminosäuresequenz insgesamt und/oder in den Positionen 35 bis 713 und/oder in den Positionen 35 bis 526 identisch ist und/oder das mit einer von der in SEQ ID NO. 1 angegebenen Nukleotidsequenz abgeleiteten Aminosäuresequenz insgesamt und/oder in den Positionen 35 bis 713 und/oder in den Positionen 35 bis 526 identisch ist.

12. Amylolytisches und/oder CGTase-Aktivität aufweisendes, durch Insertionsmutation erhältliches oder amylolytisches und/oder CGTase-Aktivität aufweisendes chimäres Protein, welches wenigstens in einem die amylolytische Aktivität und/oder die CGTase-Aktivität verleihenden Teil aus einem Protein nach einem der Ansprüche 1 bis 11 besteht.

13. Amylolytisches und/oder CGTase-Aktivität aufweisendes Derivat eines Proteins nach einem der Ansprüche 1 bis 12, erhältlich durch Prozessierung durch den produzierenden Wirtsorganismus, durch die chemische Umwandlung einer Seitenkette einer Aminosäure oder durch kovalente Bindung einer anderen Verbindung an das Protein.

14. Amylolytisches und/oder CGTase-Aktivität aufweisendes Derivat nach Anspruch 13, wobei es sich bei der kovalent gebundenen anderen Verbindung um Polyethylenglycol oder um ein anderes Protein handelt.

15. Amylolytisches und/oder CGTase-Aktivität aufweisendes Protein oder Derivat nach einem der Ansprüche 1 bis 14, **dadurch gekennzeichnet, daß** es aus einer natürlichen Quelle, insbesondere aus einem Mikroorganismus erhältlich ist.

16. Amylolytisches und/oder CGTase-Aktivität aufweisendes Protein oder Derivat nach Anspruch 15, **dadurch gekennzeichnet, daß** es sich bei dem Mikroorganismus um ein gram-positives Bakterium handelt.

17. Amylolytisches und/oder CGTase-Aktivität aufweisendes Protein oder Derivat nach Anspruch 16, **dadurch gekennzeichnet, daß** es sich bei dem gram-positiven Bakterium um eines der Gattung Bacillus handelt.

18. Amylolytisches und/oder CGTase-Aktivität aufweisendes Protein oder Derivat nach Anspruch 17, **dadurch gekennzeichnet, daß** es sich bei der Bacillus-Spezies um einen alkalophilen Bacillus, insbesondere um *Bacillus agaradherens,* ganz besonders um *Bacillus agaradherens* (DSM 9948) handelt.

19. Für ein amylolytisches und/oder CGTase-Aktivität aufweisendes Protein codierende Nukleinsäure, deren Nukleotidsequenz zu der in SEQ ID NO. 1 angegebenen Nukleotidsequenz mindestens zu 95 % identisch ist, insbesondere über den Teilbereich, der den Aminosäuren 35 bis 713 gemäß der SEQ ID NO. 2 entspricht, ganz besonders über den Teilbereich, der den Aminosäuren 35 bis 526 gemäß der SEQ ID NO. 2 entspricht.

20. Für ein amylolytisches und/oder CGTase-Aktivität aufweisendes Protein codierende Nukleinsäure nach Anspruch 19, deren Nukleotidsequenz zu der in SEQ ID NO. 1 angegebenen Nukleotidsequenz mindestens zu 97 % identisch ist, insbesondere über den Teilbereich, der den Aminosäuren 35 bis 713 gemäß der SEQ ID NO. 2 entspricht, ganz besonders über den Teilbereich, der den Aminosäuren 35 bis 526 gemäß der SEQ ID NO. 2 entspricht.

21. Für ein amylolytisches und/oder CGTase-Aktivität aufweisendes Protein codierende Nukleinsäure nach Anspruch 19 oder 20, die zu der in SEQ ID NO. 1 angegebenen Nukleotidsequenz zu 100 % identisch ist, insbesondere über den Teilbereich, der den Aminosäuren 35 bis 713 gemäß der SEQ ID NO. 2 entspricht, ganz besonders über den Teilbereich, der den Aminosäuren 35 bis 526 gemäß der SEQ ID NO. 2 entspricht.

22. Nukleinsäure, die für eines der Proteine oder Derivate nach einem der Ansprüche 1 bis 18 codiert.

23. Mikroorganismus, der eines der Proteine oder Derivate nach einem der Ansprüche 1 bis 18 natürlicherweise bildet oder eine für solch ein Protein oder Derivat codierende Nukleinsäure enthält.

24. Mikroorganismus nach Anspruch 23, **dadurch gekennzeichnet, daß** er ein Bakterium ist.

25. Mikroorganismus nach Anspruch 24, **dadurch gekennzeichnet, daß** es sich bei dem Bakterium um ein gram-positives Bakterium handelt.

26. Mikroorganismus nach Anspruch 25, **dadurch gekennzeichnet, daß** es sich bei dem gram-positiven Bakterium um eines der Gattung Bacillus, vorzugsweise um einen alkaliphilen Bacillus handelt.

27. Mikroorganismus nach Anspruch 26, **dadurch gekennzeichnet, daß** es sich bei der Bacillus-Spezies um *Bacillus agaradherens,* insbesondere um *Bacillus agaradherens* (DSM 9948) handelt.

28. Vektor, der einen Nukleinsäurebereich nach einem der Ansprüche 19 bis 22 enthält.

29. Vektor nach Anspruch 28, wobei es sich um einen Klonierungsvektor handelt.

30. Vektor nach Anspruch 28, wobei es sich um einen Expressionsvektor handelt.

31. Isolierte Wintszelle die eine der Nukleinsäuren nach einem der Ansprüche 19 bis 22 enthält, vorzugsweise auf einem Vektor nach einem der Ansprüche 28 bis 30.

32. Zelle nach Anspruch 31, die eines der Proteine oder Derivate nach einem der Ansprüche 1 bis 18 exprimiert oder zu dessen Expression angeregt werden kann, insbesondere unter Einsatz eines Expressionsvektors nach Anspruch 30.

33. Zelle nach einem der Ansprüche 31 oder 32, **dadurch gekennzeichnet, daß** sie ein Bakterium ist, insbesondere eines, das das gebildete Protein oder Derivat ins umgebende Medium sekretiert.

34. Zelle nach Anspruch 33, **dadurch gekennzeichnet, daß** sie ein Bakterium der Gattung Bacillus, insbesondere der Species *Bacillus licheniformis, Bacillus amyloliquefaciens, Bacillus subtilis* oder *Bacillus alcalophilus* ist.

35. Zelle nach einem der Ansprüche 31 bis 33, **dadurch gekennzeichnet, daß** sie ein gram-negatives Bakterium ist.

36. Zelle nach Anspruch 35, **dadurch gekennzeichnet, daß** sie der Gattung Escherichia, bevorzugt der Spezies *Escherichia coli* oder *Klebsiella,* besonders bevorzugt einem der Stämme *E. coli* JM 109, *E. coli* DH 100B, *E. coli* DH 12S oder *Klebsiella planticola* (Rf) angehört.

37. Zelle nach Anspruch 31 oder 32, **dadurch gekennzeichnet, daß** sie eine eukaryontische Zelle ist, besonders eine, die das Protein exprimiert, ganz besonders eine, die das Protein posttranslational modifiziert.

38. Verfahren zur Herstellung eines Proteins oder Derivats nach einem der Ansprüche 1 bis 18 unter Verwendung einer Nukleinsäure nach einem der Ansprüche 19 bis 22 und/oder unter Verwendung eines Organismus nach einem der Ansprüche 23 bis 27 und/oder unter Verwendung eines Vektors nach einem der Ansprüche 28 bis 30 und/oder unter Verwendung einer Zelle nach einem der Ansprüche 31 bis 37.

39. Wasch- oder Reinigungsmittel, **dadurch gekennzeichnet, daß** es ein Protein oder Derivat nach einem der Ansprüche 1 bis 18 enthält.

40. Mittel nach Anspruch 39, **dadurch gekennzeichnet, daß** es 0,0001 Gewichts-Prozent bis 5 Gew.-%, insbesondere 0,001 bis 3 Gew.-% des amylolytischen und/oder CGTase-Aktivität aufweisenden Proteins oder Derivats nach einem der Ansprüche 1-18 enthält.

41. Mittel nach Anspruch 39 oder 40, **dadurch gekennzeichnet, daß** es zusätzlich ein oder mehrere andere amylolytische Proteine, insbesondere α-Amylasen enthält.

42. Mittel nach einem der Ansprüche 39 bis 41, **dadurch gekennzeichnet, daß** es zusätzlich andere Enzyme, insbesondere eine oder mehrere Proteasen, Lipasen, Oxidoreduktasen, Hemicellulasen und/oder Cellulasen enthält.

43. Mittel nach einem der Ansprüche 39 bis 42, **dadurch gekennzeichnet, daß** es Cyclodextrine enthält, insbesondere solche, in die niedermolekulare Verbindungen eingelagert sind.

44. Mittel nach einem der Ansprüche 39 bis 43, **dadurch gekennzeichnet, daß** es aus mehr als einer Phase besteht.

45. Mittel nach einem der Ansprüche 39 bis 44, **dadurch gekennzeichnet, daß** es fest ist und mindestens zwei verschiedene feste Komponenten, insbesondere Pulver, Granulate oder Extrudate, in insgesamt loser Form miteinander vermischt vorliegen.

46. Mittel nach einem der Ansprüche 39 bis 45, **dadurch gekennzeichnet, daß** es kompaktiert ist.

47. Mittel nach einem der Ansprüche 44 bis 46, **dadurch gekennzeichnet, daß** wenigstens eine der Phasen ein Amylase-sensitives Material, insbesondere Stärke enthält oder von diesem zumindest teilweise umgeben oder beschichtet ist.

48. Mittel nach einem der Ansprüche 39 bis 47, **dadurch gekennzeichnet, daß** es flüssig, gelförmig oder pastös ist und das enthaltene Protein und/oder wenigstens eines der enthaltenen Enzyme und/oder wenigstens eine der sonstigen enthaltenen Komponenten einzeln oder zusammen mit anderen Bestandteilen verkapselt vorliegt, bevorzugt in Mikrokapseln, besonders bevorzugt in solchen aus einem Amylase-sensitiven Material.

49. Mittel nach einem der Ansprüche 39 bis 48, **dadurch gekennzeichnet, daß** die amylolytische und/oder CGTase-Aktivität durch einen der sonstigen Bestandteile des Mittels modifiziert, insbesondere stabilisiert und/oder in ihrem Beitrag zur Wasch-, beziehungsweise Reinigungsleistung des Mittels gesteigert wird.

50. Verfahren zur Reinigung von Textilien oder von harten Oberflächen, **dadurch gekennzeichnet, daß** in wenigstens einem der Verfahrensschritte ein amylolytisches und/oder CGTase-Aktivität aufweisendes Protein oder Derivat nach einem der Ansprüche 1 bis 18 aktiv wird.

51. Verfahren zur Reinigung von Textilien oder von harten Oberflächen, **dadurch gekennzeichnet, daß** in wenigstens einem der Verfahrensschritte ein Mittel nach einem der Ansprüche 39 bis 49 eingesetzt wird.

52. Verfahren nach Anspruch 50 oder 51, **dadurch gekennzeichnet, daß** das amylolytische und/oder CGTase-Aktivität aufweisende Protein oder Derivat in dem betreffenden Verfahrensschritt in einer Menge von 0,035 mg bis 2 000 mg, vorzugsweise von 0,3 mg bis 1 500 mg eingesetzt wird.

53. Verwendung eines amylolytischen und/oder CGTase-Aktivität aufweisenden Proteins oder Derivats nach einem der Ansprüche 1 bis 18 allein oder zusammen mit mindestens einem anderen reinigungsaktiven oder die Reinigungswirkung unterstützenden Wirkstoff zur Reinigung von Textilien oder von harten Oberflächen.

54. Verwendung eines Mittels nach einem der Ansprüche 39 bis 49 zur Reinigung von Textilien oder von harten Oberflächen.

55. Verwendung nach Anspruch 53 oder 54, **dadurch gekennzeichnet, daß** pro Anwendung, vorzugsweise pro Anwendung in einer Geschirrspülmaschine oder einer Waschmaschine, 0,035 mg bis 2 000 mg, bevorzugt 0,3 mg bis 1 500 mg des amylolytischen und/oder CGTase-Aktivität aufweisenden Proteins oder Derivats eingesetzt werden.

56. Verwendung eines amylolytischen und/oder CGTase-Aktivität aufweisenden Proteins oder Derivats nach einem der Ansprüche 1 bis 18 allein oder zusammen mit mindestens einem anderen reinigungsaktiven oder die Reinigungswirkung unterstützenden Wirkstoff in einem aus mehr als einer Phase bestehenden Wasch- oder Reinigungsmittel zur Aktivierung der eigenen oder anderer Phasen.

57. Verwendung eines amylolytischen und/oder CGTase-Aktivität aufweisenden Proteins oder Derivats nach einem der Ansprüche 1 bis 18 allein oder zusammen mit mindestens einem anderen reinigungsaktiven oder die Reinigungswirkung unterstützenden Wirkstoff in einem Wasch- oder Reinigungsmittel mit verkapselten Inhaltsstoffen zur Freisetzung der Inhaltsstoffe aus den Kapseln.

58. Verwendung eines Proteins oder Derivats nach einem der Ansprüche 1 bis 18 zur Behandlung von Rohmaterialien oder Zwischenprodukten in der Textilherstellung, insbesondere zum Entschlichten von Baumwolle oder zum Entfernen von Stärke- oder Stärke-ähnlichen Schutzschichten von technischen Zwischenprodukten.

59. Verfahren zur Stärkeverflüssigung, insbesondere zur Ethanolproduktion, **dadurch gekennzeichnet, daß** darin in wenigstens einem Verfahrensschritt ein Protein oder Derivat gemäß nach der Ansprüche 1 bis 18 eingesetzt wird.

60. Verwendung eines Proteins oder Derivats nach einem der Ansprüche 1 bis 18 zur Stärkeverflüssigung, insbesondere in einem Verfahren zur Ethanolproduktion.

61. Verwendung eines Proteins oder Derivats nach einem der Ansprüche 1 bis 18 zur Herstellung oder Modifizierung von linearen und/oder kurzkettigen Oligosacchariden, insbesondere eines solchen Proteins oder Derivats, dessen CGTase-Aktivität durch Deletion eingeschränkt worden ist.

62. Verwendung eines Proteins oder Derivats nach einem der Ansprüche 1 bis 18 zur Herstellung, Modifizierung oder Hydrolyse von Cyclodextrinen.

63. Verwendung eines Proteins oder Derivats nach einem der Ansprüche 1 bis 18 zur Aufnahme oder Freisetzung von nieder- oder höhermolekularen Verbindungen in, beziehungsweise aus Polysaccharidträgern, insbesondere Cyclodextrinen.

64. Verwendung nach Anspruch 63 zur Stabilisierung von chemischen Verbindungen während ihrer Herstellung oder Verarbeitung.

65. Verwendung nach Anspruch 63 oder 64 zur Herstellung oder als Bestandteil von Kosmetika oder Pharmaka.

## Claims

1. Protein having amylolytic and/or CGTase activity and an amino acid sequence which is at least 95% identical with the amino acid sequence indicated in SEQ ID No. 2 in positions 1-713.

2. Protein having amylolytic and/or CGTase activity according to Claim 1, having an amino acid sequence which is at least 97% identical with the amino acid sequence indicated in SEQ ID No. 2 in positions 1-713.

3. Protein having amylolytic and/or CGTase activity and an amino acid sequence which is at least 95% identical with the amino acid sequence indicated in SEQ ID No. 2 in positions 35 to 713.

4. Protein having amylolytic and/or CGTase activity according to Claim 3, having an amino acid sequence which is at least 97% identical with the amino acid sequence indicated in SEQ ID No. 2 in positions 35 to 713.

5. Protein having amylolytic and/or CGTase activity and an amino acid sequence which is at least 95% identical with the amino acid sequence indicated in SEQ ID No. 2 in positions 35 to 526.

6. Protein having amylolytic and/or CGTase activity according to Claim 5, having an amino acid sequence which is at least 97% identical with the amino acid sequence indicated in SEQ ID No. 2 in positions 35 to 526.

7. Protein having amylolytic and/or CGTase activity, which is derived from a nucleotide sequence which is at least 95% identical to the nucleotide sequence indicated in SEQ ID No. 1 corresponding to amino acid positions 1-713, in particular across the partial region corresponding to the amino acids 35 to 713 according to SEQ ID No. 2.

8. Protein having amylolytic and/or CGTase activity according to Claim 7, which is derived from a nucleotide sequence which is at least 97% identical to the nucleotide sequence indicated in SEQ ID No. 1 corresponding to amino acid positions 1-713, in particular across the partial region corresponding to the amino acids 35 to 713 according to SEQ ID No. 2.

9. Protein having amylolytic and/or CGTase activity, which is derived from a nucleotide sequence which is at least 95% identical to the nucleotide sequence indicated in SEQ ID No. 1 corresponding to the partial region corresponding to the amino acids 35 to 526 according to SEQ ID No. 2.

10. Protein having amylolytic and/or CGTase activity according to Claim 9, which is derived from a nucleotide sequence which is at least 97% identical to the nucleotide sequence indicated in SEQ ID No. 1 corresponding to the partial region corresponding to the amino acids 35 to 526 according to SEQ ID No. 2.

11. Protein having amylolytic and/or CGTase activity according to any of Claims 1-10, which is identical with the amino acid sequence indicated in SEQ ID No. 2 over the entire length and/or in positions 35 to 713 and/or in positions 35 to 526 and/or which is identical with an amino acid sequence derived from the nucleotide sequence indicated in SEQ ID No. 1 over the entire length and/or in positions 35 to 713 and/or in positions 35 to 526.

12. Protein having amylolytic and/or CGTase activity and obtainable by insertion mutation or a chimeric protein having amylolytic and/or CGTase activity which consists at least in one part imparting said amylolytic activity and/or said CGTase activity of a protein according to any of Claims 1 to 11.

13. Derivative, having amylolytic and/or CGTase activity, of a protein according to any of Claims 1 to 12, obtainable by processing by the producing host organism, by chemical conversion of an amino acid side chain or by covalent binding of another compound to the protein.

14. Derivative according to Claim 13, having amylolytic and/or CGTase activity, in which the covalently bound other compound is polyethylene glycol or another protein.

15. Protein or derivative having amylolytic and/or CGTase activity according to any of Claims 1 to 14, **characterized in that** it is obtainable from a natural source, in particular from a microorganism.

16. Protein or derivative having amylolytic and/or CGTase activity according to Claim 15, **characterized in that** the microorganism is a Gram-positive bacterium.

17. Protein or derivative having amylolytic and/or CGTase activity according to Claim 16, **characterized in that** the Gram-positive bacterium is any of the genus Bacillus.

18. Protein or derivative having amylolytic and/or CGTase activity according to Claim 17, **characterized in that** the bacillus species is an alkalophilic bacillus, in particular *Bacillus agaradherens,* especially *Bacillus agaradherens* (DSM 9948).

19. Nucleic acid coding for a protein having amylolytic and/or CGTase activity, whose nucleotide sequence is at least 95% identical to the nucleotide sequence indicated in SEQ ID No. 1, in particular across the partial region corresponding to the amino acids 35 to 713 according to SEQ ID No. 2, especially across the partial region corresponding to the amino acids 35 to 526 according to SEQ ID No. 2.

20. Nucleic acid coding for a protein having amylolytic and/or CGTase activity according to Claim 19, whose nucleotide sequence is at least 97% identical to the nucleotide sequence indicated in SEQ ID No. 1, in particular across the partial region corresponding to the amino acids 35 to 713 according to SEQ ID No. 2, especially across the partial region corresponding to the amino acids 35 to 526 according to SEQ ID No. 2.

21. Nucleic acid coding for a protein having amylolytic and/or CGTase activity according to Claim 19 or 20, which is 100% identical with the nucleotide sequence indicated in SEQ ID No. 1, in particular across the partial region corresponding to the amino acids 35 to 713 according to SEQ ID No. 2, especially across the partial region corresponding to the amino acids 35 to 526 according to SEQ ID No. 2.

22. Nucleic acid coding for any of the proteins or derivatives according to any of Claims 1 to 18.

23. Microorganism which produces naturally any of the proteins or derivatives according to any of Claims 1 to 18 or which contains a nucleic acid coding for such a protein or derivative.

24. Microorganism according to Claim 23, **characterized in that** it is a bacterium.

25. Microorganism according to Claim 24, **characterized in that** the bacterium is a Gram-positive bacterium.

26. Microorganism according to Claim 25, **characterized in that** the Gram-positive bacterium is any of the genus Bacillus, preferably an alkaliphilic bacillus.

27. Microorganism according to Claim 26, **characterized in that** the bacillus species is *Bacillus agaradherens,* in particular *Bacillus agaradherens* (DSM 9948).

28. Vector which contains a nucleic acid region according to any of Claims 19 to 22.

29. Vector according to Claim 28, which is a cloning vector.

30. Vector according to Claim 28, which is an expression vector.

31. Isolated host cell which contains any of the nucleic acids according to any of Claims 19 to 22, preferably on a vector according to any of Claims 28 to 30.

32. Cell according to Claim 31, which expresses or can be induced to express any of the proteins or derivatives according to any of Claims 1 to 18, in particular by using an expression vector according to Claim 30.

33. Cell according to either of Claims 31 and 32, **characterized in that** it is a bacterium, in particular one secreting the protein or derivative produced into the surrounding medium.

34. Cell according to Claim 33, **characterized in that** it is a bacterium of the genus Bacillus, in particular of the species *Bacillus licheniformis, Bacillus amyloliquefaciens, Bacillus subtilis* or *Bacillus alcalophilus.*

35. Cell according to any of Claims 31 to 33, **characterized in that** it is a Gram-negative bacterium.

36. Cell according to Claim 35, **characterized in that** it belongs to the genus Escherichia, preferably to the species *Escherichia coli* or *Klebsiella,* particularly preferably to any of the strains *E. coli* JM 109, *E. coli* DH 100B, *E. coli* DH 12S or *Klebsiella planticola* (Rf).

37. Cell according to Claim 31 or 32, **characterized in that** it is a eukaryotic cell, in particular one expressing the protein, especially one modifying said protein posttranslationally.

38. Method for preparing a protein or derivative according to any of Claims 1 to 18 by using a nucleic acid according to any of Claims 19 to 22 and/or by using an organism according to any of Claims 23 to 27 and/or by using a vector according to any of Claims 28 to 30 and/or by using a cell according to any of Claims 31 to 37.

39. Detergent or cleaning agent, **characterized in that** it contains a protein or derivative according to any of Claims 1 to 18.

40. Agent according to Claim 39, **characterized in that** it contains from 0.0001 percent by weight to 5% by weight, in particular from 0.001 to 3% by weight, of the protein or derivative having amylolytic and/or CGTase activity according to any of Claims 1-18.

41. Agent according to Claim 39 or 40, **characterized in that** it contains additionally one or more other amylolytic proteins, in particular α-amylases.

42. Agent according to any of Claims 39 to 41, **characterized in that** it contains additionally other enzymes, in particular one or more proteases, lipases, oxidoreductases, hemicellulases and/or cellulases.

43. Agent according to any of Claims 39 to 42, **characterized in that** it contains cyclodextrins, in particular those into which low-molecular weight compounds have been incorporated.

44. Agent according to any of Claims 39 to 43, **characterized in that** it comprises more than one phase.

45. Agent according to any of Claims 39 to 44, **characterized in that** it is solid and that at least two different solid components, in particular powders, granules or extrudates, are present in a by and large loose mixture.

46. Agent according to any of Claims 39 to 45, **characterized in that** it is compacted.

47. Agent according to any of Claims 44 to 46, **characterized in that** at least one of the phases contains an amylase-sensitive material, in particular starch, or is, at least partially, surrounded by or coated with said material.

48. Agent according to any of Claims 39 to 47, **characterized in that** it is in liquid, gel or paste form and that the protein contained and/or at least one of the enzymes contained and/or at least one of the other components contained are present, individually or together with other components, in encapsulated form, preferably in microcapsules, particularly preferably in those made of an amylase-sensitive material.

49. Agent according to any of Claims 39 to 48, **characterized in that** any of the other components of said agent modifies, in particular stabilizes the amylolytic and/or CGTase activity and/or increases the contribution thereof to the washing or cleaning performance of said agent.

50. Method for cleaning textiles or hard surfaces, **characterized in that** in at least one of the method steps a protein or derivative having amylolytic and/or CGTase activity according to any of Claims 1 to 18 becomes active.

51. Method for cleaning textiles or hard surfaces, **characterized in that** an agent according to any of Claims 39 to 49 is used in at least one of the method steps.

52. Method according to Claim 50 or 51, **characterized in that** the protein or derivative having amylolytic and/or CGTase activity is used in the method step in question in an amount of from 0.035 mg to 2 000 mg, preferably from 0.3 mg to 1 500 mg.

53. Use of a protein or derivative having amylolytic and/or CGTase activity according to any of Claims 1 to 18 alone or together with at least one other cleaning-active ingredient or active ingredient supporting the cleaning action for cleaning textiles or hard surfaces.

54. Use of an agent according to any of Claims 39 to 49 for cleaning textiles or hard surfaces.

55. Use according to Claim 53 or 54, **characterized in that** per application, preferably per application in a dishwasher or a washing machine, 0.035 mg to 2 000 mg, preferably 0.3 mg to 1 500 mg, of the protein or derivative having amylolytic and/or CGTase activity are used.

56. Use of a protein or derivative having amylolytic and/or CGTase activity according to any of Claims 1 to 18 alone or together with at least one other cleaning-active active ingredient or active ingredient supporting the cleaning action in a detergent or cleaning agent comprising more than one phase for activating its own or other phases.

57. Use of a protein or derivative having amylolytic and/or CGTase activity according to any of Claims 1 to 18 alone or together with at least one other cleaning-active active ingredient or active ingredient supporting the cleaning action in a detergent or cleaning agent containing encapsulated ingredients for releasing said ingredients from the capsules.

58. Use of a protein or derivative according to any of Claims 1 to 18 for the treatment of raw materials or intermediates in the manufacture of textiles, in particular for desizing cotton or for removing starch or starch-like protective layers from industrial intermediates.

59. A process for starch liquefaction, in particular for producing ethanol, **characterized in that** Protein or derivative according to Claims 1 to 18 is used in at least one process step.

60. Use of a protein or derivative according to any of Claims 1 to 18 for starch liquefaction, in particular in a process for producing ethanol.

61. Use of a protein or derivative according to any of Claims 1 to 18 for preparing or modifying linear and/or short-chain oligosaccharides, in particular of such Protein or derivative whose CGTase activity has been restricted by deletion.

62. Use of a protein or derivative according to any of Claims 1 to 18 for preparing, modifying or hydrolyzing cyclodextrins.

63. Use of a protein or derivative according to any of Claims 1 to 18 for absorbing or releasing low-molecular or high-molecular weight compounds into or from polysaccharide supports, in particular cyclodextrins.

64. Use according to Claim 63 for stabilizing chemical compounds during their preparation or processing.

65. Use according to Claim 63 or 64 for the production or as component of cosmetics or pharmaceuticals.

## Revendications

1. Protéine amylolytique et/ou présentant une activité de CGTase comprenant une séquence d'acides aminés qui manifeste une identité à concurrence d'au moins 95 % par rapport à la séquence d'acides aminés indiquée dans SEQ ID NO: 2 aux positions 1-713.

2. Protéine amylolytique et/ou présentant une activité de CGTase selon la revendication 1, comprenant une séquence d'acides aminés qui manifeste une identité à concurrence d'au moins 97 % par rapport à la séquence d'acides aminés indiquée dans SEQ ID NO: 2 aux positions 1 - 713.

3. Protéine amylolytique et/ou présentant une activité de CGTase comprenant une séquence d'acides aminés qui manifeste une identité à concurrence d'au moins 95 % par rapport à la séquence d'acides aminés indiquée dans SEQ ID NO: 2 aux positions 35 à 713

4. Protéine amylolytique et/ou présentant une activité de CGTase selon la revendication 3, comprenant une séquence d'acides aminés qui manifeste une identité à concurrence d'au moins 97 % par rapport à la séquence d'acides aminés indiquée dans SEQ ID NO: 2 aux positions 35 à 713

5. Protéine amylolytique et/ou présentant une activité de CGTase comprenant une séquence d'acides aminés qui manifeste une identité à concurrence d'au moins 95 % par rapport à la séquence d'acides aminés indiquée dans SEQ ID NO: 2 aux positions 35 à 526.

6. Protéine amylolytique et/ou présentant une activité de CGTase selon la revendication 5, comprenant une séquence d'acides aminés qui manifeste une identité à concurrence d'au moins 97 % par rapport à la séquence d'acides aminés indiquée dans SEQ ID NO: 2 aux positions 35 à 526.

7. Protéine amylolytique et/ou présentant une activité de CGTase qui dérive d'une séquence nucléotidique qui manifeste une identité à concurrence d'au moins 95 % par rapport à la séquence nucléotidique indiquée dans SEQ ID NO: 1 correspondant aux positions d'acides aminés 1 - 713, en particulier dans la région partielle qui correspond aux acides aminés 35 à 713 selon SEQ ID NO: 2.

8. Protéine amylolytique et/ou présentant une activité de CGTase selon la revendication 7, qui dérive d'une séquence nucléotidique qui manifeste une identité à concurrence d'au moins 97 % par rapport à la séquence nucléotidique indiquée dans SEQ ID NO: 1 correspondant aux positions d'acides aminés 1 - 713, en particulier dans la région partielle qui correspond aux acides aminés 35 à 713 selon SEQ ID NO: 2.

9. Protéine amylolytique et/ou présentant une activité de CGTase qui dérive d'une séquence nucléotidique qui manifeste une identité à concurrence d'au moins 95 % par rapport à la séquence nucléotidique indiquée dans SEQ ID NO: 1 correspondant à la région partielle des acides aminés 35 à 526 selon SEQ ID NO: 2.

10. Protéine amylolytique et/ou présentant une activité de CGTase selon la revendication 9, qui dérive d'une séquence nucléotidique qui manifeste une identité à concurrence d'au moins 97 % par rapport à la séquence nucléotidique indiquée dans SEQ ID NO: 1 correspondant à la région partielle des acides aminés 35 à 526 selon SEQ ID NO: 2.

11. Protéine amylolytique et/ou présentant une activité de CGTase selon l'une quelconque des revendications 1 à 10, qui manifeste une identité avec la séquence d'acides aminés indiquée dans SEQ ID NO: 2 en totalité et/ou aux positions 35 à 713 et/ou aux positions 35 à 526 et/ou qui manifeste une identité avec une séquence d'acides aminés qui dérive de la séquence nucléotidique indiquée dans SEQ ID NO: 1 en totalité et/ou aux positions 35 à 713 et/ou aux positions 35 à 526.

12. Protéine amylolytique et/ou présentant une activité de CGTase, protéine que l'on obtient par mutation insertionnelle ou protéine chimère amylolytique et/ou présentant une activité de CGTase, qui est constituée, au moins dans une partie conférant l'activité amylolytique et/ou l'activité de CGTase, d'une protéine selon l'une quelconque des revendications 1 à 11.

13. Dérivé amylolytique et/ou présentant une activité de CGTase d'une protéine selon l'une quelconque des revendications 1 à 12, que l'on obtient par traitement via l'organisme hôte producteur, par la transformation chimique d'une chaîne latérale d'un d'acide aminé ou par liaison covalente d'un autre composé à la protéine.

14. Dérivé amylolytique et/ou présentant une activité de CGTase selon la revendication 13, dans lequel, en ce qui concerne l'autre composé lié de manière covalente, il s'agit du polyéthylèneglycol ou d'une autre protéine.

15. Protéine ou dérivé amylolytique et/ou présentant une activité de CGTase selon l'une quelconque des revendications 1 à 14, **caractérisé en ce qu'**on l'obtient à partir d'une source naturelle, en particulier à partir d'un micro-organisme.

16. Protéine ou dérivé amylolytique et/ou présentant une activité de CGTase selon la revendication 15, **caractérisé en ce que**, en ce qui concerne le micro-organisme, il s'agit d'une bactérie gram-positif.

17. Protéine ou dérivé amylolytique et/ou présentant une activité de CGTase selon la revendication 16, **caractérisé en ce que**, en ce qui concerne la bactérie gram-positif, il s'agit du genre Bacillus.

18. Protéine ou dérivé amylolytique et/ou présentant une activité de CGTase selon la revendication 17, **caractérisé en ce que**, en ce qui concerne l'espèce Bacillus, il s'agit d'un Bacillus alcalophile, en particulier de *Bacillus agaradherens,* de manière tout à fait particulière de *Bacillus agaradherens* (DSM 9948).

19. Acide nucléique codant pour une protéine amylolytique et/ou présentant une activité de CGTase, dont la séquence nucléotidique manifeste une identité à concurrence d'au moins 95 % par rapport à la séquence nucléotidique indiquée dans SEQ ID NO: 1, en particulier dans la région partielle qui correspond aux acides aminés 35 à 713 selon SEQ ID NO: 2, de manière tout à fait particulière dans la région partielle qui correspond aux acides aminés 35 à 526 selon SEQ ID NO: 2.

20. Acide nucléique codant pour une protéine amylolytique et/ou présentant une activité de CGTase selon la revendication 19, dont la séquence nucléotidique manifeste une identité à concurrence d'au moins 97 % par rapport à la séquence nucléotidique indiquée dans SEQ ID NO: 1, en particulier dans la région partielle qui correspond aux acides aminés 35 à 713 selon SEQ ID NO: 2, de manière tout à fait particulière dans la région partielle qui correspond aux acides aminés 35 à 526 selon SEQ ID NO: 2.

21. Acide nucléique codant pour une protéine amylolytique et/ou présentant une activité de CGTase selon la revendication 19 ou 20, dont la séquence nucléotidique manifeste une identité à concurrence de 100 % par rapport à la séquence nucléotidique indiquée dans SEQ ID NO: 1, en particulier dans la région partielle qui correspond aux acides aminés 35 à 713 selon SEQ ID NO: 2, de manière tout à fait particulière dans la région partielle qui correspond aux acides aminés 35 à 526 selon SEQ ID NO: 2.

22. Acide nucléique qui code pour une des protéines ou un des dérivés selon l'une quelconque des revendications 1 à 18.

23. Micro-organisme qui forme naturellement une des protéines ou un des dérivés selon l'une quelconque des revendications 1 à 18 ou qui contient un acide nucléique codant pour une telle protéine ou pour un tel dérivé.

24. Micro-organisme selon la revendication 23, **caractérisé en ce qu'**il représente une bactérie.

25. Micro-organisme selon la revendication 24, **caractérisé en ce qu'**il s'agit, en ce qui concerne la bactérie, d'une bactérie gram-positif.

26. Micro-organisme selon la revendication 25, **caractérisé en ce que**, en ce qui concerne la bactérie.gram-positif, il s'agit d'une bactérie du genre Bacillus, de préférence d'un Bacillus alcalophile.

27. Micro-organisme selon la revendication 26, **caractérisé en ce que**, en ce qui concerne l'espèce Bacillus il s'agit de *Bacillus agaradherens,* en particulier de *Bacillus agaradherens* (DSM 9948).

28. Vecteur qui contient une région d'acides nucléiques selon l'une quelconque des revendications 19 à 22.

29. Vecteur selon la revendication 28, à propos duquel il s'agit d'un vecteur de clonage.

30. Vecteur selon la revendication 28, à propos duquel il s'agit d'un vecteur d'expression.

31. Cellule hôte isolée qui contient un des acides nucléiques selon l'une quelconque des revendications 19 à 22, de préférence sur un vecteur selon l'une quelconque des revendications 28 à 30.

32. Cellule selon la revendication 31, qui exprime une des protéines ou un des dérivés selon l'une quelconque des revendications 1 à 18 ou dont l'expression en question peut être activée, en particulier par insertion d'un vecteur d'expression selon la revendication 30.

33. Cellule selon l'une quelconque des revendications 31 ou 32, **caractérisée en ce qu'**il s'agit d'une bactérie, en particulier d'une bactérie qui sécrète la protéine ou le dérivé obtenu dans le milieu environnant.

34. Cellule selon la revendication 33, **caractérisée en ce qu'**il s'agit d'une bactérie du genre Bacillus, en particulier de l'espèce *Bacillus licheniformis, Bacillus amyloliquefaciens, Bacillus subtilis* ou *Bacillus alcalophilus.*

35. Cellule selon l'une quelconque des revendications 31 à 33, **caractérisée en ce qu'**il s'agit d'une bactérie gram-négatif.

36. Cellule selon la revendication 35, **caractérisée en ce qu'**elle fait partie du genre Escherichia, de préférence de l'espèce *Escherichia coli* ou *Klebsiella,* de manière particulièrement préférée d'une des souches *E. coli* JM 109, *E. coli* DH 100B, *E. coli* DH 12S ou *Klebsiella planticola* (Rf).

37. Cellule selon la revendication 31 ou 32, **caractérisée en ce qu'**il s'agit d'une cellule eucaryote, en particulier d'une cellule eucaryote qui exprime la protéine, de manière tout particulièrement préférée d'une cellule eucaryote qui soumet la protéine à une modification posttraductionnelle.

38. Procédé pour la préparation d'une protéine ou d'un dérivé selon l'une quelconque des revendications 1 à 18, en utilisant un acide nucléique selon l'une quelconque des revendications 19 à 22 et/ou en utilisant un organisme selon l'une quelconque des revendications 23 à 27 et/ou en utilisant un vecteur selon l'une quelconque des revendications 28 à 30 et/ou en utilisant une cellule selon l'une quelconque des revendications 31 à 37.

39. Agent de lavage ou de nettoyage, **caractérisé en ce qu'**il contient une protéine ou un dérivé selon l'une quelconque des revendications 1 à 18.

40. Agent selon la revendication 39, **caractérisé en ce qu'**il contient la protéine et/ou le dérivé amylolytique et/ou présentant une activité de CGTase selon l'une quelconque des revendications 1 à 18, à concurrence de 0,0001 % en poids à 5 % en poids, en particulier de 0,001 à 3 % en poids.

41. Agent selon la revendication 39 ou 40, **caractérisé en ce qu'**il contient en outre une ou plusieurs autres protéines amylolytiques, en particulier des α-amylases.

42. Agent selon l'une quelconque des revendications 39 à 41, **caractérisé en ce qu'**il contient en outre d'autres enzymes, en particulier une ou plusieurs protéases, lipases, oxydoréductases, hémicellulases et/ou cellulases.

43. Agent selon l'une quelconque des revendications 39 à 42, **caractérisé en ce qu'**il contient des cyclodextrines, en particulier celles dans lesquelles sont incorporés des composés à bas poids moléculaire.

44. Agent selon l'une quelconque des revendications 39 à 43, **caractérisé en ce qu'**il est constitué de plus d'une phase.

45. Agent selon l'une quelconque des revendications 39 à 44, **caractérisé en ce qu'**il est solide et **en ce qu'**au moins deux composants solides différents, en particulier des poudres, des produits de granulation ou des extrudats sont présents en mélange réciproque, globalement en vrac.

46. Agent selon l'une quelconque des revendications 39 à 45, **caractérisé en ce qu'**il est comprimé.

47. Agent selon l'une quelconque des revendications 44 à 46, **caractérisé en ce qu'**au moins une des phases contient une matière sensible aux amylases, en particulier de l'amidon, ou bien est entourée ou enduite au moins en partie avec ladite matière.

48. Agent selon l'une quelconque des revendications 39 à 47, **caractérisé en ce qu'**il est liquide, gélifié ou pâteux et la protéine qu'il contient et/ou au moins une des enzymes qu'il contient et/ou au moins un des autres composants qu'il contient est/sont présents à l'état encapsulé de manière individuelle ou de manière conjointe avec d'autres constituants, de préférence dans des microcapsules, de manière particulièrement préférée dans des microcapsules constituées d'une matière sensible aux amylases.

49. Agent selon l'une quelconque des revendications 39 à 48, **caractérisé en ce que** l'activité amylolytique et/ou l'activité de CGTase est modifiée, en particulier stabilisée et/ou renforcée quant à sa contribution à la puissance du lavage, respectivement du nettoyage de l'agent, via un des autres constituants de l'agent.

50. Procédé pour le nettoyage de textiles ou de surfaces dures, **caractérisé en ce que**, dans au moins une des étapes opératoires, une protéine ou un dérivé amylolytique et/ou présentant une activité de CGTase selon l'une quelconque des revendications 1 à 18, devient actif.

51. Procédé pour le nettoyage de textiles ou de surfaces dures, **caractérisé en ce que**, dans au moins une des étapes opératoires, un agent selon l'une quelconque des revendications 39 à 49, est mis en oeuvre.

52. Procédé selon la revendication 50 ou 51, **caractérisé en ce que** la protéine ou le dérivé amylolytique et/ou présentant une activité de CGTase est mis en oeuvre dans l'étape opératoire correspondante en une quantité de 0,035 mg à 2000 mg, de préférence de 0,3 mg à 1500 mg.

53. Utilisation d'une protéine ou d'un dérivé amylolytique et/ou présentant une activité de CGTase selon l'une quelconque des revendications 1 à 18, seul ou conjointement avec au moins une autre substance active présentant une activité de nettoyage ou bien soutenant une activité de nettoyage, pour le nettoyage de textiles ou de surfaces dures.

54. Utilisation d'un agent selon l'une quelconque des revendications 39 à 49 pour le nettoyage de textiles ou de surfaces dures.

55. Utilisation selon la revendication 53 ou 54, **caractérisée en ce que**, par utilisation, de préférence par utilisation dans un lave-vaisselle ou dans un lave-linge, la protéine ou le dérivé amylolytique et/ou présentant une activité de CGTase est mis en oeuvre à concurrence de 0,035 mg à 2000 mg, de préférence de 0,3 mg à 1500 mg.

56. Utilisation d'une protéine ou d'un dérivé amylolytique et/ou présentant une activité de CGTase selon l'une quelconque des revendications 1 à 18, seul ou conjointement avec au moins une autre substance active présentant une activité de nettoyage ou bien soutenant une activité de nettoyage dans un agent de lavage ou de nettoyage constitué de plus d'une phase, pour l'activation de sa propre phase ou d'autres phases.

57. Utilisation d'une protéine ou d'un dérivé amylolytique et/ou présentant une activité de CGTase selon l'une quelconque des revendications 1 à 18, seul ou conjointement avec au moins une autre substance active présentant une activité de nettoyage ou bien soutenant une activité de nettoyage dans un agent de lavage ou de nettoyage avec des constituants encapsulés à des fins de libération des constituants hors des capsules.

58. Utilisation d'une protéine ou d'un dérivé selon l'une quelconque des revendications 1 à 18, pour le traitement de matières premières ou de produits intermédiaires dans la fabrication des textiles, en particulier pour le désencollage du coton ou pour l'élimination de couches de protection d'amidon ou analogues à de l'amidon à partir de produits intermédiaires techniques.

59. Procédé pour la liquéfaction de l'amidon, en particulier pour la production d'éthanol, **caractérisé en ce qu'**on y met en oeuvre, dans au moins une étape opératoire, une protéine ou un dérivé selon l'une quelconque des revendications 1 à 18.

60. Utilisation d'une protéine ou d'un dérivé selon l'une quelconque des revendications 1 à 18 pour la liquéfaction de l'amidon, en particulier dans un procédé pour la production d'éthanol.

61. Utilisation d'une protéine ou d'un dérivé selon l'une quelconque des revendications 1 à 18 pour la préparation ou la modification d'oligosaccharides linéaires et/ou à courtes chaînes, en particulier d'une protéine ou d'un dérivé dont l'activité de CGTase a été réduite par délétion.

62. Utilisation d'une protéine ou d'un dérivé selon l'une quelconque des revendications 1 à 18 pour la préparation, la modification ou l'hydrolyse de cyclodextrines.

63. Utilisation d'une protéine ou d'un dérivé selon l'une quelconque des revendications 1 à 18 pour la fixation ou la libération de composés à bas poids moléculaires ou à poids moléculaires élevés dans des porteurs de polysaccharides, en particulier dans des cyclodextrines, respectivement à partir desdits porteurs, en particuliers desdites cyclodextrines.

64. Utilisation selon la revendication 63, pour la stabilisation de composés chimiques pendant leur préparation ou au cours de leur traitement.

65. Utilisation selon la revendication 63 ou 64 pour la préparation d'agents cosmétiques ou d'agents pharmaceutiques ou à titre de constituants desdits agents.


### SEQUENZPROTOKOLL

<110> Henkel Kommanditgesellschaft auf Aktien
<120> Neue Cyclodextrin-Glucanotransferase (CGTase) aus Bacillus agaradherens (DSM 9948) sowie Wasch- und Reinigungsmittel mit dieser neuen Cyclodextrin-Glucanotransferase
<130> H 4430 / H 4590
<140>
   <141>
<150> 10059108.6-41
   <151> 2000-11-28
<150> 10059105.1-41<151> 2000-11-28
<160> 2
<170> PatentIn Ver. 2.1
<210> 1
   <211> 2142
   <212> DNA
   <213> Bacillus agaradherens (DSM 9948)
<220>
   <221> CDS
   <222> (1)..(2142)
<400> 1
   atg agt aaa aaa act cta aag aga ttg tta gct ttg gta gta gtg tta    48
   Met Ser Lys Lys Thr Leu Lys Arg Leu Leu Ala Leu Val Val Val Leu
     1               5                  10                  15
   ttt att ttg agt gga tca ggt ata cta gat ttt tct ata aca agc gca    96
   Phe Ile Leu Ser Gly Ser Gly Ile Leu Asp Phe Ser Ile Thr Ser Ala
                20                  25                  30
   aat gca cag caa gca aca gat cgt tca aat agt gtg aac tat tca aca    144
   Asn Ala Gln Gln Ala Thr Asp Arg Ser Asn Ser Val Asn Tyr Ser Thr
            35                  40                  45
   gat ggt att tac caa att gta act gat agg ttt tac gat ggt gat gaa    192
   Asp Gly Ile Tyr Gln Ile Val Thr Asp Arg Phe Tyr Asp Gly Asp Glu
        50                  55                  60
   agt aac aat cca tca ggg gaa ctt tat tcg gag ggt tgt aaa aac cta    240
   Ser Asn Asn Pro Ser Gly Glu Leu Tyr Ser Glu Gly Cys Lys Asn Leu
    65                  70                  75                  80
   aga aaa tat tgt ggt gga gat tgg caa ggg ata ata gat aaa ata gat    288
   Arg Lys Tyr Cys Gly Gly Asp Trp Gln Gly Ile Ile Asp Lys Ile Asp
                    85                  90                  95
   gat ggt tat cta acg aac atg ggt gtg acg gca cta tgg atc tca cct    336
   Asp Gly Tyr Leu Thr Asn Met Gly Val Thr Ala Leu Trp Ile Ser Pro
               100                 105                 110
   cca gtt gaa aat att ttt gaa act att gat gat gaa tct ggg aca act    384
   Pro Val Glu Asn Ile Phe Glu Thr Ile Asp Asp Glu Ser Gly Thr Thr
           115                 120                 125
   tct tat cac ggt tat tgg gca cga gat tat aag aaa acg aac cct ttt    432
   Ser Tyr His Gly Tyr Trp Ala Arg Asp Tyr Lys Lys Thr Asn Pro Phe
       130                 135                 140
   ttc gga agt aca gaa gat ttt gaa aga tta ata gaa act gca cat agt    480
   Phe Gly Ser Thr Glu Asp Phe Glu Arg Leu Ile Glu Thr Ala His Ser
   145                 150                 155                 160
   cac gat att aaa att gtt att gat tta gct cca aac cat aca tca cct    528
   His Asp Ile Lys Ile Val Ile Asp Leu Ala Pro Asn His Thr Ser Pro
                   165                 170                 175
   gca gat ttt gat aat cct aat tat gct gaa aat ggt atc tta tat gat    576
   Ala Asp Phe Asp Asn Pro Asn Tyr Ala Glu Asn Gly Ile Leu Tyr Asp
               180                 185                 190
   aat ggt aat tat gta agc tcg tac tca gat aat tcc gat tta ttt tta    624
   Asn Gly Asn Tyr Val Ser Ser Tyr Ser Asp Asn Ser Asp Leu Phe Leu
           195                 200                 205
   tat aac ggc gga aca gac ttc tct acc tat gaa gat gag att tat aga    672
   Tyr Asn Gly Gly Thr Asp Phe Ser Thr Tyr Glu Asp Glu Ile Tyr Arg
       210                 215                 220
   aat ttg ttt gac tta gct agt ttt aat cat att aac gct gag ctg aat    720
   Asn Leu Phe Asp Leu Ala Ser Phe Asn His Ile Asn Ala Glu Leu Asn
   225                 230                 235                 240
   aat tac tta gaa gat gca gtg aaa aag tgg tta gat tta ggt ata gat    768
   Asn Tyr Leu Glu Asp Ala Val Lys Lys Trp Leu Asp Leu Gly Ile Asp
                   245                 250                 255
   ggg att cga atc gat gct gta gct cac atg cca cca ggt tgg caa aaa    816
   Gly Ile Arg Ile Asp Ala Val Ala His Met Pro Pro Gly Trp Gln Lys
               260                 265                 270
   gct tac atg gat act ata tat gac cac aga gcg gtt ttt act ttt gga    864
   Ala Tyr Met Asp Thr Ile Tyr Asp His Arg Ala Val Phe Thr Phe Gly
           275                 280                 285
   gaa tgg ttt act gga cct tat gga aac gag gat tac act aaa ttt gca    912
   Glu Trp Phe Thr Gly Pro Tyr Gly Asn Glu Asp Tyr Thr Lys Phe Ala
       290                 295                 300
   aat aat agt ggc atg agt gta tta gat ttc cgt ttt gct caa act aca    960
   Asn Asn Ser Gly Met Ser Val Leu Asp Phe Arg Phe Ala Gln Thr Thr
   305                 310                 315                 320
   cga aat gtc atc ggt aac aat aat gga acg atg tat gat att gaa aag    1008
   Arg Asn Val Ile Gly Asn Asn Asn Gly Thr Met Tyr Asp Ile Glu Lys
                   325                 330                 335
   atg cta aca gac aca gag aat gac tat gat cgt cct caa gat caa gtt    1056
   Met Leu Thr Asp Thr Glu Asn Asp Tyr Asp Arg Pro Gln Asp Gln Val
           340                 345                 350
   act ttt ctt gat aat cat gac atg agt cga ttt acg aat gat ggt gaa      1104
   Thr Phe Leu Asp Asn His Asp Met Ser Arg Phe Thr Asn Asp Gly Glu
           355                 360                 365
   tca aca cgt acc aca gat att gga tta gct tta atg tta aca tct cgt      1152
   Ser Thr Arg Thr Thr Asp Ile Gly Leu Ala Leu Met Leu Thr Ser Arg
       370                 375                 380
   gga gtt cct acc ata tat tat gga aca gaa caa tac atg gaa ggt gat      1200
   Gly Val Pro Thr Ile Tyr Tyr Gly Thr Glu Gln Tyr Met Glu Gly Asp
   385                 390                 395                 400
   gga gat cca ggt agc cgg gga atg atg gaa tcc ttt ggt gaa aat aca      1248
   Gly Asp Pro Gly Ser Arg Gly Met Met Glu Ser Phe Gly Glu Asn Thr
                   405                 410                 415
   gat gct tat aag cta att caa aaa tta gcg ccg tta aga aaa agt aat      1296
   Asp Ala Tyr Lys Leu Ile Gln Lys Leu Ala Pro Leu Arg Lys Ser Asn
               420                 425                 430
   ccg gca tat gga tac gga aca aca aaa gaa cgt tgg ata aat gat gat      1344
   Pro Ala Tyr Gly Tyr Gly Thr Thr Lys Glu Arg Trp Ile Asn Asp Asp
           435                 440                 445
   gtc atc att tat gaa aga aat ttt ggt gat aat tat gct tta att gcg      1392
   Val Ile Ile Tyr Glu Arg Asn Phe Gly Asp Asn Tyr Ala Leu Ile Ala
       450                 455                 460
   ata aat aga aac tta aat acc tca tat aat atc caa gga tta caa aca      1440
   Ile Asn Arg Asn Leu Asn Thr Ser Tyr Asn Ile Gln Gly Leu Gln Thr
   465                 470                 475                 480
   gag atg ccc tcc aat tca tat gac gat gta tta gat ggc tta ttg gat      1488
   Glu Met Pro Ser Asn Ser Tyr Asp Asp Val Leu Asp Gly Leu Leu Asp
                   485                 490                 495
   gga caa tcg att gtt gtg gat aac aat ggg gaa gtt aat gaa ttc caa      1536
   Gly Gln Ser Ile Val Val Asp Asn Asn Gly Glu Val Asn Glu Phe Gln
               500                 505                 510
   atg tct cca gga gag gtg ggt gta tgg gaa ttc gaa gcg aca aat gtt      1584
   Met Ser Pro Gly Glu Val Gly Val Trp Glu Phe Glu Ala Thr Asn Val
           515                 520                 525
   gac aag cct tca att gga caa gtt ggc cca ata att ggt gag gca gga      1632
   Asp Lys Pro Ser Ile Gly Gln Val Gly Pro Ile Ile Gly Glu Ala Gly
       530                 535                 540
   cga act gtt aca ata agt gga gaa gga ttc gga tct tcg ccg ggg act      1680
   Arg Thr Val Thr Ile Ser Gly Glu Gly Phe Gly Ser Ser Pro Gly Thr
   545                 550                 555                 560
   gtt caa ttt ggt tca act tca gca gaa atc gtt tct tgg aat gac aca      1728
   Val Gln Phe Gly Ser Thr Ser Ala Glu Ile Val Ser Trp Asn Asp Thr
                   565                 570                 575
   gtc att atc ata act gtg ccg aac aat gag gca gga tac cat gat atc      1776
   Val Ile Ile Ile Thr Val Pro Asn Asn Glu Ala Gly Tyr His Asp Ile
                   580                 585                 590
   act gta gta aca gaa gat gaa caa gta agt aat gcc tat gaa ttt gaa      1824
   Thr Val Val Thr Glu Asp Glu Gln Val Ser Asn Ala Tyr Glu Phe Glu
           595                 600                 605
   gtt ctc acg gcc gat caa gtc aca gtt cgc ttt atc ata gac aat gca      1872
   Val Leu Thr Ala Asp Gln Val Thr Val Arg Phe Ile Ile Asp Asn Ala
       610                 615                 620
   gaa acg aag atg ggt gaa aat att ttc ctt gta ggt aac gtt cat gaa      1920
   Glu Thr Lys Met Gly Glu Asn Ile Phe Leu Val Gly Asn Val His Glu
   625                 630                 635                 640
   tta ggc aat tgg gac cca gag caa tca gtg ggg aga ttt ttc aat cag      1968
   Leu Gly Asn Trp Asp Pro Glu Gln Ser Val Gly Arg Phe Phe Asn Gln
                   645                 650                 655
   gta gta tat caa tat cca aca tgg tat tat gat gtg aat gtt cct gca      2016
   Val Val Tyr Gln Tyr Pro Thr Trp Tyr Tyr Asp Val Asn Val Pro Ala
               660                 665                 670
   aat aca gac tta gaa ttc aag ttt att aaa ata gat caa gat aat aat      2064
   Asn Thr Asp Leu Glu Phe Lys Phe Ile Lys Ile Asp Gln Asp Asn Asn
           675                 680                 685
   gtc act tgg cag agt gga gct aat cat acc tat tct tcg cca gaa agt      2112
   Val Thr Trp Gln Ser Gly Ala Asn His Thr Tyr Ser Ser Pro Glu Ser
       690                 695                 700
   gga acg ggt att att aga gtt gat tgg taa                              2142
   Gly Thr Gly Ile Ile Arg Val Asp Trp
   705                 710
<210> 2
   <211> 713
   <212> PRT
   <213> Bacillus agaradherens (DSM 9948)
<400> 2
   Met Ser Lys Lys Thr Leu Lys Arg Leu Leu Ala Leu Val Val Val Leu
    1               5                  10                  15
   Phe Ile Leu Ser Gly Ser Gly Ile Leu Asp Phe Ser Ile Thr Ser Ala
               20                  25                  30
   Asn Ala Gln Gln Ala Thr Asp Arg Ser Asn Ser Val Asn Tyr Ser Thr
           35                  40                  45
   Asp Gly Ile Tyr Gln Ile Val Thr Asp Arg Phe Tyr Asp Gly Asp Glu
       50                  55                  60
   Ser Asn Asn Pro Ser Gly Glu Leu Tyr Ser Glu Gly Cys Lys Asn Leu
   65                  70                  75                  80
   Arg Lys Tyr Cys Gly Gly Asp Trp Gln Gly Ile Ile Asp Lys Ile Asp
                   85                  90                  95
   Asp Gly Tyr Leu Thr Asn Met Gly Val Thr Ala Leu Trp Ile Ser Pro
               100                 105                 110
   Pro Val Glu Asn Ile Phe Glu Thr Ile Asp Asp Glu Ser Gly Thr Thr
           115                 120                 125
   Ser Tyr His Gly Tyr Trp Ala Arg Asp Tyr Lys Lys Thr Asn Pro Phe
       130                 135                 140
   Phe Gly Ser Thr Glu Asp Phe Glu Arg Leu Ile Glu Thr Ala His Ser
   145 150 155 160
   His Asp Ile Lys Ile Val Ile Asp Leu Ala Pro Asn His Thr Ser Pro
   165 170 175
   Ala Asp Phe Asp Asn Pro Asn Tyr Ala Glu Asn Gly Ile Leu Tyr Asp
   180 185 190
   Asn Gly Asn Tyr Val Ser Ser Tyr Ser Asp Asn Ser Asp Leu Phe Leu
   195 200 205
   Tyr Asn Gly Gly Thr Asp Phe Ser Thr Tyr Glu Asp Glu Ile Tyr Arg
   210 215 220
   Asn Leu Phe Asp Leu Ala Ser Phe Asn His Ile Asn Ala Glu Leu Asn
   225 230 235 240
   Asn Tyr Leu Glu Asp Ala Val Lys Lys Trp Leu Asp Leu Gly Ile Asp
   245 250 255
   Gly Ile Arg Ile Asp Ala Val Ala His Met Pro Pro Gly Trp Gln Lys
   260 265 270
   Ala Tyr Met Asp Thr Ile Tyr Asp His Arg Ala Val Phe Thr Phe Gly
   275 280 285
   Glu Trp Phe Thr Gly Pro Tyr Gly Asn Glu Asp Tyr Thr Lys Phe Ala
   290 295 300
   Asn Asn Ser Gly Met Ser Val Leu Asp Phe Arg Phe Ala Gln Thr Thr
   305 310 315 320
   Arg Asn Val Ile Gly Asn Asn Asn Gly Thr Met Tyr Asp Ile Glu Lys
   325 330 335
   Met Leu Thr Asp Thr Glu Asn Asp Tyr Asp Arg Pro Gln Asp Gln Val
   340 345 350
   Thr Phe Leu Asp Asn His Asp Met Ser Arg Phe Thr Asn Asp Gly Glu
   355 360 365
   Ser Thr Arg Thr Thr Asp Ile Gly Leu Ala Leu Met Leu Thr Ser Arg
   370 375 380
   Gly Val Pro Thr Ile Tyr Tyr Gly Thr Glu Gln Tyr Met Glu Gly Asp
   385 390 395 400
   Gly Asp Pro Gly Ser Arg Gly Met Met Glu Ser Phe Gly Glu Asn Thr
   405 410 415
   Asp Ala Tyr Lys Leu Ile Gln Lys Leu Ala Pro Leu Arg Lys Ser Asn
   420 425 430
   Pro Ala Tyr Gly Tyr Gly Thr Thr Lys Glu Arg Trp Ile Asn Asp Asp
   435 440 445
   Val Ile Ile Tyr Glu Arg Asn Phe Gly Asp Asn Tyr Ala Leu Ile Ala
   450 455 460
   Ile Asn Arg Asn Leu Asn Thr Ser Tyr Asn Ile Gln Gly Leu Gln Thr
   465 470 475 480
   Glu Met Pro Ser Asn Ser Tyr Asp Asp Val Leu Asp Gly Leu Leu Asp
   485 490 495
   Gly Gln Ser Ile Val Val Asp Asn Asn Gly Glu Val Asn Glu Phe Gln
   500 505 510
   Met Ser Pro Gly Glu Val Gly Val Trp Glu Phe Glu Ala Thr Asn Val
   515 520 525
   Asp Lys Pro Ser Ile Gly Gln Val Gly Pro Ile Ile Gly Glu Ala Gly
   530 535 540
   Arg Thr Val Thr Ile Ser Gly Glu Gly Phe Gly Ser Ser Pro Gly Thr
   545 550 555 560
   Val Gln Phe Gly Ser Thr Ser Ala Glu Ile Val Ser Trp Asn Asp Thr
   565 570 575
   Val Ile Ile Ile Thr Val Pro Asn Asn Glu Ala Gly Tyr His Asp Ile
   580 585 590
   Thr Val Val Thr Glu Asp Glu Gln Val Ser Asn Ala Tyr Glu Phe Glu
   595 600 605
   Val Leu Thr Ala Asp Gln Val Thr Val Arg Phe Ile Ile Asp Asn Ala
   610 615 620
   Glu Thr Lys Met Gly Glu Asn Ile Phe Leu Val Gly Asn Val His Glu
   625                 630                 635                 640
   Leu Gly Asn Trp Asp Pro Glu Gln Ser Val Gly Arg Phe Phe Asn Gln
                   645                 650                 655
   Val Val Tyr Gln Tyr Pro Thr Trp Tyr Tyr Asp Val Asn Val Pro Ala
               660                 665                 670
   Asn Thr Asp Leu Glu Phe Lys Phe Ile Lys Ile Asp Gln Asp Asn Asn
           675                 680                 685
   Val Thr Trp Gln Ser Gly Ala Asn His Thr Tyr Ser Ser Pro Glu Ser
       690                 695                 700
   Gly Thr Gly Ile Ile Arg Val Asp Trp
   705                 710